# EUROPEAN PATENT APPLICATION

(11) **EP 4 759 805 A1**
(43) Date of publication of application: **17.06.2026**
(21) Application number: 24853684.9
(22) Date of filing: 09.08.2024
(51) Int. Cl.: C07D 237/20, C07D 403/12, C07D 401/04, A61K 31/50, A61P 37/06

(54) **NLRP3 INFLAMMASOME INHIBITOR AND USE THEREOF**

(30) Priority: 11.08.2023 CN 202311008972
(71) Applicant: NEUROCRINE BIOSCIENCES, INC., San Diego, CA 92130 (US)
(72) Inventor: LI, Lin, Nanjing, Jiangsu 210032 (CN)
(74) Representative: Mewburn Ellis LLP
(86) International application number: PCT/CN2024/110960
(87) International publication number: WO 2025/036275

(57) **Abstract**

The present disclosure belongs to the technical field of pharmaceuticals, and relates to an NLRP3 inflammasome inhibitor and the use thereof. Specifically, the present disclosure relates to a compound represented by general formula (I), or a deuterated compound thereof, a stereoisomer thereof, or a pharmaceutically acceptable salt thereof. The definition of each group is as defined in the description. Studies show that the compound represented by general formula (I), or the deuterated compound thereof, the stereoisomer thereof, or the pharmaceutically acceptable salt thereof has a relatively high biological activity against an NLRP3 inflammasome, and has an important clinical development value for the treatment of NLRP3-related diseases Y-W-R₃ (I).

## Description

### CROSS-REFERENCE TO RELATED APPLICATION

The present disclosure claims priority to Chinese Patent Application No. 202311008972.0 filed on August 11, 2023 in China and entitled "NLRP3 inflammasome inhibitor and use thereof", the entire contents of which are incorporated herein by reference.

### TECHNICAL FIELD

The present disclosure belongs to the technical field of medicine, and specifically relates to NLRP3 inflammasome inhibitor and use thereof.

### BACKGROUND

Nucleotide-binding oligomerization domain-like receptor protein 3 (NOD-like receptor protein 3, NLRP3) belongs to the family of nucleotide-binding oligomerization domain-like receptors (NOD-like receptors, NLRs), and is also known as "pyrin domain-containing protein 3". NLRP3 comprises three modules: a pyrin domain (PYD), a nucleotide-binding domain (NBD) and a leucine-rich repeat (LRR). Upon receiving stimulation by sterile inflammatory danger signals, NLRP3 interacts with adapter protein apoptosis-associated speck-like protein (ASC) and pro-caspase 1, thus forming an NLRP3 inflammasome. The activation of the NLRP3 inflammasome results in the release of interleukin-1β (IL-1β) and interleukin-18 (IL-18).

The activation of the NLRP3 inflammasome typically requires two steps. The first step involves initiating a signal, in which Toll-like receptor recognizes pathogen-associated molecular pattern (PAMP) or damage-associated molecular pattern (DAMP), and then transmits the signal into a cell to mediate the activation of NF-κB signaling pathway, thereby upregulating the transcriptional levels of NLRP3 inflammasome-related components including inactive NLRP3, pro-IL-1β and the like. The second step is activating the signal. After P2X7 receptor and the like receive signal stimulation from ATP, nigericin, and the like, an NLRP3 monomer oligomerizes to form an NLRP3 oligomer, which then recruits ASC and pro-caspase 1 to assemble into an NLRP3 inflammasome complex. This triggers the conversion of pro-caspase 1 to caspase 1, as well as the production and secretion of mature IL-1β and IL-18.

Activation of the NLRP3 inflammasome is associated with a variety of diseases, for example, auto inflammatory fever syndrome such as cryopyrin-associated periodic syndrome (CAPS), sickle cell disease, systemic lupus erythematosus (SLE), chronic liver disease, non-alcoholic steatosis hepatitis (NASH), gout, pseudogout (chondrocalcinosis), type I and type II diabetes and related complications (for example, kidney disease and retinopathy), neuroinflammation-related disorders (for example, multiple sclerosis, brain infection, acute injury, neurodegenerative diseases, Alzheimer's disease), atherosclerosis and cardiovascular risk (for example, hypertension), hidradenitis suppurativa, wound healing and cicatrization, and cancers (for example, colorectal cancer, lung cancer, myeloproliferative neoplasm, leukemia, myelodysplastic syndrome (MDS), myelofibrosis). The majority of the therapeutic methods include symptomatic treatment, slowing down the progression of the disease/disorder, and taking therapeutic surgery as a last resort.

Currently, there are few varieties of NLRP3 inflammasome inhibitors under development. Developing NLRP3 inflammasome inhibitors with higher activity and better druggability has become a clinical necessity.

### SUMMARY

The present disclosure has investigated the following compounds, or the deuterated compounds thereof, the stereoisomers thereof or the pharmaceutically acceptable salts thereof. Studies have found that these compounds, or the deuterated compounds thereof, the stereoisomers thereof or the pharmaceutically acceptable salts thereof have relatively high bioactivity against the NLRP3 inflammasome, and have an important value in clinical development for the treatment of NLRP3-related diseases.

To achieve the above objectives, the present disclosure provides the following solutions.

A compound represented by general formula (I), or a deuterated compound thereof, a stereoisomer thereof or a pharmaceutically acceptable salt thereof:

Y-W-R₃ (I)

wherein W is selected from the group consisting of 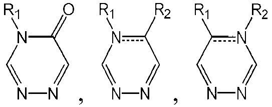 and 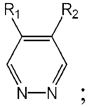
-̅ -̅ -̅ -̅ -̅ is a single bond or a double bond;
R₁ is independently selected from the group consisting of hydrogen, oxo, thio, hydroxyl, amino, carboxyl, cyano, nitro, halogen, carbonyl, C₁₋₆ alkyl, halogenated C₁₋₆ alkyl, C₁₋₆ alkoxy, -S-C₁₋₆ alkyl, halogenated C₁₋₆ alkoxy, C₂₋₆ alkenyl, C₂₋₆ alkynyl, 3-7 membered heterocyclyl, 3-7 membered cycloalkyl, 5-7 membered cycloalkenyl, aryl, 5-7 membered heteroaryl, sulfonyl, -N(C₁₋₆ alkyl)₂, and null;
R₂ is independently selected from the group consisting of hydrogen, oxo, thio, hydroxyl, amino, carboxyl, cyano, nitro, halogen, carbonyl, C₁₋₆ alkyl, halogenated C₁₋₆ alkyl, C₁₋₆ alkoxy, -S-C₁₋₆ alkyl, halogenated C₁₋₆ alkoxy, C₂₋₆ alkenyl, C₂₋₆ alkynyl, 3-7 membered heterocyclyl, 3-7 membered cycloalkyl, 5-7 membered cycloalkenyl, aryl, 5-7 membered heteroaryl, sulfonyl, -N(C₁₋₆ alkyl)₂, and null;
said R₁ and R₂ are independently and optionally substituted with 1-3 substituents selected from the group consisting of hydroxyl, C₀₋₆ alkylamino, carboxyl, cyano, nitro, halogen, C₀₋₆ alkylcarbonyl, C₁₋₆ alkyl, halogenated C₁₋₆ alkyl, C₁₋₆ alkoxy, halogenated C₁₋₆ alkoxy, C₂₋₆ alkenyl, C₂₋₆ alkynyl, 3-7 membered heterocyclyl, 3-7 membered cycloalkyl, 5-7 membered cycloalkenyl, aryl, 5-7 membered heteroaryl, and C₀₋₆ alkylsulfonyl;
or, R₁ and R₂, together with the C atom or N atom to which they are attached, form a 5-12 membered ring A;
said 5-12 membered ring A is optionally substituted with 1-4 substituents selected from the group consisting of hydroxyl, amino, carboxyl, cyano, nitro, halogen, carbonyl, oxo, C₁₋₆ alkyl, -NH-C₁₋₆ alkyl, halogenated C₁₋₆ alkyl, C₁₋₆ alkoxy, halogenated C₁₋₆ alkoxy, 3-7 membered heterocyclyl, 3-7 membered cycloalkyl, aryl, 5-7 membered heteroaryl, C₁₋₆ alkylsulfonyl, and -N(C₁₋₆ alkyl)₂;
R₃ is -(CH₂)ₙ-NR₄-Z-(CH₂)ₘ-R₅ or -(CH₂)ₙ-NR₄-Z-CR^{c}R^{d}-R₅;
n is an integer from 0 to 6;
m is an integer from 0 to 3;
R₄ is hydrogen or C₁₋₆ alkyl;
Z is selected from the group consisting of C=O, C=S, S(O) and S(O)₂;
R₅ is selected from the group consisting of 3-7 membered heterocyclyl, 3-7 membered cycloalkyl, 5-7 membered cycloalkenyl, aryl, 5-7 membered heteroaryl, NR^{a}R^{b}, OR^{b}, and C₁₋₆ alkyl;
R^{a} and R^{c} are hydrogen or C₁₋₆ alkyl;
R^{b} and R^{d} are selected from the group consisting of hydrogen, C₁₋₆ alkyl, halogenated C₁₋₆ alkyl, C₁₋₆ alkoxy, -S-C₁₋₆ alkyl, halogenated C₁₋₆ alkoxy, C₂₋₆ alkenyl, C₂₋₆ alkynyl, 3-7 membered heterocyclyl, 3-7 membered cycloalkyl, 5-7 membered cycloalkenyl, aryl, 5-7 membered heteroaryl, sulfonyl, and -N(C₁₋₆ alkyl)₂;
said R^{b} is optionally substituted with 1-2 substituents selected from the group consisting of hydroxyl, halogen, 3-7 membered cycloalkyl, halogenated C₁₋₆ alkyl, and C₁₋₆ alkoxy;
said R^{d} is optionally substituted with 1-2 substituents selected from the group consisting of hydroxyl, halogen, 3-7 membered cycloalkyl, halogenated C₁₋₆ alkyl, C₁₋₆ alkoxy, aryl, cyano, and carboxyl;
or, R^{c} and R^{d}, together with the C atom to which they are attached, form a 3-7 membered cycloalkyl;
or, when R₅ is NR^{a}R^{b}, R^{a} is attached to R₄ such that R₃ forms a 4-7 membered heterocyclyl;
said R₅ is optionally substituted with 1-4 substituents selected from the group consisting of oxo, thio, hydroxyl, amino, carboxyl, cyano, nitro, halogen, carbonyl, C₁₋₆ alkyl, halogenated C₁₋₆ alkyl, C₁₋₆ alkoxy, -S-C₁₋₆ alkyl, halogenated C₁₋₆ alkoxy, C₂₋₆ alkenyl, C₂₋₆ alkynyl, 3-7 membered heterocyclyl, 3-7 membered cycloalkyl, 5-7 membered cycloalkenyl, aryl, 5-7 membered heteroaryl, sulfonyl, ureido, and hydrazino;
a substituent on R₅ may be further optionally substituted with 1-3 substituents selected from the group consisting of hydroxyl, C₀₋₆ alkylamino, carboxyl, cyano, nitro, halogen, C₀₋₆ alkylcarbonyl, C₁₋₆ alkyl, halogenated C₁₋₆ alkyl, C₁₋₆ alkoxy, -S-C₁₋₆ alkyl, halogenated C₁₋₆ alkoxy, C₂₋₆ alkenyl, C₂₋₆ alkynyl, 3-6 membered heterocyclyl, 3-6 membered cycloalkyl, 5-6 membered cycloalkenyl, aryl, 5-7 membered heteroaryl, C₀₋₆ alkylsulfonyl, ureido, and hydrazino;
Y is selected from the group consisting of aryl, 5-14 membered heteroaryl, 3-14 membered heterocyclyl, 3-12 membered cycloalkyl, and 3-12 membered cycloalkenyl;
said Y is optionally substituted with 1-4 substituents selected from the group consisting of oxo, thio, hydroxyl, amino, carboxyl, cyano, nitro, halogen, carbonyl, C₁₋₆ alkyl, halogenated C₁₋₆ alkyl, C₁₋₆ alkoxy, -S-C₁₋₆ alkyl, halogenated C₁₋₆ alkoxy, C₂₋₆ alkenyl, C₂₋₆ alkynyl, 3-7 membered heterocyclyl, 3-7 membered cycloalkyl, 5-7 membered cycloalkenyl, aryl, 5-7 membered heteroaryl, and sulfonyl;
a substituent on Y may be further optionally substituted with 1-3 substituents selected from the group consisting of hydroxyl, C₀₋₆ alkylamino, carboxyl, cyano, nitro, halogen, C₀₋₆ alkylcarbonyl, C₁₋₆ alkyl, halogenated C₁₋₆ alkyl, C₁₋₆ alkoxy, -S-C₁₋₆ alkyl, halogenated C₁₋₆ alkoxy, C₂₋₆ alkenyl, C₂₋₆ alkynyl, 3-6 membered heterocyclyl, 3-7 membered cycloalkyl, 5-6 membered cycloalkenyl, aryl, 5-7 membered heteroaryl, and C₀₋₆ alkylsulfonyl.

In any one of the above technical solutions, W is

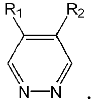

In any one of the above technical solutions,
R₁ is independently selected from the group consisting of hydrogen, oxo, thio, hydroxyl, amino, carboxyl, cyano, nitro, halogen, carbonyl, C₁₋₆ alkyl, halogenated C₁₋₆ alkyl, C₁₋₆ alkoxy, -S-C₁₋₆ alkyl, halogenated C₁₋₆ alkoxy, C₂₋₆ alkenyl, C₂₋₆ alkynyl, 3-7 membered heterocyclyl, 3-7 membered cycloalkyl, 5-7 membered cycloalkenyl, aryl, 5-7 membered heteroaryl, and sulfonyl;
R₂ is independently selected from the group consisting of hydrogen, oxo, thio, hydroxyl, amino, carboxyl, cyano, nitro, halogen, carbonyl, C₁₋₆ alkyl, halogenated C₁₋₆ alkyl, C₁₋₆ alkoxy, -S-C₁₋₆ alkyl, halogenated C₁₋₆ alkoxy, C₂₋₆ alkenyl, C₂₋₆ alkynyl, 3-7 membered heterocyclyl, 3-7 membered cycloalkyl, 5-7 membered cycloalkenyl, aryl, 5-7 membered heteroaryl, and sulfonyl;
said R₁ and R₂ are independently and optionally substituted with 1-3 substituents selected from the group consisting of hydroxyl, C₀₋₆ alkylamino, carboxyl, cyano, nitro, halogen, C₀₋₆ alkylcarbonyl, C₁₋₆ alkyl, halogenated C₁₋₆ alkyl, C₁₋₆ alkoxy, halogenated C₁₋₆ alkoxy, C₂₋₆ alkenyl, C₂₋₆ alkynyl, 3-7 membered heterocyclyl, 3-7 membered cycloalkyl, 5-7 membered cycloalkenyl, aryl, 5-7 membered heteroaryl, and C₀₋₆ alkylsulfonyl.

In any one of the above technical solutions, R₁ and R₂ are independently selected from the group consisting of hydrogen, cyano, halogen, C₁₋₆ alkyl, halogenated C₁₋₆ alkyl, C₁₋₆ alkoxy, halogenated C₁₋₆ alkoxy, 3-7 membered cycloalkyl, -N(C₁₋₆ alkyl)₂, and null; preferably, R₁ and R₂ are independently selected from the group consisting of hydrogen, cyano, halogen, C₁₋₆ alkyl, halogenated C₁₋₆ alkyl, cyclopropyl, cyclobutyl, and null.

In any one of the above technical solutions, R₁ and R₂ are independently selected from the group consisting of hydrogen, hydroxyl, amino, cyano, halogen, C₁₋₆ alkyl, halogenated C₁₋₆ alkyl, C₁₋₆ alkoxy, 3-7 membered cycloalkyl, and sulfonyl; preferably, R₁ and R₂ are independently selected from the group consisting of hydrogen, amino, cyano, halogen, C₁₋₆ alkyl, halogenated C₁₋₆ alkyl, C₁₋₆ alkoxy, and 3-7 membered cycloalkyl; further preferably, R₁ and R₂ are independently selected from the group consisting of hydrogen, C₁₋₆ alkyl, halogenated C₁₋₆ alkyl, and 3-7 membered cycloalkyl; more preferably, R₁ and R₂ are independently selected from the group consisting of hydrogen, methyl, ethyl, trifluoromethyl, cyclopropyl, and cyclobutyl. When R₁ and R₂ are independently amino, R₁ and R₂ may be further substituted with C₁₋₆ alkyl. In any one of the above technical solutions, R₁ and R₂ are independently hydrogen or halogen; preferably, R₁ and R₂ are independently selected from the group consisting of hydrogen, fluorine, chlorine, and bromine.

In any one of the above technical solutions, R₁ and R₂ are independently C₁₋₆ alkyl or C₁₋₆ alkoxy; preferably, R₁ and R₂ are independently selected from the group consisting of methyl, ethyl, isopropyl, butyl, and methoxy.

In any one of the above technical solutions, R₁ and R₂ are independently halogenated C₁₋₆ alkyl; preferably, R₁ and R₂ are independently trifluoromethyl or difluoromethyl.

In any one of the above technical solutions, R₁ and R₂ are independently 3-7 membered cycloalkyl or halogenated 3-7 membered cycloalkyl; preferably, R₁ and R₂ are independently cyclopropyl or cyclobutyl.

In any one of the above technical solutions, R₁ and R₂, together with the C atom or N atom to which they are attached, form a 5-12 membered ring A.

In some embodiments, the compound, or the deuterated compound thereof, the stereoisomer thereof or the pharmaceutically acceptable salt thereof as provided by the present disclosure has the structure as represented by general formula (II): 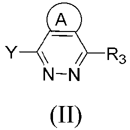 wherein ring A is selected from the group consisting of 5-7 membered cycloalkenyl, 5-7 membered cycloalkyl, 5-7 membered heterocyclyl, phenyl, and 5-7 membered heteroaryl; ring A is optionally substituted with 1-4 substituents selected from the group consisting of hydroxyl, amino, carboxyl, cyano, nitro, halogen, C₁₋₆ alkyl, -NH-C₁₋₆ alkyl, halogenated C₁₋₆ alkyl, C₁₋₆ alkoxy, halogenated C₁₋₆ alkoxy, 3-7 membered heterocyclyl, 3-7 membered cycloalkyl, aryl, 5-7 membered heteroaryl, C₁₋₆ alkylsulfonyl, and -N(C₁₋₆ alkyl)₂.

Further, said ring A is selected from the group consisting of phenyl, 5-7 membered cycloalkyl, and 5-7 membered heteroaryl; ring A is optionally substituted with 1-4 substituents selected from the group consisting of hydroxyl, amino, carboxyl, cyano, nitro, halogen, C₁₋₆ alkyl, -NH-C₁₋₆ alkyl, halogenated C₁₋₆ alkyl, C₁₋₆ alkoxy, halogenated C₁₋₆ alkoxy, 3-7 membered heterocyclyl, 3-7 membered cycloalkyl, C₁₋₆ alkylsulfonyl, and -N(C₁₋₆ alkyl)₂.

Furthermore, said ring A is selected from the group consisting of 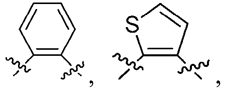 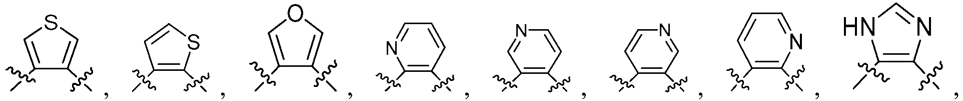 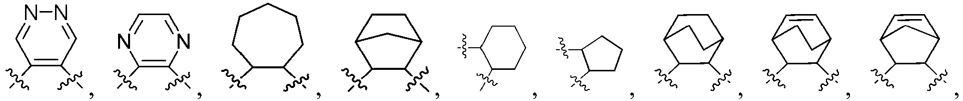 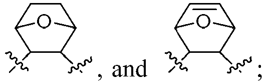 ring A is optionally substituted with 1-2 substituents selected from the group consisting of cyano, halogen, C₁₋₆ alkyl, -NH-C₁₋₆ alkyl, halogenated C₁₋₆ alkyl, C₁₋₆ alkoxy, 3-7 membered cycloalkyl, 5-7 membered heteroaryl, C₁₋₆ alkylsulfonyl, and -N(C₁₋₆ alkyl)₂.

In any one of the above technical solutions, R₃ is -(CH₂)ₙ-NR₄-Z-(CH₂)ₘ-R₅ or -(CH₂)ₙ-NR₄-Z-CR^{c}R^{d}-R₅;
n is an integer from 0 to 2;
m is an integer from 0 to 2;
R₄ is hydrogen or C₁₋₆ alkyl;
Z is C=O;
R₅ is selected from the group consisting of 3-7 membered heterocyclyl, NR^{a}R^{b}, OR^{b}, and C₁₋₆ alkyl;
R^{a} and R^{c} are hydrogen or C₁₋₆ alkyl;
R^{b} and R^{d} are selected from the group consisting of hydrogen, C₁₋₆ alkyl, halogenated C₁₋₆ alkyl, C₁₋₆ alkoxy, -S-C₁₋₆ alkyl, halogenated C₁₋₆ alkoxy, 3-7 membered heterocyclyl, 3-7 membered cycloalkyl, aryl, and 5-7 membered heteroaryl;
said R^{b} is optionally substituted with 1-2 substituents selected from the group consisting of hydroxyl, halogen, 3-7 membered cycloalkyl, halogenated C₁₋₆ alkyl, and C₁₋₆ alkoxy;
said R^{d} is optionally substituted with 1-2 substituents selected from the group consisting of hydroxyl, halogen, 3-7 membered cycloalkyl, halogenated C₁₋₆ alkyl, C₁₋₆ alkoxy, aryl, cyano, and carboxyl.

Further, among which,
n is 0;
m is 0;
R₅ is 3-7 membered heterocyclyl;
said R₅ is optionally substituted with 1-4 substituents selected from the group consisting of hydroxyl, amino, carboxyl, cyano, halogen, C₁₋₆ alkyl, halogenated C₁₋₆ alkyl, C₁₋₆ alkoxy, 3-7 membered heterocyclyl, 3-7 membered cycloalkyl, and aryl;

Further, said R₅ is a 4-7 membered heterocyclyl containing 1~2 heteroatoms selected from the group consisting of O, N and S; preferably, R₅ is a 4-7 membered heterocyclyl containing one N heteroatom; preferably, R₅ is selected from the group consisting of

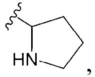

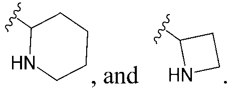

The substituent on R₅ may be further optionally substituted with 1-3 substituents selected from the group consisting of hydroxyl, halogen, and C₁₋₆ alkyl.

Further, among which,
n is 0;
m is 0;
R₅ is NR^{a}R^{b};
R^{a} and R^{c} are hydrogen or C₁₋₆ alkyl;
R^{b} and R^{d} are selected from the group consisting of hydrogen, C₁₋₆ alkyl, halogenated C₁₋₆ alkyl, C₁₋₆ alkoxy, cyclopropyl, and cyclobutyl;
said R^{b} is optionally substituted with 1-2 substituents selected from the group consisting of hydroxyl, halogen, cyclopropyl, cyclobutyl, and C₁₋₆ alkoxy;
said R^{d} is optionally substituted with 1-2 substituents selected from the group consisting of hydroxyl, halogen, cyclopropyl, cyclobutyl, halogenated C₁₋₆ alkyl, C₁₋₆ alkoxy, and phenyl.

In any one of the above technical solutions, R₃ is

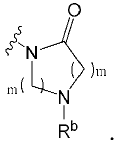

Further, m is an integer from 1 to 2; preferably, m is 1.

In any one of the above technical solutions, R₃ is -(CH₂)ₙ-NR₄-Z-(CH₂)ₘ-R₅;
n is an integer from 0 to 6;
m is an integer from 0 to 3;
preferably, n is an integer from 0 to 2, and m is an integer from 1 to 2; more preferably, n is 0, and m is 1.

In any one of the above technical solutions, R₃ is -(CH₂)ₙ-NR₄-Z-(CH₂)ₘ-R₅ or -(CH₂)ₙ-NR₄-Z-CR^{c}R^{d}-R₅;
n is an integer from 0 to 2;
m is an integer from 0 to 2;
preferably, n is 0, and m is an integer from 0 to 2; preferably, n is 0, and m is 0 or 1.

In any one of the above technical solutions, R₄ is selected from the group consisting of hydrogen, methyl, and ethyl.

In any one of the above technical solutions,
R₅ is NR^{a}R^{b} or OR^{b};
R^{a} is hydrogen or C₁₋₆ alkyl;
R^{b} is selected from the group consisting of hydrogen, C₁₋₆ alkyl, halogenated C₁₋₆ alkyl, 3-7 membered heterocyclyl, and 3-7 membered cycloalkyl;
said R^{b} is optionally substituted with 1-2 substituents selected from the group consisting of hydroxyl, halogen, 3-7 membered cycloalkyl, and halogenated C₁₋₆ alkyl.

In any one of the above technical solutions,
R^{a} is hydrogen or C₁₋₆ alkyl;
R^{b} is selected from the group consisting of hydrogen, C₁₋₆ alkyl, halogenated C₁₋₆ alkyl, and 3-7 membered cycloalkyl, said R^{b} is optionally substituted with 1-2 substituents selected from the group consisting of hydroxyl, halogen, 3-7 membered cycloalkyl, and halogenated C₁₋₆ alkyl.

Further, R^{b} is selected from the group consisting of hydrogen, methyl, ethyl, isopropyl, cyclopropyl, and cyclobutyl, said R^{b} is optionally substituted with 1-2 substituents selected from the group consisting of hydroxyl, cyclopropyl, and trifluoromethyl.

In any one of the above technical solutions,
R^{c} is hydrogen or C₁₋₆ alkyl;
R^{d} is selected from the group consisting of hydrogen, C₁₋₆ alkyl, halogenated C₁₋₆ alkyl, C₁₋₆ alkoxy, -S-C₁₋₆ alkyl, and 3-7 membered cycloalkyl, said R^{d} is optionally substituted with 1-2 substituents selected from the group consisting of hydroxyl, halogen, 3-7 membered cycloalkyl, halogenated C₁₋₆ alkyl, C₁₋₆ alkoxy, aryl, and cyano.

Further, R^{d} is selected from the group consisting of hydrogen, methyl, ethyl, isopropyl, and cyclopropyl, said R^{d} is optionally substituted with 1-2 substituents selected from the group consisting of hydroxyl, halogen, cyclopropyl, cyclobutyl, trifluoromethyl, methoxy, phenyl, and cyano.

In any one of the above technical solutions,
Y is phenyl or 5-14 membered heteroaryl;
said Y is optionally substituted with 1-4 substituents selected from the group consisting of hydroxyl, amino, cyano, halogen, C₁₋₆ alkyl, halogenated C₁₋₆ alkyl, C₁₋₆ alkoxy, -S-C₁₋₆ alkyl, 3-7 membered cycloalkyl, C₂₋₆ alkenyl, and C₂₋₆ alkynyl;
when a substituent on Y is selected from the group consisting of C₁₋₆ alkyl, 3-7 membered cycloalkyl, C₂₋₆ alkenyl, and C₂₋₆ alkynyl, said substituent is further optionally substituted with 1-3 substituents selected from the group consisting of hydroxyl, halogen, C₁₋₆ alkyl, halogenated C₁₋₆ alkyl, and 3-7 membered cycloalkyl;
preferably, said Y is substituted with hydroxyl, Y may also be further optionally substituted with 1-3 substituents selected from the group consisting of amino, cyano, halogen, C₁₋₆ alkyl, halogenated C₁₋₆ alkyl, C₁₋₆ alkoxy, C₂₋₆ alkenyl, C₂₋₆ alkynyl, and cyclopropyl, and the substituent on Y may be further optionally substituted with 1-3 substituents selected from the group consisting of halogen, C₁₋₆ alkyl, cyclopropyl, trifluoromethyl, and difluoromethyl.

Further, Y is phenyl or 5-6 membered heteroaryl; preferably, Y is phenyl or 5-6 membered heteroaryl containing 1-2 N heteroatoms.

Further, Y is selected from the group consisting of phenyl, pyridinyl, and pyrimidinyl.

Furthermore, Y is selected from the group consisting of 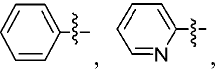 and

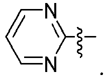

In any one of the above technical solutions, said Y is optionally substituted with 1-4 substituents selected from the group consisting of hydroxyl, amino, carboxyl, cyano, nitro, halogen, carbonyl, C₁₋₆ alkyl, halogenated C₁₋₆ alkyl, C₁₋₆ alkoxy, -S-C₁₋₆ alkyl, halogenated C₁₋₆ alkoxy, C₂₋₆ alkenyl, C₂₋₆ alkynyl, 3-7 membered heterocyclyl, 3-7 membered cycloalkyl, 5-7 membered cycloalkenyl, aryl, 5-7 membered heteroaryl, and sulfonyl; preferably, said Y is optionally substituted with 1-4 substituents selected from the group consisting of hydroxyl, amino, carboxyl, cyano, nitro, halogen, carbonyl, C₁₋₆ alkyl, trifluoromethyl, difluoromethyl, methoxy, -S-methyl, C₂₋₆ alkenyl, C₂₋₆ alkynyl, 4-6 membered heterocyclyl, cyclopropyl, and 5-6 membered heteroaryl; preferably, said Y is optionally substituted with 1-4 substituents selected from the group consisting of methyl, hydroxyl, cyano, cyclopropyl, halogen, methoxy, trifluoromethyl, vinyl, propenyl, ethynyl, propynyl, and -S-methyl.

In any one of the above technical solutions, the substituent on Y may be further optionally substituted with 1-3 substituents selected from the group consisting of hydroxyl, C₀₋₆ alkylamino, carboxyl, cyano, nitro, halogen, C₀₋₆ alkylcarbonyl, C₁₋₆ alkyl, halogenated C₁₋₆ alkyl, C₁₋₆ alkoxy, -S-C₁₋₆ alkyl, halogenated C₁₋₆ alkoxy, C₂₋₆ alkenyl, C₂₋₆ alkynyl, 3-6 membered heterocyclyl, 3-7 membered cycloalkyl, 5-6 membered cycloalkenyl, aryl, 5-7 membered heteroaryl, and C₀₋₆ alkylsulfonyl.

Further, the substituent on Y may be further optionally substituted with 1-3 substituents selected from the group consisting of hydroxyl, methylamino, dimethylamino, cyano, fluorine, chlorine, methylcarbonyl, methyl, ethyl, trifluoromethyl, difluoromethyl, methoxy, vinyl, propenyl, ethynyl, propynyl, 4-6 membered heterocyclyl, cyclopropyl, 5-6 membered heteroaryl, and C₀₋₆ alkylsulfonyl.

In any one of the above technical solutions, Y is optionally substituted with 1-4 substituents selected from the group consisting of hydroxyl, 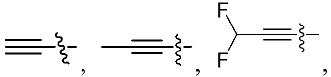 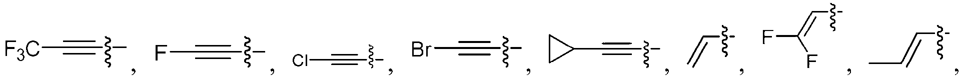 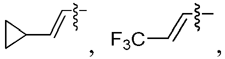 methyl, trifluoromethyl, halogen, cyano, and cyclopropyl.

In any one of the above technical solutions, said Y is substituted with hydroxyl, said Y may also be further optionally substituted with 1-3 substituents selected from the group consisting of amino, cyano, halogen, C₁₋₆ alkyl, halogenated C₁₋₆ alkyl, C₁₋₆ alkoxy, -S-C₁₋₆ alkyl, C₂₋₆ alkenyl, C₂₋₆ alkynyl, cyclopropyl, and sulfonyl, the substituent on Y may be further optionally substituted with 1-3 substituents selected from the group consisting of halogen, C₁₋₆ alkyl, 3-7 membered cycloalkyl, and halogenated C₁₋₆ alkyl.

Further, the substituent on Y may be further optionally substituted with 1-3 substituents selected from the group consisting of halogen, C₁₋₆ alkyl, cyclopropyl, trifluoromethyl, and difluoromethyl.

In one embodiment of the present disclosure, the compounds, or the deuterated compounds thereof, the stereoisomers thereof or the pharmaceutically acceptable salts thereof as described above are shown in Table 1.

**Table 1**

| No. | Structure | No. | Structure |
|---|---|---|---|
| 1 | 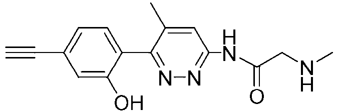 | 2 | 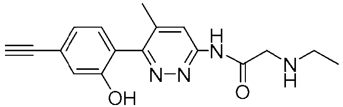 |
| 3 | 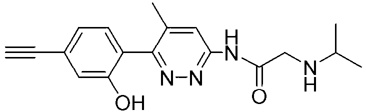 | 4 | 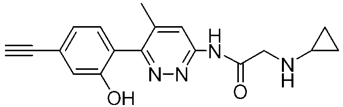 |
| 5 | 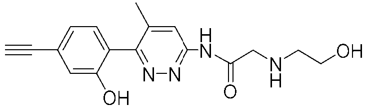 | 6 | 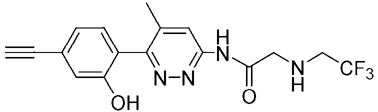 |
| 7 | 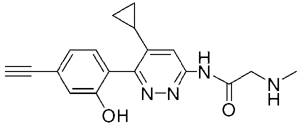 | 8 | 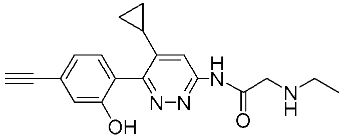 |
| 9 | 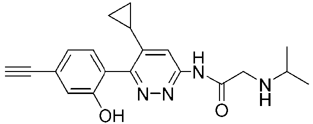 | 10 | 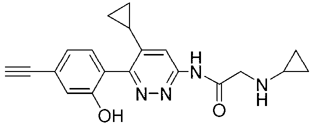 |
| 11 | 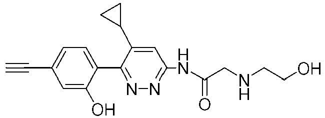 | 12 | 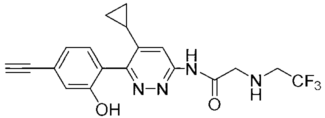 |
| 13 | 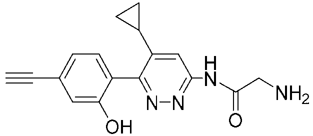 | 14 | 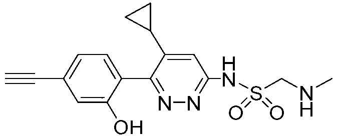 |
| 15 | 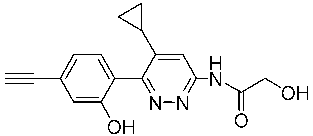 | 16 | 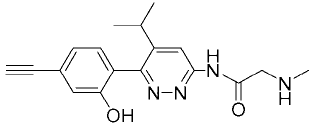 |
| 17 | 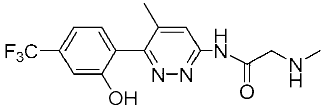 | 18 | 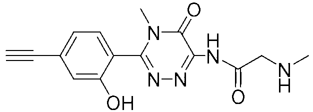 |
| 19 | 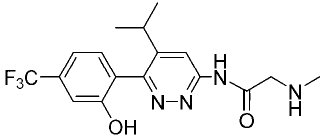 | 20 | 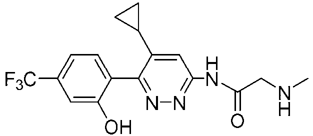 |
| 21 | 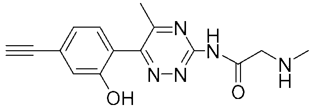 | 22 | 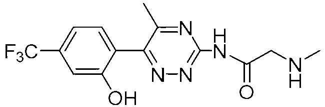 |
| 23 | 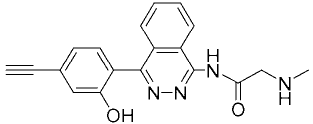 | 24 | 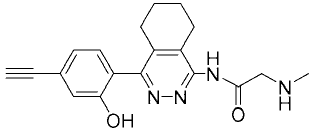 |
| 25 | 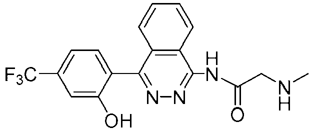 | 26 | 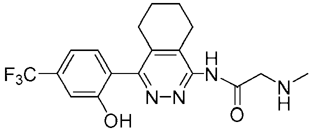 |
| 27 | 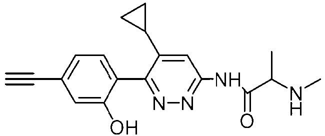 | 28 | 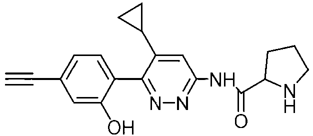 |
| 29 | 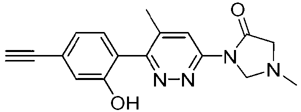 | 30 | 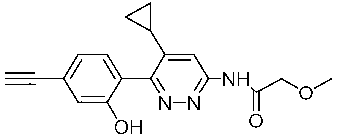 |
| 31 | 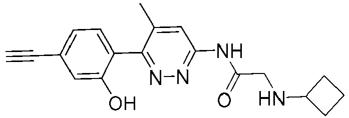 | 32 | 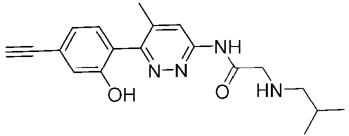 |
| 33 | 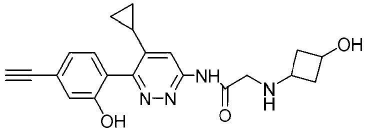 | 34 | 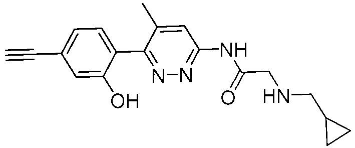 |
| 35 | 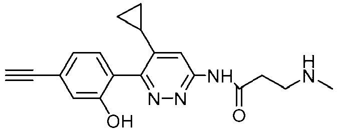 | 36 | 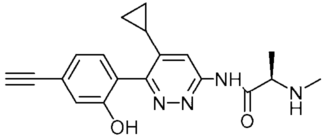 |
| 37 | 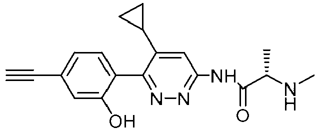 | 38 | 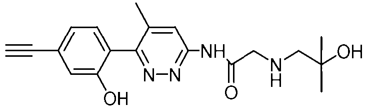 |
| 39 | 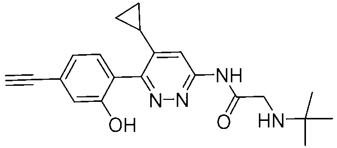 | 40 | 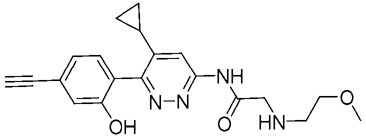 |
| 41 | 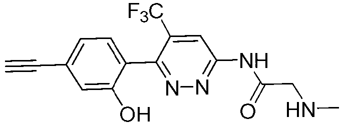 | 42 | 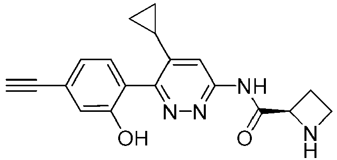 |
| 43 | 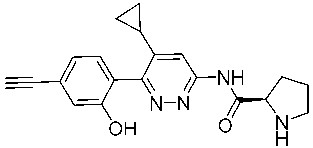 | 44 | 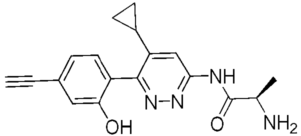 |
| 45 | 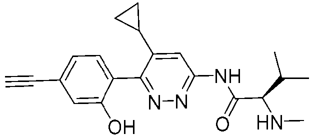 | 46 | 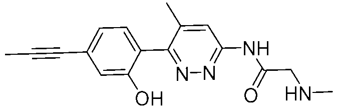 |
| 47 | 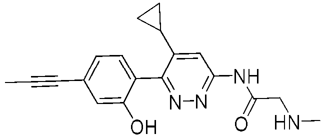 | 48 | 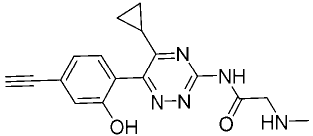 |
| 49 | 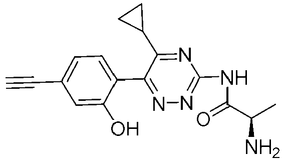 | 50 | 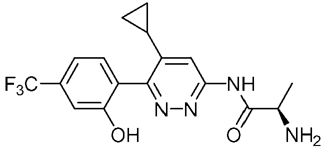 |
| 51 | 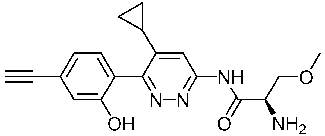 | 52 | 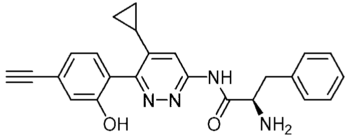 |
| 53 | 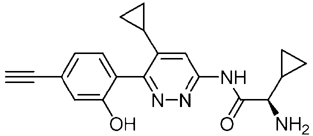 | 54 | 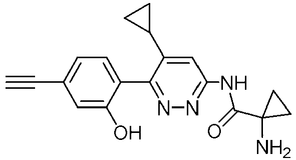 |
| 55 | 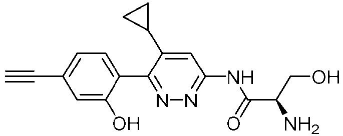 | 56 | 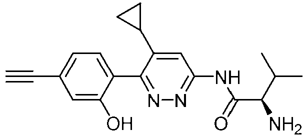 |
| 57 | 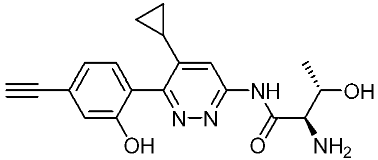 | 58 | 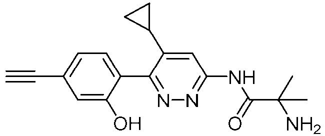 |
| 59 | 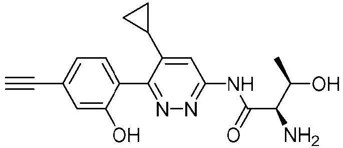 | 60 | 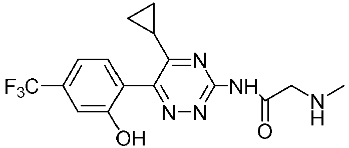 |
| 61 | 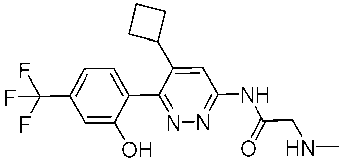 | 62 | 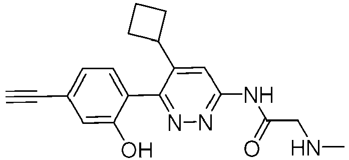 |
| 63 | 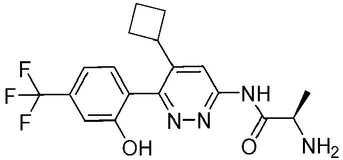 | 64 | 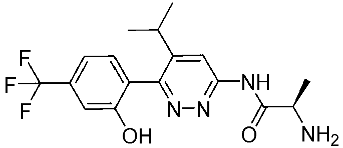 |
| 65 | 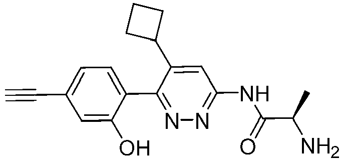 | 66 | 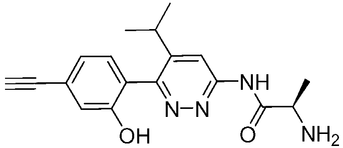 |
| 67 | 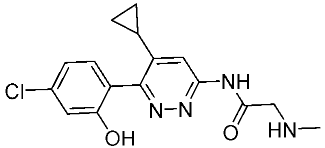 | 68 | 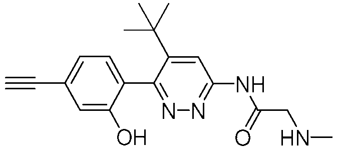 |
| 69 | 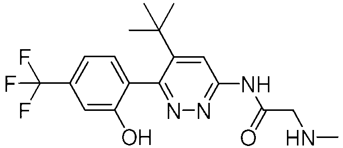 | 70 | 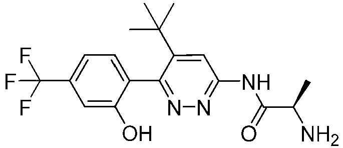 |
| 71 | 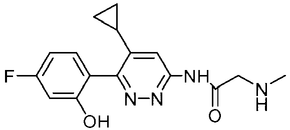 | 72 | 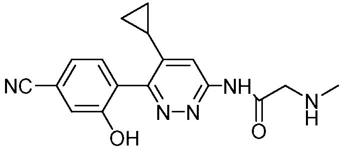 |
| 73 | 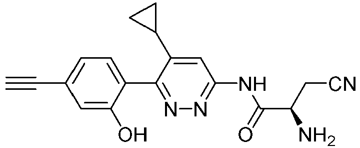 | 74 | 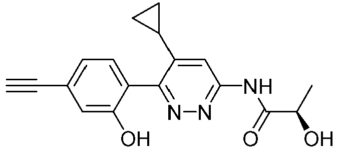 |
| 75 | 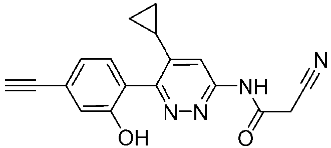 | 76 | 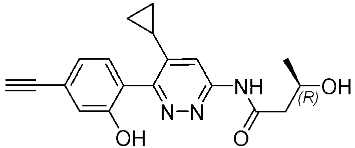 |
| 77 | 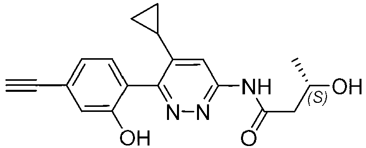 | 78 | 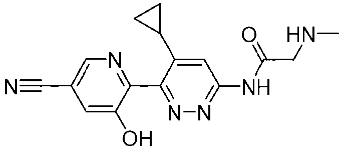 |
| 79 | 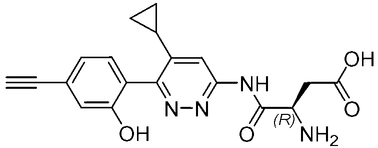 | 80 | 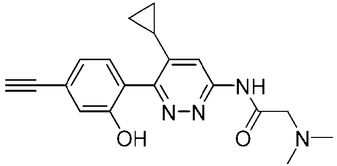 |

In one embodiment of the present disclosure, provided is a pharmaceutical composition, comprising a combination of any one of the compounds, or the deuterated compounds thereof, the stereoisomers thereof or the pharmaceutically acceptable salts thereof described above and one or more pharmaceutical carriers.

The present disclosure also provides the use of any one of the compounds, or the deuterated compounds thereof, the stereoisomers thereof or the pharmaceutically acceptable salts thereof described above, or the pharmaceutical composition described above in the manufacture of a medicament for preventing and/or treating an NLRP3 inflammasome-related disease.

The present disclosure also provides the use of any one of the compounds, or the deuterated compounds thereof, the stereoisomers thereof or the pharmaceutically acceptable salts thereof described above, or the pharmaceutical composition described above in the manufacture of a medicament for preventing and/or treating an inflammasome-related disease, an immune disease, an inflammatory disease, an autoimmune disease or an autoinflammatory disease.

### DETAILED DESCRIPTION OF THE DISCLOSURE

"Halogen" as described in the present disclosure refers to fluorine, chlorine, and bromine, and iodine.

"Hydroxyl" as described in the present disclosure refers to a group represented by -OH.

"Cyano" as described in the present disclosure refers to a group represented by -CN.

"Amino" as described in the present disclosure refers to a group represented by -NH₂.

"Carboxyl" as described in the present disclosure refers to a group represented by -COOH.

"Nitro" as described in the present disclosure refers to a group represented by -NO₂.

"Oxo" as described in the present disclosure refers to a group represented by =O.

"Thio" as described in the present disclosure refers to a group represented by =S.

"Ureido" as described in the present disclosure refers to a group represented by -HNCONH₂.

"Hydrazino" as described in the present disclosure refers to a group represented by -NHNH₂.

"C₁₋₆ alkyl" as described in the present disclosure refers to a linear or branched alkyl group derived from the removal of one hydrogen atom from a hydrocarbon molecule containing 1 to 6 carbon atoms, such as methyl, ethyl, *n*-propyl, isopropyl, *n*-butyl, isobutyl, *sec*-butyl, *tert-butyl, n*-pentyl, isopentyl, 2-methylbutyl, neopentyl, 1-ethylpropyl, *n*-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1-methyl-2-methylpropyl, and the like.

The suffix "ene" in "C₁₋₆ alkylene" as described in the present disclosure refers to a divalent group derived from the removal of two hydrogen atoms from a C₁₋₆ alkyl group.

"Halogenated C₁₋₆ alkyl" as described in the present disclosure refers to a C₁₋₆ alkyl group that is substituted with one or more kinds of halogen groups as defined above. Examples of halogenated C₁₋₆ alkyl include, but are not limited to, trifluoromethyl, difluoromethyl, fluoromethyl, trichloromethyl, 2,2,2-trifluoroethyl, 1,3- dibromopropan-2-yl, 3-bromo-2-fluoropropyl, and 1,4,4-trifluorobutan-2-yl.

"C₁₋₆ alkoxy" as described in the present disclosure refers to a group in which "C₁₋₆ alkyl" as previously defined is attached to a parent molecule via an oxygen atom, i.e., "C₁₋₆ alkyl-O-" group, such as methoxy, ethoxy, n-propoxy, isopropoxy, n-butoxy, tert-butoxy, n-pentoxy, neopentoxy, n-hexoxy, and the like.

"Halogenated C₁₋₆ alkoxy" as described in the present disclosure refers to a C₁-C₆ alkoxy group that is substituted with one or more kinds of halogen groups as defined above. Examples thereof include, but are not limited to, fluoromethoxy, chloromethoxy, trifluoromethoxy, trifluoroethoxy, fluoroethoxy and fluoropropoxy.

"C₂₋₆ alkenyl" as described in the present disclosure refers to a linear or branched alkenyl group derived from the removal of one hydrogen atom from an alkene molecule containing 2 to 6 carbon atoms and at least one carbon-carbon double bond, such as vinyl, propen-1-yl, propen-2-yl, buten-1-yl, buten-2-yl, buta-1,3-dien-1-yl, pent-1-en-3-yl, pent-2-en-1-yl, pent-3-en-1-yl, pent-3-en-2-yl, penta-1,3-dien-1-yl, penta-1,4-dien-3-yl, hex-1-en-3-yl, hexa-1,4-dien-1-yl. Preferably, "C₂₋₆ alkenyl" contains one carbon-carbon double bond.

"C₂₋₆ alkynyl" as described in the present disclosure refers to a linear or branched alkynyl group derived from the removal of one hydrogen atom from an alkyne molecule containing 2 to 6 carbon atoms and at least one carbon-carbon triple bond, such as ethynyl, propynyl, butynyl, pentynyl, hexynyl, and the like. Preferably, "C₂₋₆ alkynyl" contains one carbon-carbon triple bond.

"C₀₋₆ alkylamino" and "C₀₋₆ alkylsulfonyl" as described in the present disclosure refer to groups presented in the forms of C₀₋₆ alkyl-NH- and C₀₋₆ alkyl-S(O)₂-, respectively.

"5-12 membered ring" as described in the present disclosure includes carbocyclic rings or heterocyclic rings that are chemically feasible to form, such as 5-12 membered cycloalkyl, 5-7 membered cycloalkyl, 5-12 membered cycloalkenyl, 5-7 membered cycloalkenyl, 6-12 membered fused cycloalkyl, 5-12 membered heterocyclyl, 5-7 membered heterocyclyl, 6-12 membered fused heterocyclyl, aryl, 5-12 membered heteroaryl, 8-12 membered fused heteroaryl, 5-7 membered heteroaryl, and the like.

"3-12 membered cycloalkyl" as described in the present disclosure refers to a monovalent or divalent group (as required) derived from 3-12 membered cycloalkane (for example, 5-12 membered cycloalkyl), and it may be a monocyclic, bicyclic or polycyclic cycloalkyl system. Unless otherwise specified, it includes all monocyclic and fused cyclic rings that may be formed (such as 6-12 membered fused cycloalkyl), including those fused in condensed, spiro and bridged forms. Monocyclic system is generally a cyclic hydrocarbon group containing 3-12 carbon atoms, such as 3-8 or 3-6 carbon atoms. Examples of cycloalkyl include, but are not limited to, cyclopropyl, cyclobutyl, cyclopentyl, cyclohexyl, cycloheptyl, cyclooctyl, cyclopentane-1,3-diyl, cyclohexane-1,4-diyl, cycloheptane-1,4-diyl, and the like. Fused cyclic cycloalkyl groups include condensed cycloalkyl groups, bridged cycloalkyl groups, and spirocycloalkyl groups. Condensed cycloalkyl groups may be 6-11 membered condensed cycloalkyl groups such as 7-10 membered condensed cycloalkyl groups. Representative examples thereof include, but are not limited to, bicyclo[3.1.1]heptanyl, bicyclo[2.2.1]heptanyl, bicyclo[2.2.2]octanyl, bicyclo[3.2.2]nonanyl, bicyclo[3.3.1]nonanyl and bicyclo[4.2.1]nonanyl. Spirocycloalkyl groups may be 7-12 membered spirocycloalkyl groups, such as 7-11 membered spirocycloalkyl groups. Examples thereof include, but are not limited to, 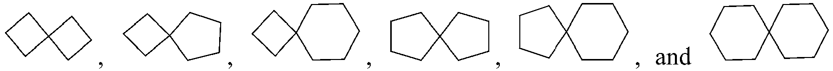 groups. Bridged cycloalkyl groups may be 6-10 membered bridged cycloalkyl groups, such as 7-10 membered bridged cycloalkyl groups. Examples thereof include, but are not limited to, 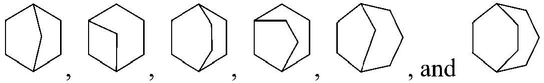 groups.

"3-7 membered cycloalkyl" as described in the present disclosure refers to a monovalent or divalent group (as required) derived from 3-7 membered cycloalkane. "3-7 membered cycloalkyl", such as "3-6 membered cycloalkyl" and "4-6 membered cycloalkyl", may be 3, 4, 5, 6, or 7 membered cycloalkyl. Examples of 3-7 membered cycloalkyl include cyclopropyl, cyclobutyl, cyclopentyl, and cyclohexyl.

"Cycloalkenyl" as described in the present disclosure refers to a group formed by having at least one double bond in a cycloalkyl group mentioned above. For example, it may be "3-12 membered cycloalkenyl", that is, it may have 3, 4, 5, 6, 7, 8, 9, 10, 11 or 12 ring-forming carbon atoms. Unless otherwise specified, a certain membered cycloalkenyl includes all monocyclic and fused cyclic rings that may be formed (including those fused in condensed, spiro and bridged forms). Cycloalkenyl may be 3-12 membered cycloalkenyl, 3-8 membered cycloalkenyl, 3-7 membered cycloalkenyl, 4-6 membered cycloalkenyl, 5-6 membered cycloalkenyl, 5-7 membered cycloalkenyl, 7-11 membered spirocycloalkenyl, 7-11 membered condensed cycloalkenyl, 6-11 membered bridged cycloalkenyl, and the like. Examples of cycloalkenyl may be listed as cyclobutenyl, cyclopentenyl, cyclopentadienyl, cyclohexenyl, cyclohexa-1,4-dien-1-yl, cycloheptenyl, cyclohepta-1,4-dien-1-yl, cyclooctenyl, cycloocta-1,5-diene-1-yl, and the like, but are not limited thereto.

"5-7 membered cycloalkenyl" as described in the present disclosure refers to a group formed by having at least one double bond in a 5-7 membered cycloalkyl group, such as cyclobutenyl, cyclopentenyl, cyclohexenyl, cycloheptenyl, and the like.

"3-14 membered heterocyclyl" as described in the present disclosure refers to a monovalent or divalent group (as required) derived from 3-14 membered heterocycloalkane, that is, a 3-14 membered nonaromatic cyclic group in which at least one ring carbon atom is replaced by a heteroatom selected from the group consisting of O, S, S(O), S(O)₂, C(O) and N, and preferably, 1-3 heteroatoms are contained. "3-14 membered heterocyclyl" (for example, 5-14 membered heterocyclyl, 5-12 membered heterocyclyl) includes monocyclic heterocyclyl systems, bicyclic heterocyclyl systems, or polycyclic heterocyclyl systems, in which one or more rings may be saturated or partially saturated and aromatic ring is not included. Unless otherwise specified, it includes all monocyclic and fused cyclic rings (both saturated rings and partially saturated rings) that may be formed (including those fused in condensed, spiro and bridged forms).

Monocyclic heterocyclyl may be 3-8 membered heterocyclyl, such as 5-7 membered heterocyclyl, 3-7 membered heterocyclyl, 3-6 membered heterocyclyl, 4-7 membered heterocyclyl, or 5-6 membered heterocyclyl; 3-8 membered nitrogen-containing heterocyclyl such as 4-7 membered nitrogen-containing heterocyclyl or 5-6 membered nitrogen-containing heterocyclyl; 3-8 membered saturated heterocyclyl such as 5-6 membered saturated heterocyclyl; and the like. Examples thereof include, but are not limited to, aziridinyl, oxiranyl, thiiranyl, azetidinyl, oxetanyl, thietanyl, tetrahydrofuranyl, tetrahydropyrrolyl, tetrahydrothienyl, imidazolidinyl, pyrazolidinyl, 1,2-oxazolidinyl, 1,3-oxazolidinyl, 1,2-thiazolidinyl, 1,3-thiazolidinyl, tetrahydro-2H-pyranyl, tetrahydro-2H-thiopyranyl, piperidinyl, piperazinyl, morpholinyl, 1,4-dioxanyl, 1,4-oxathianyl, 4,5-dihydroisoxazolyl, 4,5-dihydrooxazolyl, 2,5-dihydrooxazolyl, 2,3-dihydrooxazolyl, 3,4-dihydro-2H-pyrrolyl, 2,3-dihydro-1H-pyrrolyl, 2,5-dihydro-1H-imidazolyl, 4,5-dihydro-1H-imidazolyl, 4,5-dihydro-1H-pyrazolyl, 4,5-dihydro-3H-pyrazolyl, 4,5-dihydrothiazolyl, 2,5-dihydrothiazolyl, 2H-pyranyl, 4H-pyranyl, 2H-thiopyranyl, 4H-thiopyranyl, 2,3,4,5-tetrahydropyridinyl, 1,2-isoxazinyl, 1,4-isoxazinyl, 6H-1,3-oxazinyl, or the like.

Fused heterocyclyl (such as 6-12 membered fused heterocyclyl) includes condensed heterocyclyl, spiroheterocyclyl, and bridged heterocyclyl, which may be saturated, partially saturated, or unsaturated, but not aromatic. Fused heterocyclyl may be a 5-6 membered monocyclic heterocyclyl ring that is fused to phenyl, 5-6 membered monocyclic cycloalkyl, 5-6 membered monocyclic cycloalkenyl, 5-6 membered monocyclic heterocyclyl or 5-6 membered monocyclic heteroaryl.

Condensed heterocyclyl as described may be 6-12 membered condensed heterocyclyl such as 6-11 membered condensed heterocyclyl or 7-10 membered condensed heterocyclyl, 6-11 membered saturated condensed heterocyclyl, or 6-11 membered nitrogen-containing condensed heterocyclyl. Representative examples thereof include, but are not limited to, 3-azabicyclo[3.1.0]hexanyl, 3,6-diazabicyclo[3.2.0]heptanyl, 3,8-diazabicyclo[4.2.0]octanyl, 3,7-diazabicyclo[4.2.0]octanyl, octahydropyrrolo[3,4-c]pyrrolyl, octahydropyrrolo[3,4-b]pyrrolyl, octahydropyrrolo[3,4-b][1,4]oxazinyl, octahydro-1H-pyrrolo[3,4-c]pyridinyl, 2,3-dihydrobenzofuran-2-yl, 2,3-dihydrobenzofuran-3-yl, indolin-1-yl, indolin-2-yl, indolin-3-yl, 2,3-dihydrobenzothiophen-2-yl, octahydro-1H-indolyl, and octahydrobenzofuranyl.

Spiroheterocyclyl as described may be 6-12 membered spiroheterocyclyl such as 7-12 membered spiroheterocyclyl, 7-12 membered saturated spiroheterocyclyl, and 7-12 membered nitrogen-containing spiroheterocyclyl. Examples thereof include, but are not limited to, 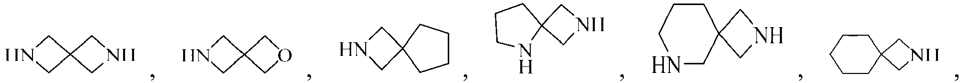 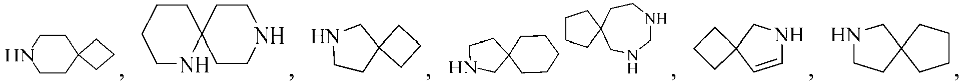 and

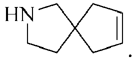

Bridged heterocyclyl as described may be 6-12 membered bridged heterocyclyl such as 6-10 membered bridged heterocyclyl (for example, 6-10 membered nitrogen-containing bridged heterocyclyl, in particular, 7-membered nitrogen-containing bridged heterocyclyl), and 7-10 membered bridged heterocyclyl. Examples thereof include, but are not limited to, 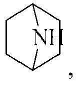 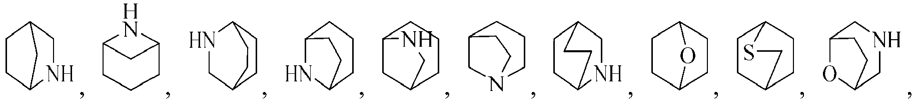 and

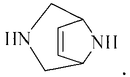

"Aryl" as described in the present disclosure refers to a monovalent or divalent (as required) cyclic aromatic group derived from an aromatic cyclohydrocarbon containing 6-14 carbon atoms, including phenyl, naphthyl, phenanthryl, and the like.

"5-14 membered heteroaryl" as described in the present disclosure refers to an aromatic 5-14 membered cyclic group in which at least one ring carbon atom is replaced by a heteroatom selected from the group consisting of O, S and N. "5-14 membered heteroaryl" may be 5, 6, 7, 8, 9, 10, 11, 12, 13, or 14-membered heteroaryl (preferably containing 1-3 heteroatoms), and meanwhile, the cases where the ring carbon atom or the ring sulfur atom is replaced by oxygen- or nitrogen-containing atomic group, for example, the carbon atom is replaced by C(O), or the sulfur atom is replaced by S(O) or S(O)₂, are encompassed. Heteroaryl includes monocyclic heteroaryl and fused heteroaryl. Unless otherwise specified, a certain membered heteroaryl includes all monocyclic and fused cyclic rings (both fully aromatic rings and partially aromatic rings) that may be formed. Monocyclic heteroaryl may be 5-7 membered heteroaryl such as 5-6 membered heteroaryl. Examples thereof include, but are not limited to, furanyl, imidazolyl, isoxazolyl, thiazolyl, isothiazolyl, oxadiazolyl, oxazolyl, pyridinyl, pyridazinyl, pyrimidinyl, pyrazinyl, pyrazolyl, pyrrolyl, tetrazolyl, thiadiazolyl, thienyl, triazol, and triazinyl.

In some embodiments, fused heteroaryl refers to a group formed by fusing a monocyclic heteroaryl ring to phenyl, cycloalkenyl, heteroaryl, cycloalkyl or heterocyclyl. In some embodiments, fused heteroaryl (such as 8-14 membered fused heteroaryl) may be 8-14 membered condensed heteroaryl such as 9-10 membered condensed heteroaryl. Examples include, but are not limited to, benzoimidazolyl, benzofuranyl, benzothienyl, benzooxadiazolyl, benzothiadiazolyl, benzothiazolyl, cinnolinyl, 5,6-dihydroquinolin-2-yl, 5,6-dihydroisoquinolin -1-yl, furanopyridinyl, indazolyl, indolyl, isoindolyl, isoquinolinyl, naphthyridinyl, purinyl, quinolinyl, 5,6,7,8-tetrahydroquinolin-2-yl, 5,6,7,8-tetrahydroquinolinyl, 5,6,7,8-tetrahydroquinolin-4-yl, 5,6,7,8-tetrahydroisoquinolin-1-yl, thienopyridinyl, 4,5,6,7-tetrahydro[c][1,2,5]oxadiazolyl and 6,7-dihydro[c][1,2,5]oxadiazol-4(5H)-onyl.

"Pharmaceutically acceptable salts" as described in the present disclosure refer to addition salts and solvates formed by using pharmaceutical acids and bases. Such pharmaceutical salts include the salts formed by using the following acids such as hydrochloric acid, phosphoric acid, hydrobromic acid, sulfuric acid, sulfurous acid, formic acid, toluenesulfonic acid, methanesulfonic acid, nitric acid, benzoic acid, citric acid, tartaric acid, maleic acid, hydroiodic acid, alkanoic acids (such as acetic acid, HOOC-(CH₂)ₙ-COOH (wherein n=0~4)), and the like. Such pharmaceutical salts also include salts formed by using the following bases such as sodium, potassium, calcium, ammonium, and the like. A variety of non-toxic pharmaceutical addition salts are known to those skilled in the art.

All numerical ranges as described in the present disclosure are intended to include the two endpoints of the range, all integers within the range, and subranges formed by these integers. For example, "3-7 membered" includes 3, 4, 5, 6, and 7-membered; "1-4" includes 1, 2, 3 and 4; "1-3" includes 1, 2 and 3.

The term "optional" or "optionally" refers to that the situation described subsequently may occur or may not occur, and this description includes both the occurrence and the nonoccurrence of said situation.

"Stereoisomers" of the compound as described in the present disclosure refer to isomers resulting from different spatial arrangements of atoms in a molecule. Enantiomers would be generated when an asymmetric carbon atom is present in a compound. Cis-trans isomers would be generated when a carbon-carbon double bond or a cyclic structure is present in a compound.

The term "tautomers" refer to a special type of functional group isomers where different functional group isomers are in dynamic equilibrium and may be transformed to each other rapidly. For example, when a ketone or an oxime is present, tautomers would be generated. Representative examples are for example keto-enol tautomers, phenol-keto tautomers, nitroso-oxime tautomers, imine-enamine tautomers, and the like. The enantiomers, diastereomers, racemic isomers, cis-trans isomers, tautomers, geometric isomers, epimers, and mixtures thereof of all compounds are included within the scope of the present disclosure.

In the chemical configurations of the compounds as described in the present disclosure, the bond "/" indicates that the configuration is not specified. That is, if the chemical structure has chiral isomers, the bond " 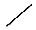 " may be " 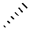 " or " 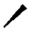 ", or simultaneously comprises both configurations of " 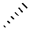 " and " 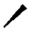 ". In the chemical structures of the compounds as described in the present disclosure, " 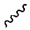 " represents a variable attachment point to the parent molecule.

-̅ -̅ -̅ -̅ -̅ is a single bond or a double bond.

"Deuterated" means that one or more hydrogen atoms in a compound or group are substituted with deuterium atoms.

### General preparation methods of the compounds of the present disclosure

Unless otherwise specified, all raw materials or intermediates involved in the preparation methods for which no preparation method is provided are commercially available or may be synthesized using methods known in published literatures. Suitable post-processing methods described in the preparation methods may include one or a combination of two or more of the following conventional post-processing methods, such as quenching with water, concentration, adjustment of pH value, extraction with a suitable solvent (for example, ethyl acetate, dichloromethane), filtration, drying, and the like. Suitable purification methods described in the preparation methods may include one or a combination of two or more of the following purification methods, such as silica gel column chromatography, preparative thin-layer chromatography, reversed-phase preparative chromatography, recrystallization, slurrying, and the like.

The compounds of the present disclosure may be prepared according to Reaction Scheme 1 as below. 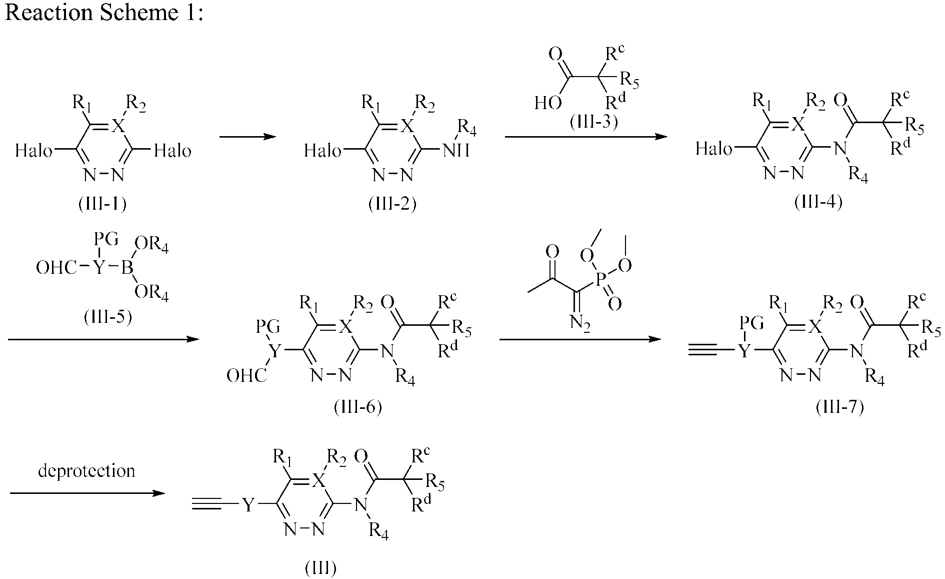 wherein,
X is C or N;
R₁, R₂ and R₄ are as defined above, when X is N, R₂ is absent;
Halo is halogen or other leaving group such as a sulfonate group or an alkylsulfonyl;
PG is a suitable protecting group;
R₄ in the intermediate represented by formula (III-5) may form a 5-7 membered heterocycle with B and O atoms;
R^{c}, R^{d}, R₅ and Y are as defined above.

The preparation methods for the compounds represented by the general formulae as shown in Reaction Scheme 1 are provided below.

The intermediate represented by formula (III-2) may be obtained via an aromatic nucleophilic substitution reaction of the intermediate represented by formula (III-1) and a corresponding amine under heating conditions.

The intermediate represented by formula (III-2) and the intermediate represented by formula (III-3) undergo a condensation reaction in the presence of a base (for example, triethylamine or N,N-diisopropylethylamine) and a suitable condensation reagent in peptide synthesis (for example, 2-(7-azabenzotriazol-1-yl)-*N,N,N',N'*-tetramethyluronium hexafluorophosphate, N,N,N,N-tetramethylchloroformamidinium hexafluorophosphate or isopropyl chloroformate), so as to afford the intermediate represented by formula (III-4).

Under the protection of an inert gas (for example, nitrogen), the intermediate represented by formula (III-4) and the intermediate represented by formula (III-5) were heated to a suitable temperature (for example, 90°C~110°C) to undergo a Suzuki coupling reaction in the presence of a suitable catalyst (for example, tetrakis(triphenylphosphine)palladium or [1,1'-bis(diphenylphosphino)ferrocene]palladium(II) dichloride) and a suitable base (for example, potassium carbonate, sodium carbonate, sodium bicarbonate, or the like), so as to afford the intermediate represented by formula (III-6).

The intermediate represented by formula (III-6) reacts with the reagent dimethyl (1-diazo-2-oxopropyl)phosphonate in the presence of a suitable base (for example, potassium carbonate), so as to generate the intermediate represented by formula (III-7).

The intermediate represented by formula (III-7) is deprotected in the presence of a suitable deprotection reagent (for example, a solution of hydrogen chloride in 1,4-dioxane, a solution of hydrogen chloride in ethanol, a solution of hydrogen chloride in ethyl acetate, boron tribromide, piperidine, diethylamine, trifluoroacetic acid, or the like), thus affording the compound of the present disclosure.

In some embodiments, the above intermediate represented by formula (III-7) may be prepared according to the following Reaction Scheme 2. 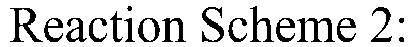 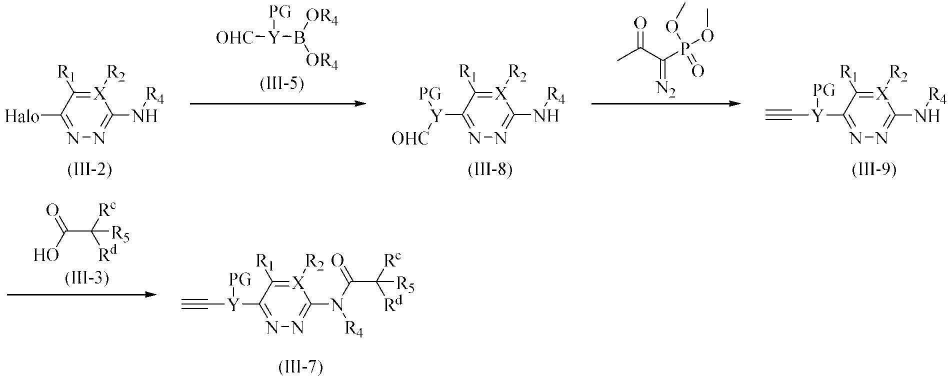 wherein X, R₁, R₂, Halo, R₄, PG, R^{c}, R^{d}, R₅ and Y are as defined above. The preparation method for the intermediate represented by formula (III-7) in Reaction Scheme 2 is provided below.

Under the protection of an inert gas (for example, nitrogen), the intermediate represented by formula (III-2) and the intermediate represented by formula (III-5) were heated to a suitable temperature (for example, 90°C~110°C) to undergo a Suzuki coupling reaction in the presence of a suitable catalyst (for example, tetrakis(triphenylphosphine)palladium or [1,1'-bis(diphenylphosphino)ferrocene]palladium(II) dichloride) and a suitable base (for example, potassium carbonate, sodium carbonate, sodium bicarbonate, or the like), so as to afford the intermediate represented by formula (III-8).

The intermediate represented by formula (III-8) reacts with the reagent dimethyl (1-diazo-2-oxopropyl)phosphonate, in the presence of a suitable base (for example, potassium carbonate ), so as to generate the intermediate represented by formula (III-9).

The intermediate represented by formula (III-9) and the intermediate represented by formula (III-3) undergo a condensation reaction in the presence of a base (for example, triethylamine or N,N-diisopropylethylamine) and a suitable condensation reagent in peptide synthesis (for example, 2-(7-azabenzotriazol-1-yl)-*N,N,N',N'*-tetramethyluronium hexafluorophosphate, *N,N,N',N'*-tetramethylchloroformamidinium hexafluorophosphate or isopropyl chloroformate), so as to afford the intermediate represented by formula (III-7).

The compounds of the present disclosure may also be prepared according to Reaction Scheme 3 as below. 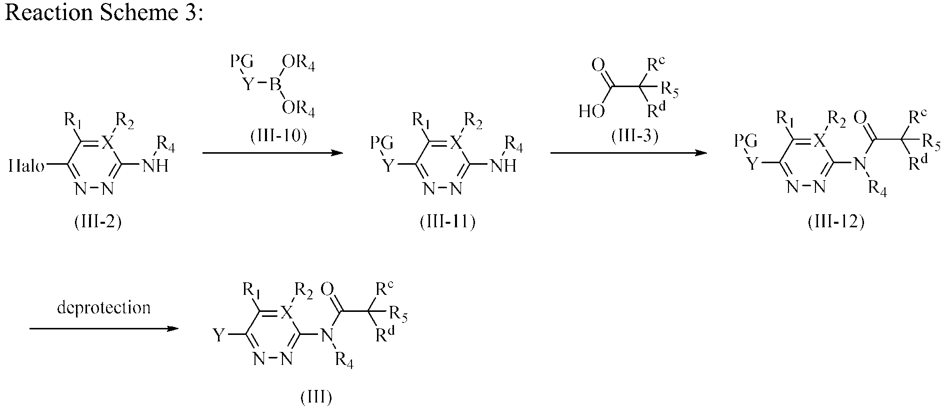 wherein X, R₁, R₂, Halo, R₄, PG, R^{c}, R^{d}, R₅ and Y are as defined above. The preparation methods for the compounds represented by the general formulae as shown in Reaction Scheme 3 are provided below.

Under the protection of an inert gas (for example, nitrogen), the intermediate represented by formula (III-2) and the intermediate represented by formula (III-10) were heated to a suitable temperature (for example, 90°C~110°C) to undergo a Suzuki coupling reaction in the presence of a suitable catalyst (for example, tetrakis(triphenylphosphine)palladium or [1,1'-bis(diphenylphosphino)ferrocene]palladium(II) dichloride) and a suitable base (for example, potassium carbonate, sodium carbonate, sodium bicarbonate, or the like), so as to afford the intermediate represented by formula (III-11).

The intermediate represented by formula (III-11) and the intermediate represented by formula (III-3) undergo a condensation reaction in the presence of a base (for example, triethylamine or N,N-diisopropylethylamine) and a suitable condensation reagent in peptide synthesis (for example, 2-(7-azabenzotriazol-1-yl)-*N,N,N',N'*-tetramethyluronium hexafluorophosphate, *N,N,N',N'*-tetramethylchloroformamidinium hexafluorophosphate or isopropyl chloroformate), so as to afford the intermediate represented by formula (III-12).

The intermediate represented by formula (III-12) is deprotected in the presence of a suitable deprotection reagent (for example, a solution of hydrogen chloride in 1,4-dioxane, a solution of hydrogen chloride in ethanol, a solution of hydrogen chloride in ethyl acetate, boron tribromide, piperidine, diethylamine, trifluoroacetic acid, or the like), thus affording the compound of the present disclosure.

In some embodiments, the intermediate represented by formula (III-12) may also be prepared according to Reaction Scheme 4 as below. 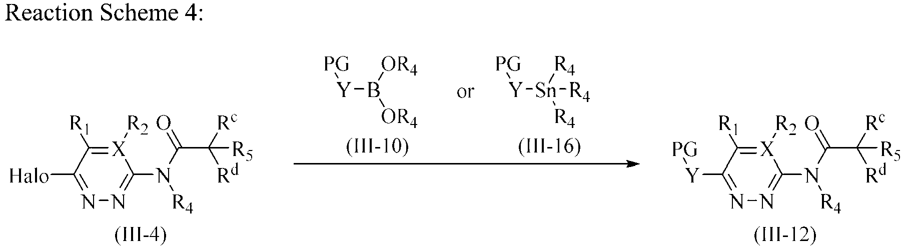 wherein X, R₁, R₂, Halo, R₄, PG, R^{c}, R^{d}, R₅ and Y are as defined above. The preparation method for the intermediate represented by formula (III-12) in Reaction Scheme 4 is provided below.

Under the protection of an inert gas (for example, nitrogen), the intermediate represented by formula (III-4) and the intermediate represented by formula (III-10) or the intermediate represented by formula (III-16) were heated to a suitable temperature (for example, 90°C~110°C) to undergo a Suzuki coupling reaction or a Stille coupling reaction in the presence of a suitable catalyst (for example, [1,1'-bis(diphenylphosphino)ferrocene]palladium(II) dichloride, tetrakis(triphenylphosphine)palladium or tetrakis(triphenylphosphine)palladium, and cuprous iodide) and a suitable base (for example, potassium carbonate, sodium carbonate, sodium bicarbonate, cesium fluoride, or the like), so as to afford the intermediate represented by formula (III-12).

The compounds of the present disclosure may also be prepared according to Reaction Scheme 5 as below. 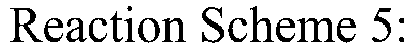 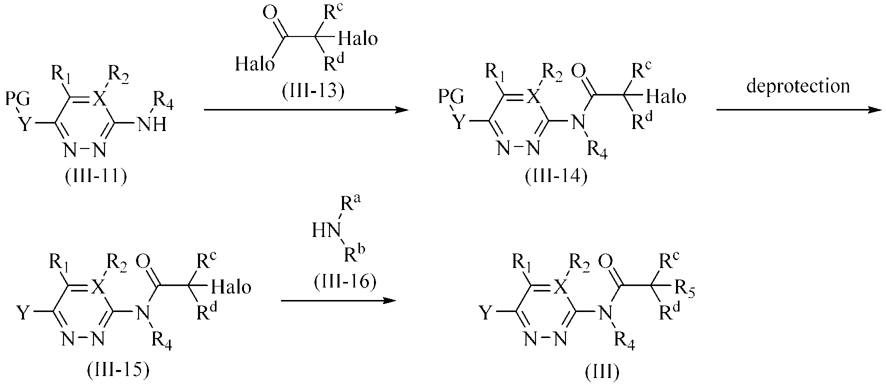 wherein X, R₁, R₂, Halo, R₄, PG, R^{c}, R^{d}, R^{a}, R^{b}, R₅ and Y are as defined above. The preparation methods for the compounds represented by the general formulae as shown in Reaction Scheme 5 are provided below.

The intermediate represented by formula (III-11) and the intermediate represented by formula (III-13) undergo a condensation reaction in the presence of a base (for example, triethylamine or N,N-diisopropylethylamine), so as to afford the intermediate represented by formula (III-14).

The intermediate represented by formula (III-14) is deprotected in the presence of a suitable deprotection reagent (for example, a solution of hydrogen chloride in 1,4-dioxane, a solution of hydrogen chloride in ethanol, a solution of hydrogen chloride in ethyl acetate, boron tribromide, trifluoroacetic acid, or the like), thus affording the intermediate represented by formula (III-15).

The intermediate represented by formula (III-15) and the intermediate represented by formula (III-16) undergo a substitution reaction in the presence of a suitable base (for example, triethylamine or N,N-diisopropylethylamine), thus affording the compound of the present disclosure.

### Advantageous effects of the present disclosure

Studies have found that the compounds, or the deuterated compounds thereof, the stereoisomers thereof or the pharmaceutically acceptable salts thereof as provided by the present disclosure exhibit good inhibitory activity against the NLRP3 inflammasome. Therefore, the compounds of the present disclosure may be used for preventing and/or treating NLRP3 inflammasome-related diseases. Meanwhile, the compounds of the present disclosure are metabolically stable, exhibit low toxicity, and possess favorable pharmacokinetic properties and higher safety.

### DETAILED DESCRIPTION

In order to make the objectives, technical solutions and advantages of the present disclosure clearer, the present disclosure is further described in detail below. Apparently, the examples as described are merely a part of the examples of the present disclosure, rather than all the examples. All other examples obtained by those of ordinary skill in the art based on the examples of the present disclosure without making creative labor are encompassed within the protection scope of the present disclosure.

The abbreviations and English expressions used in the present disclosure have the following meanings.

"THF" refers to tetrahydrofuran; "DMF" refers to N,N-dimethylformamide; "MeOH" refers to methanol; "EA" refers to ethyl acetate; "DCM" refers to dichloromethane; "PE" refers to petroleum ether;

"FBS" refers to fetal bovine serum; "PBS" refers to phosphate buffered saline solution; "PMA" refers to phorbol ester; "LPS" refers to lipopolysaccharide; "Nigericin" refers to nigericin.

### Intermediate preparation example 1: Synthesis of 6-chloro-5-cyclopropylpyridazin-3-amine

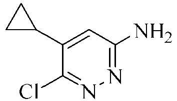

### Step 1: Synthesis of 3,6-dichloro-4-cyclopropylpyridazine

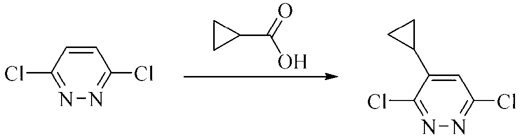

3,6-Dichloropyridazine (20.0 g, 134.2 mmol, 1.0 eq) was dissolved in water (200 mL), and cyclopropanecarboxylic acid (11.56 g, 134.2 mmol, 1.0 eq) and silver nitrate (22.8 g, 134.2 mmol, 1.0 eq) were added thereto. At 50°C, concentrated sulfuric acid (21.6 mL, 402.6 mmol, 3.0 eq) was added dropwise to the reaction solution. After completion of the dropwise addition, the resultant was heated to 60°C, and an aqueous solution (200 mL) of ammonium persulfate (91.88 g, 402.6 mmol, 3.0 eq) was added dropwise to the reaction solution. After completion of the dropwise addition, the resultant was heated to 70°C and reacted for 30 min. TLC monitoring showed the completion of the reaction. The reaction solution was adjusted to have a pH value of 8 with 1 mol/L aqueous sodium hydroxide solution. The resultant was extracted with ethyl acetate (400 mL×2). The organic phases were combined, dried, and concentrated. The crude product was first subjected to silica gel column chromatography (petroleum ether:ethyl acetate=50: 1). After standing, a large amount of solid precipitated from the resulting product, which was then filtered. The filter cake was slurried with a mixed solvent of petroleum ether and ethyl acetate, so as to afford the product (17 g, yield: 67.1%).

### Step 2: Synthesis of 6-chloro-5-cyclopropylpyridazin-3-amine

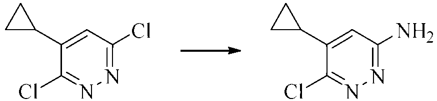

3,6-Dichloro-4-cyclopropylpyridazine (10.0 g, 52.90 mmol, 1.0 eq.) and aqueous ammonia (30.0 mL) were added to a sealed tube, and the resultant was stirred at 140°C for 14 hours. TLC monitoring showed the completion of the reaction. Water (50.0 mL) was added, and the resultant was extracted with dichloromethane (100.0 mL). The organic phase was dried over anhydrous sodium sulfate and filtered. The filtrate was concentrated under reduced pressure. The crude product was purified by silica gel column chromatography (dichloromethane:methanol=100:1-40:1), so as to afford the product (4.3 g, yield: 47.9%).

### Intermediate preparation example 2: Synthesis of 6-chloro-5-isopropylpyridazin-3-amine

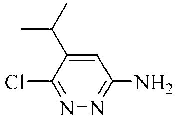

### Step 1: Synthesis of 3,6-dichloro-4-isopropylpyridazine

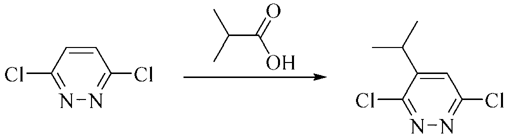

The product (10.1 g, yield: 78.9%) was prepared in accordance with the method in Step 1 of **Intermediate preparation example** 1.

### Step 2: Synthesis of 6-chloro-5-isopropylpyridazin-3-amine

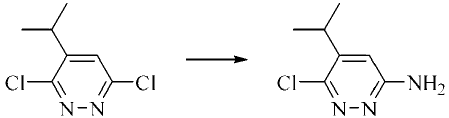

The compound was prepared in accordance with the method in Step 2 of **Intermediate preparation example** 1, and the crude product was purified by silica gel column chromatography (dichloromethane:methanol=130:1-100:1), so as to afford the product (2.0 g, yield: 44.4%).

### Intermediate preparation example 3: Synthesis of 6-chloro-5-cyclobutylpyridazin-3-amine

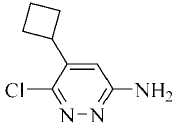

### Step 1: Synthesis of 3,6-dichloro-4-cyclobutylpyridazine

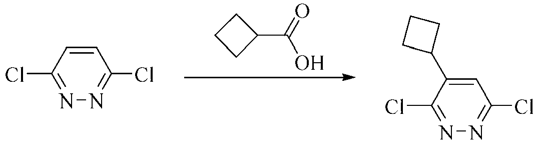

The product (9.8 g, yield: 72.0%) was prepared in accordance with the method in Step 1 of **Intermediate preparation example 1.**

### Step 2: Synthesis of 6-chloro-5-cyclobutylpyridazin-3-amine

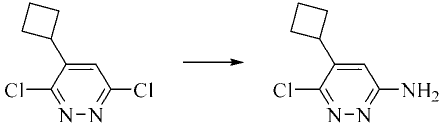

The compound was prepared in accordance with the method in Step 2 of **Intermediate preparation example 1,** and the crude product was purified by silica gel column chromatography (dichloromethane:methanol=130:1-100:1), so as to afford the product (1.5 g, yield: 33.3%).

### Intermediate preparation example 4: Synthesis of 6-chloro-5-cyclobutylpyridazin-3-amine

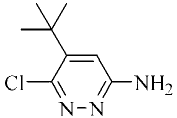

### Step 1: Synthesis of 4-(tert-butyl)-3,6-dichloropyridazine

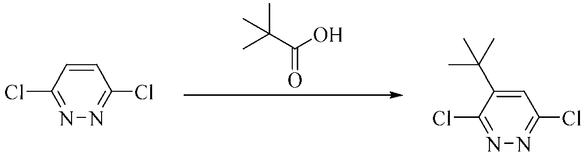

The compound was prepared in accordance with the method in Step 1 of **Intermediate preparation example** 1, the crude product was purified by silica gel column chromatography (PE:EA=20:1), so as to afford the product (23.0 g, yield: 66.8%).

### Step 2: Synthesis of 5-(tert-butyl)-6-chloropyridazin-3-amine

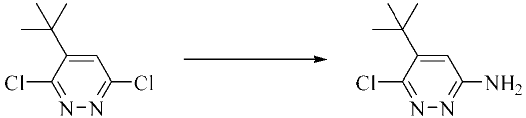

The compound was prepared in accordance with the method in Step 2 of **Intermediate preparation example 1,** and the crude product was purified by silica gel column chromatography (DCM:MeOH=200:1~50:1), so as to afford the product (14.2 g, yield: 68.2%).

### Intermediate preparation example 5: Synthesis of 3-(ethoxymethoxy)-4-(4,4,5,5-tetramethyl-1,3,2-dioxaborolan-2-yl)benzaldehyde

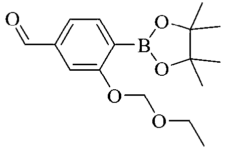

### Step 1: Synthesis of 4-bromo-3-hydroxybenzaldehyde

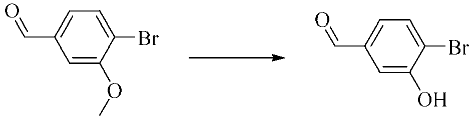

4-Bromo-3-methoxybenzaldehyde (10.0 g, 46.5 mmol, 1.0 eq) was added to a 40 wt% aqueous HBr solution (60 mL), and the resultant was heated to 100°C and reacted for 20 hours. The resultant was cooled to room temperature, water was added thereto, and the resultant was extracted with EA (50 mL×2). The organic phase was dried over anhydrous sodium sulfate and filtered. The filtrate was concentrated under reduced pressure. The crude product was purified by silica gel column chromatography (PE:EA=50:1~5:1), so as to afford the product (7.80 g, yield: 83.4%).

### Step 2: Synthesis of 4-bromo-3-(ethoxymethoxy)benzaldehyde

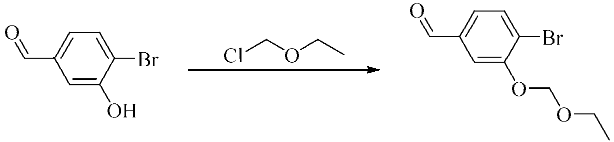

4-Bromo-3-hydroxybenzaldehyde (7.80 g, 38.8 mmol, 1.0 eq) was dissolved in THF (100 mL), and the resultant was cooled to 0°C. 60 wt% NaH (2.33 g, 58.2 mmol, 1.5 eq) was added in portions. The resultant was stirred for 20 minutes, chloromethyl ethyl ether (5.50 g, 86.2 mmol, 1.5 eq) was then added, and the reaction was allowed to proceed for 2 hours. The reaction was quenched by adding saturated aqueous NH₄Cl solution, and the resultant was subjected to liquid separation. The aqueous phase was extracted with EA (30 mL×2). The organic phases were combined, dried over anhydrous sodium sulfate, and filtered. The filtrate was concentrated under reduced pressure. The crude product was purified by silica gel column chromatography (PE:EA=50:1~5:1), so as to afford the product (7.60 g, yield: 75.6%).

### Step 3: Synthesis of 3-(ethoxymethoxy)-4-(4,4,5,5-tetramethyl-1,3,2-dioxaborolan-2-yl)benzaldehyde

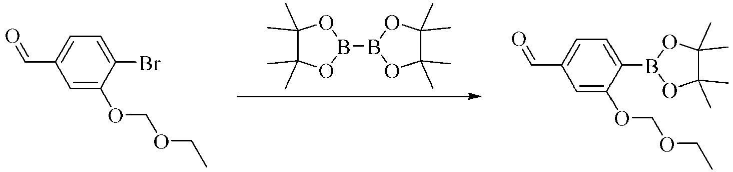

4-Bromo-3-(ethoxymethoxy)benzaldehyde (5.00 g, 19.3 mmol, 1.0 eq), bis(pinacolato)diboron (7.35 g, 29.0 mmol, 1.5 eq), Pd(dppf)Cl₂ (1.41 g, 1.93 mmol, 0.1 eq) and KOAc (3.79 g, 38.6 mmol, 2.0 eq) were sequentially added to 1,4-dioxane (50 mL). Under nitrogen protection, the resultant was heated to 100°C and reacted for 20 hours. The resultant was cooled to room temperature, water (50 mL) was added thereto, and the resultant was extracted with EA (50 mL×2). The organic phase was dried over anhydrous sodium sulfate and filtered. The filtrate was concentrated under reduced pressure. The crude product was purified by silica gel column chromatography (PE:EA=20:1~5:1), so as to afford the product (5.20 g, yield: 88.0%).

### Intermediate preparation example 6: Synthesis of (2-(ethoxymethoxy)-4-(trifluoromethyl)phenyl)boronic acid

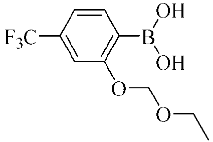

### Step 1: Synthesis of 1-bromo-2-(ethoxymethoxy)-4-(trifluoromethyl)benzene

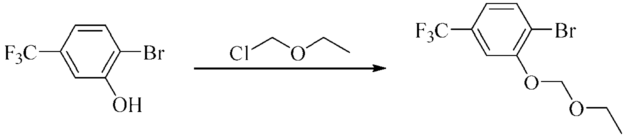

2-Bromo-5-trifluoromethylphenol (5.00 g, 20.7 mmol, 1.0 eq) was dissolved in THF (50 mL), and the resultant was cooled to 0°C. NaH (60%, 2.94 g, 31.1 mmol, 1.5 eq) was added in portions. The resultant was stirred for 20 minutes, and chloromethyl ethyl ether (1.24 g, 31.1 mmol, 1.5 eq) was then added. After reacting for 4 hours, the reaction was quenched by adding saturated aqueous NH₄Cl solution, and the resultant was extracted with EA (50 mL×2). The organic phase was dried over anhydrous sodium sulfate and filtered. The filtrate was concentrated under reduced pressure, so as to afford the product (6.08 g, yield: 98.0%).

### Step 2: Synthesis of (2-(ethoxymethoxy)-4-(trifluoromethyl)phenyl)boronic acid

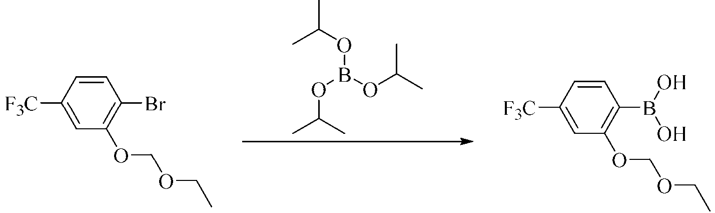

1-Bromo-2-(ethoxymethoxy)-4-(trifluoromethyl)benzene (3.00 g, 10.0 mmol, 1.0 eq) and triisopropyl borate (2.82 g, 15.0 mmol, 1.5 eq) were dissolved in THF (30 mL). Under nitrogen protection, the resultant was cooled to -60°C, *n*-butyllithium (1.6 mol/L solution in THF, 9.4 mL, 15.0 mmol, 1.5 eq) was then added dropwise, and the reaction was allowed to proceed for 3 hours. The reaction was quenched by adding saturated aqueous NH₄Cl solution, and the resultant was extracted with EA (50 mL). The organic phase was dried over anhydrous sodium sulfate and filtered. The filtrate was concentrated under reduced pressure. The crude product was slurried with petroleum ether, so as to afford the product (2.20 g, yield: 83.1%).

### Intermediate preparation example 7: Synthesis of (2-methoxy-4-(propyn-1-yl)phenyl)boronic acid

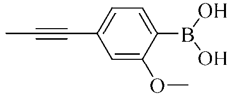

### Step 1: Synthesis of 1-bromo-2-methoxy-4-(propyn-1-yl)benzene

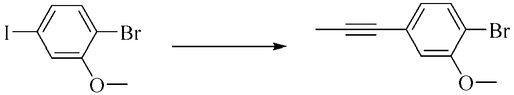

2-Bromo-5-iodoanisole (6.7 g, 21.41 mmol, 1.0 eq) was dissolved in tetrahydrofuran (60 mL), N,N-diisopropylethylamine (4.15 g, 32.12 mmol, 1.5 eq), cupric iodide (815 mg, 4.28 mmol, 0.2 eq) and bis(triphenylphosphine)palladium dichloride (1.5 g, 2.14 mmol, 0.1 eq) were sequentially added thereto. Under nitrogen protection, 1 mol/L solution of propyne in tetrahydrofuran (23.6 mL, 23.6 mmol, 1.1 eq) was added dropwise thereto, and the resultant was reacted at 25°C for 4 h. TLC monitoring showed the completion of the reaction. The reaction solution was added with saturated aqueous ammonium chloride solution (150 mL), and was extracted with ethyl acetate (100 mL×2). The organic phases were combined, dried over anhydrous sodium sulfate, and concentrated. The crude product was purified by silica gel column chromatography (petroleum ether:ethyl acetate=100:1), so as to afford the product (4.34 g, yield: 90.0%).

### Step 2: Synthesis of intermediate (2-methoxy-4-(propyn-1-yl)phenyl)boronic acid

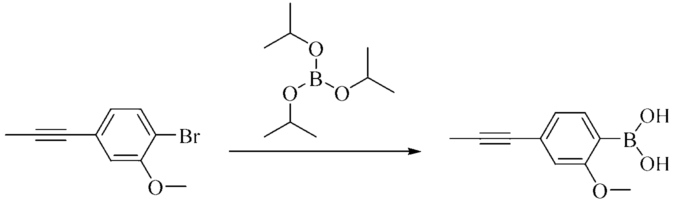

1-Bromo-2-methoxy-4-(propyn-1-yl)benzene (3.34 g, 14.84 mmol, 1.0 eq) and triisopropyl borate (3.35 g, 17.81 mmol, 1.2 eq) were dissolved in tetrahydrofuran (35 mL). At -65°C, under nitrogen protection, 2.5 mol/L solution of n-butyllithium in tetrahydrofuran (7.2 mL, 17.81 mmol, 1.2 eq) was slowly added dropwise thereto, and the resultant was reacted at 25°C for 2 hours. TLC monitoring showed the completion of the reaction. The reaction was quenched by pouring saturated aqueous ammonium chloride solution (40 mL) into the reaction solution, and the resultant was extracted with ethyl acetate (50 mL×3). The organic phases were combined and concentrated. The crude product was slurried with petroleum ether (5 mL) and filtered, and the filter cake *per se* is the product (1.5 g, yield: 53.2%).

### Intermediate preparation example 8: Synthesis of (2-(ethoxymethoxy)-4-fluorophenyl)boronic acid

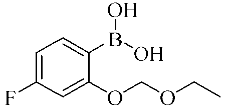

### Step 1: Synthesis of 1-bromo-2-(ethoxymethoxy)-4-fluorobenzene

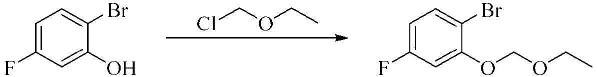

2-Bromo-5-fluorophenol (9.0 g, 47.12 mmol, 1.0 eq.) was added to tetrahydrofuran (40.0 mL). In an ice-water bath, sodium hydride (60%) (2.8 g, 70.68 mmol, 1.5 eq.) was added in portions. After reacting for 1 hour, chloromethyl ethyl ether (6.7 g, 70.68 mmol, 1.5 eq.) was added. TLC monitoring showed the completion of the reaction. Ethyl acetate (100.0 mL) was added, and the resultant was washed with water (100.0 mL). The organic phase was dried over anhydrous sodium sulfate and filtered. The filtrate was concentrated under reduced pressure. The crude product was purified by silica gel column chromatography (petroleum ether:ethyl acetate=100:1), so as to afford the product (9.7 g, yield: 82.9%).

### Step 2: Synthesis of (2-(ethoxymethoxy)-4-fluorophenyl)boronic acid

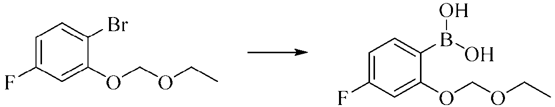

1-Bromo-2-(ethoxymethoxy)-4-fluorobenzene (9.0 g, 36.13 mmol, 1.0 eq.) and triisopropyl borate (10.2 g, 54.20 mmol, 1.5 eq.) were added to tetrahydrofuran (50.0 mL), and the resultant was cooled to -70°C. n-Butyllithium (2.5 mol/L, 21.0 mL) was added dropwise, and the resultant was warmed to room temperature and reacted for 2 hours. TLC monitoring showed the completion of the reaction. Ethyl acetate (100.0 mL) was added, and the resultant was washed with saturated aqueous ammonium chloride solution (100.0 mL). The organic phase was dried over anhydrous sodium sulfate and filtered. The filtrate was concentrated under reduced pressure, so as to afford the product (6.1 g, yield: 79.2%).

### Example 1: Synthesis of N-(5-cyclopropyl-6-(4-ethynyl-2-hydroxyphenyl)pyridazin-3-yl)-2-(methylamino)acetami de (Compound 7)

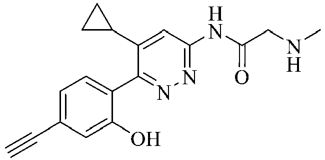

### Step 1: Synthesis of 6-chloro-5-cyclopropylpyridazin-3-amine

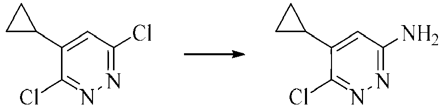

3,6-Dichloro-4-cyclopropylpyridazine (9.0 g, 47.60 mmol, 1.0 eq.) and aqueous ammonia (28%) (3.0 g, 238.0 mmol, 5.0 eq.) were mixed in a sealed tube and reacted at 140°C for 14 hours. The completion of the reaction was monitored by TLC. Dichloromethane (100.0 mL) was added, and the resultant was washed with water (50.0 mL). The organic phase was dried and filtered. The filtrate was concentrated under reduced pressure. The crude product was purified by silica gel column chromatography (dichloromethane:methanol=200:1-100:1), so as to afford the product (3.5 g, yield:43.7%).

### Step 2: Synthesis of tert-butyl (2-((6-chloro-5-cyclopropylpyridazin-3-yl)amino)-2-oxoethyl)(methyl)carbamate

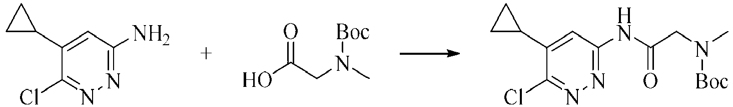

N-(tert-butoxycarbonyl)-N-methylglycine (825.5 mg, 4.36 mmol, 2.0 eq.) and triethylamine (662.0 mg, 6.54 mmol, 3.0 eq.) were added to dichloromethane (15.0 mL). In an ice-water bath, isopropyl chloroformate (668.2 mg, 5.45 mmol, 2.5 eq.) was added dropwise. In an ice-water bath, the resultant was stirred for 1 hour, a solution of **6-chloro-5-cyclopropylpyridazin-3-amine** (370.0 mg, 2.18 mmol, 1.0 eq.) in dichloromethane (5.0 mL) was then added thereto, and the resultant was gradually warmed to room temperature and reacted for 12 hours. The completion of the reaction was monitored by TLC. Saturated aqueous ammonium chloride solution (50.0 mL) was added, and the resultant was extracted with dichloromethane (100.0 mL). The organic phase was dried over anhydrous sodium sulfate and filtered. The filtrate was concentrated under reduced pressure. The crude product was purified by silica gel column chromatography (dichloromethane:methanol=100:1-80:1), so as to afford the product (660.0 mg, yield:88.8%).

### Step 3: Synthesis of tert-butyl (2-((5-cyclopropyl-6-(2-(ethoxymethoxy)-4-formylphenyl)pyridazin-3-yl)amino)-2-oxoethyl)( methyl)carbamate

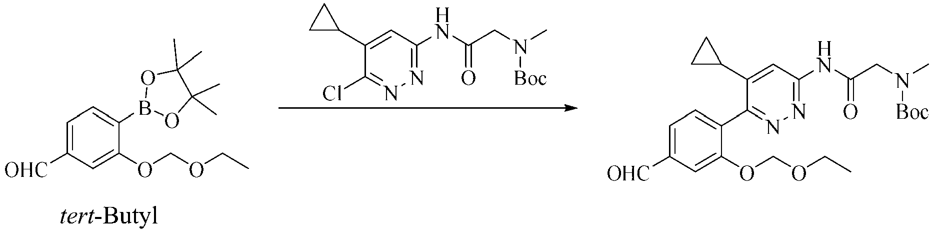

(2-((6-chloro-5-cyclopropylpyridazin-3-yl)amino)-2-oxoethyl)(methyl)carbamate (625.0 mg, 1.83 mmol, 1.0 eq.), 3-(ethoxymethoxy)-4-(4,4,5,5-tetramethyl-1,3,2-dioxaborolan-2-yl)benzaldehyde (673.8 mg, 2.20 mmol, 1.2 eq.), potassium carbonate (506.9 mg, 3.66 mmol, 2.0 eq.) and Pd(PPh₃)₄ (211.9 mg, 0.18 mmol, 0.1 eq.) were added to a mixed solution of 1,4-dioxane (10.0 mL) and water (5.0 mL). Under nitrogen protection, the resultant was reacted at 100°C for 4 hours. The completion of the reaction was monitored by TLC. Water (100.0 mL) was added, and the resultant was extracted with ethyl acetate (100.0 mL). The organic phase was dried over anhydrous sodium sulfate and filtered. The filtrate was concentrated under reduced pressure. The crude product was purified by silica gel column chromatography (dichloromethane:methanol=100:1-80:1), so as to afford the product (504.0 mg, yield: 56.7%).

### Step 4: Synthesis of tert-butyl (2-((5-cyclopropyl-6-(2-(ethoxymethoxy)-4-ethynylphenyl)pyridazin-3-yl)amino)-2-oxoethyl)( methyl)carbamate

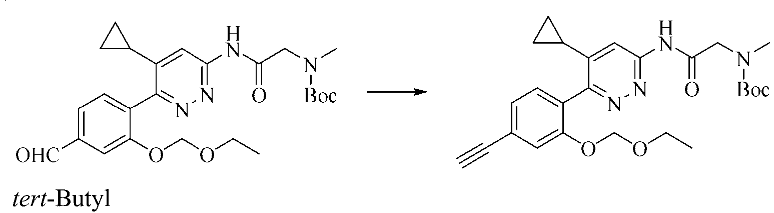

(2-((5-cyclopropyl-6-(2-(ethoxymethoxy)-4-formylphenyl)pyridazin-3-yl)amino)-2-oxoethyl)( methyl)carbamate (504.0 mg, 1.04 mmol, 1.0 eq.), dimethyl (1-diazo-2-oxopropyl)phosphonate (299.6 mg, 1.5 mmol, 1.5 eq.) and anhydrous potassium carbonate (287.4 mg, 2.08 mmol, 2.0 eq.) were added to methanol (10.0 mL), and the resultant was reacted at room temperature for 4 hours. The completion of the reaction was monitored by TLC. The system was concentrated under reduced pressure. The crude product was purified by silica gel column chromatography (dichloromethane:methanol=100:1-80:1), so as to afford the product (272.0 mg, yield: 56.0%).

### Step 5: Synthesis of N-(5-cyclopropyl-6-(4-ethynyl-2-hydroxyphenyl)pyridazin-3-yl)-2-(methylamino)acetamide

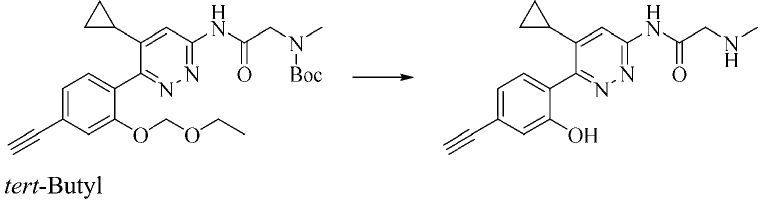

(2-((5-cyclopropyl-6-(2-(ethoxymethoxy)-4-ethynylphenyl)pyridazin-3-yl)amino)-2-oxoethyl)( methyl)carbamate (272.0 mg, 0.56 mmol, 1.0 eq.) was added to dichloromethane (10.0 mL), trifluoroacetic acid (3.0 mL) was added dropwise, and the resultant was reacted at room temperature for 1 hour. The completion of the reaction was monitored by TLC. The system was adjusted to have a pH value of 8~9 with saturated aqueous sodium carbonate solution, and was extracted with dichloromethane (100.0 mL). The organic phase was dried over anhydrous sodium sulfate and filtered. The filtrate was concentrated under reduced pressure. The crude product was purified by silica gel column chromatography (dichloromethane:methanol=40:1-10:1), so as to afford the product (90.0 mg, yield:49.4%).

¹HNMR (400MHz, DMSO-*d₆*) δ(ppm): 7.82 (s, 1H), 7.27-7.25 (d, J=8 Hz, 1H), 7.06-7.04 (m, 2H), 4.21 (s, 1H), 3.34 (s, 3H), 2.33 (s, 3H), 1.69-1.65 (m, 1H), 1.02-0.99 (m, 2H), 0.72-0.70 (m, 2H).

Molecular formula: C₁₈H₁₈N₄O₂ Precise molecular weight: 322.14 LC-MS (m/z)=323.19[M+H]⁺.

### Example 2: Synthesis of N-(5-cyclopropyl-6-(4-ethynyl-2-hydroxyphenyl)pyridazin-3-yl)-2-(dimethylamino)aceta mide (Compound 80)

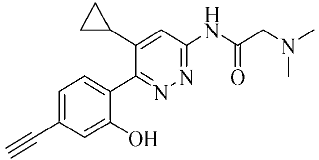

### Step 1 : Synthesis of N-(6-chloro-5-cyclopropylpyridazin-3-yl)-2-(dimethylamino)acetamide

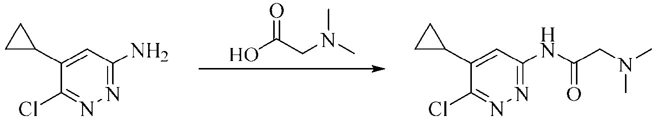

N,N-Dimethylglycine (486.3 mg, 4.72 mmol, 2.0 eq.) and triethylamine (954.4 mg, 9.43 mmol, 4.0 eq.) were added to dichloromethane (15.0 mL). In an ice-water bath, isopropyl chloroformate (866.9 mg, 7.07 mmol, 3.0 eq.) was added dropwise. In an ice-water bath, the resultant was stirred for 1 hour, a solution of 6-chloro-5-cyclopropylpyridazin-3-amine (400.0 mg, 2.36 mmol, 1.0 eq.) in dichloromethane (5.0 mL) was then added, and the resultant was gradually warmed to room temperature and reacted for 14 hours. TLC monitoring showed the completion of the reaction. The reaction solution was washed with saturated aqueous ammonium chloride solution (50.0 mL), and the aqueous phase was extracted with dichloromethane (100.0 mL). The organic phases were combined, dried over anhydrous sodium sulfate, and filtered. The filtrate was concentrated under reduced pressure. The crude product was purified by silica gel column chromatography (dichloromethane:methanol=100:1-80:1), so as to afford the product (468.2 mg, yield: 78.0%).

### Step 2: Synthesis of N-(5-cyclopropyl-6-(2-(ethoxymethoxy)-4-formylphenyl)pyridazin-3-yl)-2-(dimethylamino)ac etamide

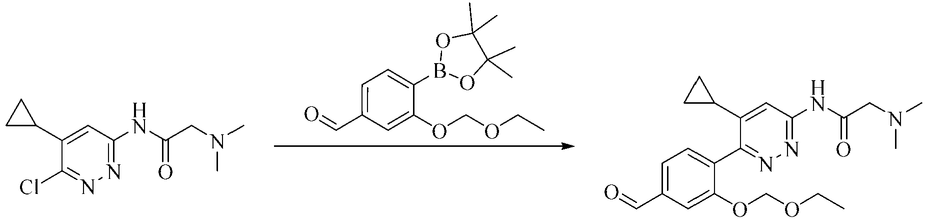

*N*-(6-Chloro-5-cyclopropylpyridazin-3-yl)-2-(dimethylamino)acetamide (468.2 mg, 1.84 mmol, 1.0 eq.), 3-(ethoxymethoxy)-4-(4,4,5,5-tetramethyl-1,3,2-dioxaborolan-2-yl)benzaldehyde (731.6 mg, 2.39 mmol, 1.3 eq.), potassium carbonate (508.0 mg, 3.68 mmol, 2.0 eq.) and Pd(PPh₃)₄ (212.4 mg, 0.18 mmol, 0.1 eq.) were added to a mixed solution of 1,4-dioxane (10.0 mL) and water (5.0 mL). Under nitrogen protection, the resultant was reacted at 100°C for 6 hours. TLC monitoring showed the completion of the reaction. Water (100.0 mL) was added, and the resultant was extracted with ethyl acetate (100.0 mL). The organic phase was dried over anhydrous sodium sulfate and filtered. The filtrate was concentrated under reduced pressure. The crude product was purified by silica gel column chromatography (dichloromethane:methanol=100:1-50:1), so as to afford the product (450.0 mg, yield: 61.4%).

### Step 3: Synthesis of N-(5-cyclopropyl-6-(2-(ethoxymethoxy)-4-ethynylphenyl)pyridazin-3-yl)-2-(dimethylamino)ac etamide

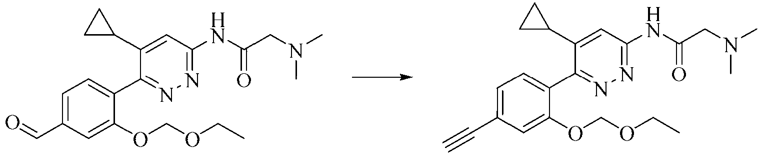

*N*-(5-cyclopropyl-6-(2-(ethoxymethoxy)-4-formylphenyl)pyridazin-3-yl)-2-(dimethylami no)acetamide (450.0 mg, 1.13 mmol, 1.0 eq.), dimethyl (1-diazo-2-oxopropyl)phosphonate (325.3 mg, 1.69 mmol, 1.5 eq.) and anhydrous potassium carbonate (312.0 mg, 2.26 mmol, 2.0 eq.) were added to methanol (10.0 mL), and the resultant was reacted at room temperature for 4 hours. TLC monitoring showed the completion of the reaction. The resultant was concentrated under reduced pressure. The crude product was purified by silica gel column chromatography (dichloromethane:methanol=100:1-50:1), so as to afford the product (388.0 mg, yield: 87.2%).

### Step 4: Synthesis of N-(5-cyclopropyl-6-(4-ethynyl-2-hydroxyphenyl)pyridazin-3-yl)-2-(dimethylamino)acetamide

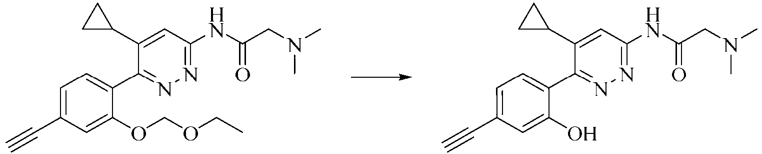

*N*-(5-cyclopropyl-6-(2-(ethoxymethoxy)-4-ethynylphenyl)pyridazin-3-yl)-2-(dimethylam ino)acetamide (380.0 mg, 0.96 mmol, 1.0 eq.) was added to dichloromethane (10.0 mL), trifluoroacetic acid (4.0 mL) was added dropwise, and the resultant was reacted at room temperature for 1 hour. TLC monitoring showed the completion of the reaction. The resultant was adjusted to have a pH value of 8~9 with saturated aqueous sodium carbonate solution, and was extracted with dichloromethane (100.0 mL). The organic phase was dried over anhydrous sodium sulfate and filtered. The filtrate was concentrated under reduced pressure. The crude product was purified by silica gel column chromatography (dichloromethane:methanol=100:1-80:1), so as to afford the product (160.0 mg, yield: 49.3%)
¹HNMR (400 MHz, DMSO-*d₆*) δ(ppm): 10.46 (s, 1H), 10.04 (s, 1H), 7.79 (s, 1H), 7.27-7.25 (d, *J*=8 Hz, 1H), 7.06-7.04 (m, 2H), 4.21 (s, 1H), 3.19 (s, 2H), 2.32 (s, 6H), 1.69-1.65 (m, 1H), 1.03-0.98 (m, 2H), 0.74-0.70 (m, 2H).

Molecular formula: C₁₉H₂₀N₄O₂ Precise molecular weight: 336.16 LC-MS (m/z)=337.16[M+H]⁺.

### Example 3: Synthesis of N-(5-cyclopropyl-6-(4-ethynyl-2-hydroxyphenyl)pyridazin-3-yl)-2-(methylamino)propana mide (Compound 27)

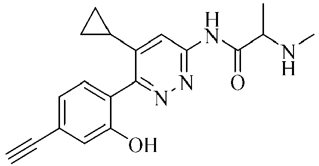

### Step 1: Synthesis of tert-butyl (1-((6-chloro-5-cyclopropylpyridazin-3-yl)amino)-1-oxopropan-2-yl)(methyl)carbamate

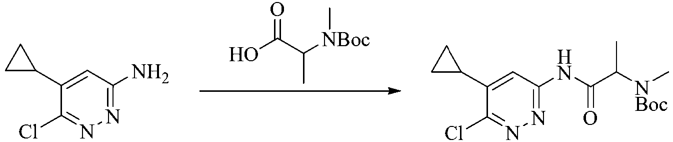

N-(tert-butoxycarbonyl)-N-methylalanine (838.5 mg, 4.13 mmol, 2.0 eq.) and triethylamine (626.3 mg, 6.19 mmol, 3.0 eq.) were added to dichloromethane (15.0 mL). In an ice-water bath, isopropyl chloroformate (505.6 mg, 4.13 mmol, 2.0 eq.) was added dropwise, the resultant was stirred for 1 hour, 6-chloro-5-cyclopropylpyridazin-3-amine (350.0 mg, 2.06 mmol, 1.0 eq.) was then added, and the resultant was gradually warmed to room temperature and reacted for 12 hours. TLC monitoring showed the completion of the reaction. The reaction solution was washed with saturated aqueous ammonium chloride solution (50.0 mL), and the aqueous phase was extracted with dichloromethane (50.0 mL). The organic phases were combined, dried over anhydrous sodium sulfate, and filtered. The filtrate was concentrated under reduced pressure. The crude product was purified by silica gel column chromatography (petroleum ether:ethyl acetate=10:1-5:1), so as to afford the product (354.0 mg, yield: 48.3%).

### Step 2: Synthesis of tert-butyl (1-((5-cyclopropyl-6-(2-(ethoxymethoxy)-4-formylphenyl)pyridazin-3-yl)amino)-1-oxopropan-2-yl)(methyl)carbamate

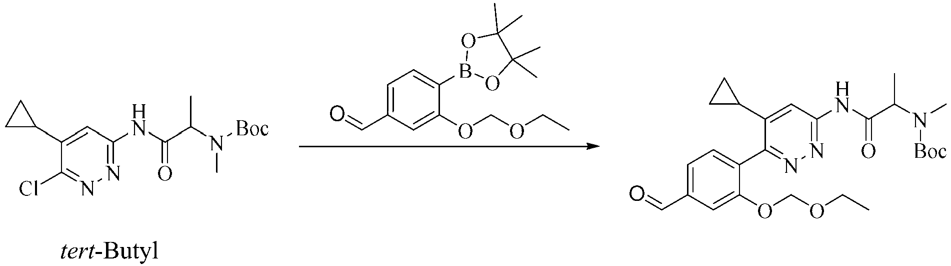

(1-((6-chloro-5-cyclopropylpyridazin-3-yl)amino)-1-oxopropan-2-yl)(methyl)carbamate (354.0 mg, 0.99 mmol, 1.0 eq.), 3-(ethoxymethoxy)-4-(4,4,5,5-tetramethyl-1,3,2-dioxaborolan-2-yl)benzaldehyde (396.8 mg, 1.29 mmol, 1.3 eq.), potassium carbonate (206.6 mg, 1.49 mmol, 1.5 eq.) and Pd(PPh₃)₄ (115.2 mg, 0.09 mmol, 0.1 eq.) were added to a mixed solution of 1,4-dioxane (10.0 mL) and water (5.0 mL). Under nitrogen protection, the resultant was reacted at 100°C for 4 hours. TLC monitoring showed the completion of the reaction. Water (50.0 mL) was added, and the resultant was extracted with ethyl acetate (50.0 mL). The organic phase was dried over anhydrous sodium sulfate and filtered. The filtrate was concentrated under reduced pressure. The crude product was purified by silica gel column chromatography (petroleum ether:ethyl acetate=10:1-1:1), so as to afford the product (270.0 mg, yield: 54.3%).

### Step 3: Synthesis of tert-butyl (1-((5-cyclopropyl-6-(2-(ethoxymethoxy)-4-ethynylphenyl)pyridazin-3-yl)amino)-1-oxopropan -2-yl)(methyl)carbamate

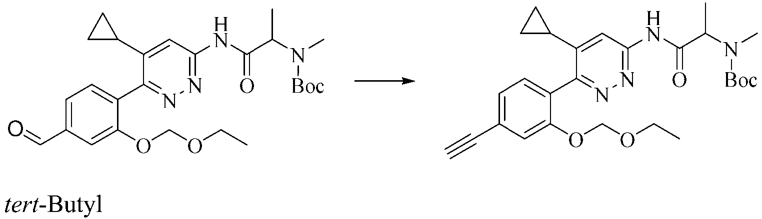

(1-((5-cyclopropyl-6-(2-(ethoxymethoxy)-4-formylphenyl)pyridazin-3-yl)amino)-1-oxopropan-2-yl)(methyl)carbamate (260.0 mg, 0.52 mmol, 1.0 eq.), dimethyl (1-diazo-2-oxopropyl)phosphonate (150.0 mg, 0.78 mmol, 1.5 eq.) and anhydrous potassium carbonate (144.0 mg, 1.04 mmol, 2.0 eq.) were added to methanol (15.0 mL), and the resultant was reacted at room temperature for 3 hours. TLC monitoring showed the completion of the reaction. The resultant was concentrated under reduced pressure. The crude product was purified by silica gel column chromatography (petroleum ether:ethyl acetate=5:1-1:1), so as to afford the product (179.0 mg, yield: 69.4%).

### Step 4: Synthesis of N-(5-cyclopropyl-6-(4-ethynyl-2-hydroxyphenyl)pyridazin-3-yl)-2-(methylamino)propanamide

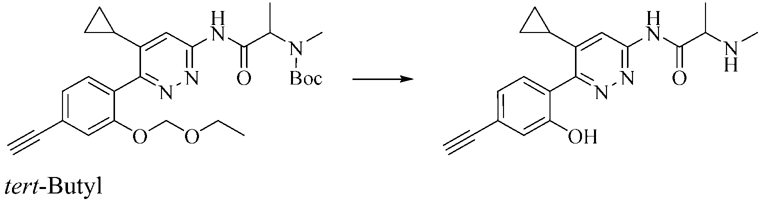

(1-((5-cyclopropyl-6-(2-(ethoxymethoxy)-4-ethynylphenyl)pyridazin-3-yl)amino)-1-oxopropan -2-yl)(methyl)carbamate (179.0 mg, 0.36 mmol, 1.0 eq.) was added to dichloromethane (5.0 mL), a solution of hydrogen chloride in 1,4-dioxane (4.0 mol/L, 3.0 mL) was added dropwise, and the resultant was reacted at room temperature for 3 hours. TLC monitoring showed the completion of the reaction. The resultant was adjusted to have a pH value of 8~9 with saturated aqueous sodium carbonate solution, and was extracted with dichloromethane (50.0 mL). The organic phase was dried over anhydrous sodium sulfate and filtered. The filtrate was concentrated under reduced pressure. The crude product was purified by preparative thin-layer chromatography, so as to afford the product (20.0 mg, yield: 16.4%).

¹HNMR (400 MHz, DMSO-*d₆*) δ(ppm): 10.04 (s, 1H), 7.84 (s, 1H), 7.27-7.25 (d, J=8 Hz, 1H), 7.07-7.04 (m, 2H), 4.22 (s, 1H), 3.30 (s, 1H), 2.29 (s, 3H), 1.69-1.65 (m, 1H), 1.30 (s, 1H), 1.26 (s, 1H), 1.24 (s, 3H), 1.03-0.98 (m, 2H), 0.74-0.70 (m, 2H).

Molecular formula: C₁₉H₂₀N₄O₂ Precise molecular weight: 336.16 LC-MS (m/z)=337.20 [M+H]⁺.

### Example 4: Synthesis of N-(5-cyclopropyl-6-(4-ethynyl-2-hydroxyphenyl)pyridazin-3-yl)pyrrolidine-2-carboxamid e (Compound 28)

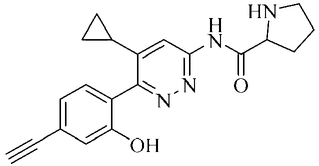

The compound was prepared in accordance with the synthetic method of Example 3.

¹HNMR (400 MHz, DMSO-*d₆*) δ(ppm): 10.73 (s, 1H), 10.10 (s, 1H), 7.83 (s, 1H), 7.27-7.25 (d, *J*=8 Hz, 1H), 7.06-7.04 (m, 2H), 4.22 (s, 1H), 3.83-3.30 (m, 1H), 2.98-2.85 (m, 2H), 2.12-2.03 (m, 1H), 1.87-1.79 (m, 1H), 1.70-1.63 (m, 3H), 1.03-0.98 (m, 2H), 0.73-0.70 (m, 2H).

**Molecular formula: C₂₀H₂₀N₄O₂** Precise molecular weight: 348.16 LC-MS **(m/z)=349.20 [M+H]⁺.**

### Example 5: Synthesis of compound 3-(6-(4-ethynyl-2-hydroxyphenyl)-5-methylpyridazin-3-yl)-1-methylimidazolidin-4-one (Compound 29)

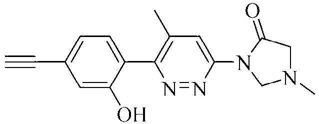

### Step 1 : Synthesis of intermediate 4-(6-amino-4-methylpyridazin-3-yl)-3-(ethoxymethoxy)benzaldehyde

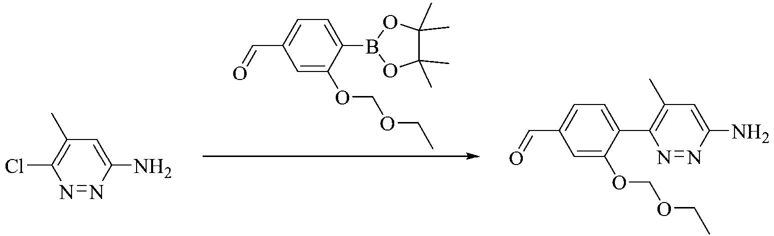

3-Amino-5-methyl-6-chloropyridazine (5.0 g, 34.83 mmol, 1.0 eq), 3-(ethoxymethoxy)-4-(4,4,5,5-tetramethyl-1,3,2-dioxaborolan-2-yl)benzaldehyde (13.86 g, 45.28 mmol, 1.3 eq), an aqueous solution (15 mL) of potassium carbonate (9.63 g, 69.66 mmol, 2.0 eq), and tetrakis(triphenylphosphine)palladium (4.03 g, 3.483 mmol, 0.1 eq) were added to 1,4-dioxane (50 mL). Under nitrogen protection, the resultant was reacted at 90°C for 17h. TLC monitoring showed the completion of the reaction. The reaction solution was filtered through celite, and rinsed with dichloromethane (100 mL). The filtrate was dried over anhydrous magnesium sulfate and filtered. The filtrate was concentrated to give a crude product, which was purified by silica gel column chromatography (dichloromethane:methanol=50:1~20:1), so as to afford the product (8.0 g, yield: 80%).

### Step 2: Synthesis of intermediate 6-(2-(ethoxymethoxy)-4-ethynyl)-5-methylpyridazin-3-amine

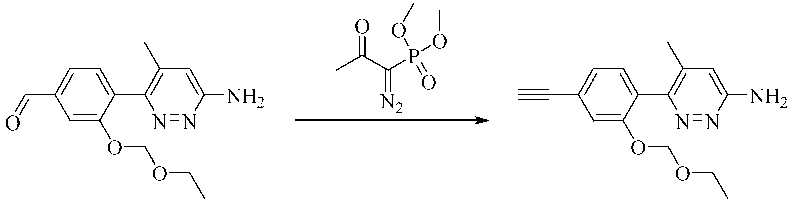

4-(6-Amino-4-methylpyridazin-3-yl)-3-(ethoxymethoxy)benzaldehyde (8.0 g, 27.84 mmol, 1.0 eq) was dissolved in methanol (80 mL), potassium carbonate (7.7 g, 55.68 mmol, 2.0 eq) was added thereto, and the resultant was reacted at 25°C for 2 hours. Dimethyl (1-diazo-2-oxopropyl)phosphonate (8.02 g, 41.76 mmol, 1.5 eq) was added, and the resultant was reacted at 25°C for 1.5 hours, TLC monitoring showed the completion of the reaction. The reaction solution was filtered through celite. The filtrate was concentrated to give a crude product, which was purified by silica gel column chromatography (dichloromethane:methanol=50:1~30:1), so as to afford the product (4.0 g, yield: 50.7%).

### Step 3: Synthesis of intermediate tert-butyl (2-((6-(2-(ethoxymethoxy)-4-ethynylphenyl)-5-methylpyridazin-3-yl)amino)-2-oxoethyl)(meth yl)carbamate

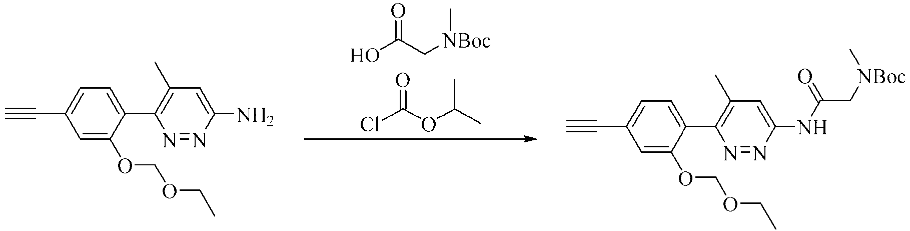

N-(tert-butoxycarbonyl)sarcosine (200 mg, 1.058 mmol, 2.0 eq) was dissolved in dichloromethane (2 mL), and triethylamine (161 mg, 1.587 mmol, 3.0 eq) was added thereto. At 0°C, isopropyl chloroformate (162 mg, 1.323 mmol, 2.5 eq) was added dropwise thereto, and the resultant was reacted at 0°C for 1 hour. A solution of 6-(2-(ethoxymethoxy)-4-ethynyl)-5-methylpyridazin-3-amine (150 mg, 0.529 mmol, 1.0 eq) in dichloromethane (2 mL) was added, and the resultant was heated to 25°C and reacted for 1 hour. TLC monitoring showed the completion of the reaction. The reaction solution was poured into water (2 mL), and the resultant was extracted with dichloromethane (5 mL×3). The organic phases were combined, dried over anhydrous magnesium sulfate, and filtered. The filtrate was concentrated to give a crude product, which was purified by preparative thin-layer chromatography (ethyl acetate), so as to afford the product (110 mg, yield: 45.8%).

### Step 4: Synthesis of compound 3-(6-(4-ethynyl-2-hydroxyphenyl)-5-methylpyridazin-3-yl)-1-methylimidazolidin-4-one

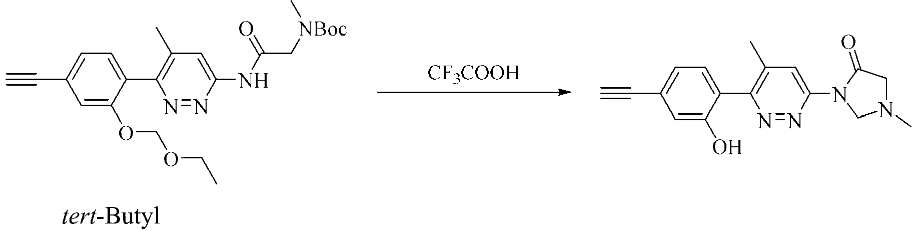

(2-((6-(2-(ethoxymethoxy)-4-ethynylphenyl)-5-methylpyridazin-3-yl)amino)-2-oxoethyl)(meth yl)carbamate (110 mg, 0.242 mmol, 1.0 eq) was dissolved in dichloromethane (1 mL), the resulting mixture was added dropwise to trifluoroacetic acid (1 mL), and the resultant was reacted at 25°C for 0.5 hours. TLC monitoring showed the completion of the reaction. The reaction solution was concentrated and poured into saturated aqueous sodium bicarbonate solution (10 mL), and the resultant was extracted with a mixed solvent of dichloromethane and methanol (10:1) (10 mL×6). The organic phases were combined, dried over anhydrous magnesium sulfate, and filtered. The filtrate was concentrated to give a crude product, which was purified by preparative thin-layer chromatography (dichloromethane:methanol=10:1), so as to afford the product (11 mg, yield: 14.7%).

¹H-NMR (400 MHz, DMSO-*d₆*) δ(ppm): 10.13 (s, 1H), 8.36 (d, 1H), 7.24-7.22 (s, 1H), 7.07-7.05 (m, 2H), 4.84 (s, 2H), 4.23 (s, 1H), 3.49 (s, 2H), 2.48 (s, 3H), 2.21-2.20 (d, 3H).

### Molecular formula: C₁₇H₁₆N₄O₂ Precise molecular weight: 308.13 LC-MS (m/z): 309.13 [M+H]⁺

### Example 6: Synthesis of N-(5-cyclopropyl-6-(4-ethynyl-2-hydroxyphenyl)pyridazin-3-yl)-2-methoxyacetamide (Compound 30)

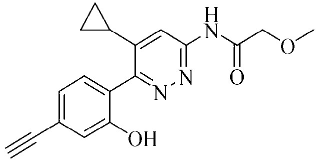

### Step 1 : Synthesis of 4-(6-amino-4-cyclopropylpyridazin-3-yl)-3-(ethoxymethoxy)benzaldehyde

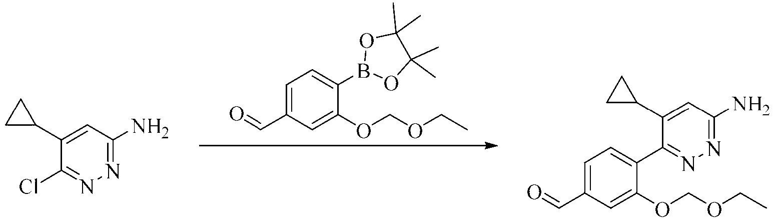

6-Chloro-5-cyclopropylpyridazin-3-amine (3.0 g, 17.68 mmol, 1.0 eq.), 3-(ethoxymethoxy)-4-(4,4,5,5-tetramethyl-1,3,2-dioxaborolan-2-yl)benzaldehyde (6.5 g, 21.22 mmol, 1.2 eq.), potassium carbonate (3.6 g, 26.53 mmol, 1.5 eq.) and Pd(PPh₃)₄ (1.0 g, 0.88 mmol, 0.05 eq.) were added to a mixed solution of 1,4-dioxane (30.0 mL) and water (15.0 mL). Under nitrogen protection, the resultant was reacted at 90°C for 12 hours. TLC monitoring showed the completion of the reaction. Ethyl acetate (100.0 mL) was added, and the resultant was washed with water (100.0 mL). The organic phase was dried over anhydrous sodium sulfate and filtered. The filtrate was concentrated under reduced pressure. The crude product was purified by silica gel column chromatography (petroleum ether:ethyl acetate=10:1-2:1), so as to afford the product (4.0 g, yield: 72.2%).

### Step 2: Synthesis of 5-cyclopropyl-6-(2-(ethoxymethoxy)-4-ethynylphenyl)pyridazin-3-amine

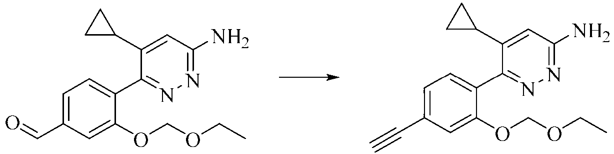

4-(6-Amino-4-cyclopropylpyridazin-3-yl)-3-(ethoxymethoxy)benzaldehyde (3.95 g, 12.61 mmol, 1.0 eq.), dimethyl (1-diazo-2-oxopropyl)phosphonate (3.6 g, 18.91 mmol, 1.5 eq.) and anhydrous potassium carbonate (3.48 g, 25.21 mmol, 2.0 eq.) were added to methanol (40.0 mL), and the resultant was reacted at room temperature for 4 hours. TLC monitoring showed the completion of the reaction. The resultant was concentrated under reduced pressure. The crude product was purified by silica gel column chromatography (dichloromethane:methanol=80:1-40:1), so as to afford the product (3.2 g, yield: 82.0%).

### Step 3: Synthesis of N-(5-cyclopropyl-6-(2-ethoxymethoxy)-4-ethynylphenyl)pyridazin-3-yl)-2-methoxyacetamide

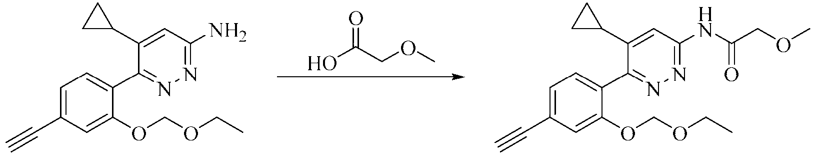

2-Methoxyacetic acid (203.7 mg, 2.26 mmol, 2.0 eq.), *N,N*-diisopropylethylamine (438.5 mg, 3.39 mmol, 3.0 eq.) and HATU (634.6 mg, 2.26 mmol, 2.0 eq.) were added to DMF (10.0 mL), the resultant was stirred for 1 hour, 5-cyclopropyl-6-(2-(ethoxymethoxy)-4-ethynylphenyl)pyridazin-3-amine (350.0 mg, 1.13 mmol, 1.0 eq.) was then added, and the resultant was reacted at room temperature for 12 hours. TLC monitoring showed the completion of the reaction. Ethyl acetate (100.0 mL) was added, and the resultant was washed with water (100.0 mL). The organic phase was dried over anhydrous sodium sulfate and filtered. The filtrate was concentrated under reduced pressure. The crude product was purified by silica gel column chromatography (petroleum ether:ethyl acetate=10:1-5:1), so as to afford the product (174.0 mg, yield: 40.3%).

### Step 4: Synthesis of N-(5-cyclopropyl-6-(4-ethynyl-2-hydroxyphenyl)pyridazin-3-yl)-2-methoxyacetamide

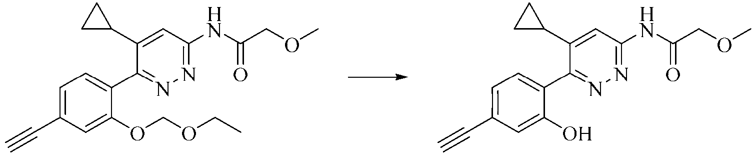

*N*-(5-cyclopropyl-6-(2-ethoxymethoxy)-4-ethynylphenyl)pyridazin-3-yl)-2-methoxyaceta mide (174.0 mg, 0.45 mmol, 1.0 eq.) was added to dichloromethane (10.0 mL), trifluoroacetic acid (2.0 mL) was added dropwise, and the resultant was reacted at room temperature for 1 hour. TLC monitoring showed the completion of the reaction. The resultant was adjusted to have a pH value of 8~9 with saturated aqueous sodium carbonate solution, and the resultant was extracted with dichloromethane (50.0 mL). The organic phase was dried over anhydrous sodium sulfate and filtered. The filtrate was concentrated under reduced pressure. The crude product was purified by preparative thin-layer chromatography, so as to afford the product (85.0 mg, yield: 57.8%).

¹HNMR (400 MHz, DMSO-*d₆*) δ(ppm): 10.67 (s, 1H), 10.06 (s, 1H), 7.76 (s, 1H), 7.28-7.26 (d, *J*=8 Hz, 1H), 7.07-7.04 (m, 2H), 4.21 (s, 1H), 4.12 (s, 2H), 3.38 (s, 3H), 1.70-1.64 (m, 1H), 1.03-0.98 (m, 2H), 0.74-0.70 (m, 2H).

Molecular formula: C₁₈H₁₇N₃O₃ Precise molecular weight: 323.13 LC-MS (m/z)=324.14 [M+H]⁺.

### Example 7: Synthesis of N-(5-cyclopropyl-6-(4-ethynyl-2-hydroxyphenyl)pyridazin-3-yl)-2-(cyclopropylamino)acet amide (Compound 10)

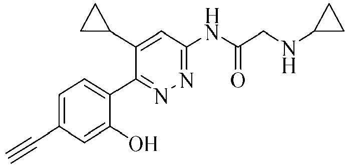

### Step 1 : Synthesis of 2-bromo-N-(5-cyclopropyl-6-(2-(ethoxymethoxy)-4-ethynylphenyl)pyridazin-3-yl)acetamide

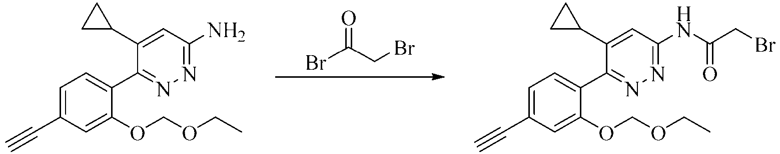

5-Cyclopropyl-6-(2-(ethoxymethoxy)-4-ethynylphenyl)pyridazin-3-amine (1.0 g, 3.23 mmol, 1.0 eq.) and *N,N*-diisopropylethylamine (1.25 g, 9.69 mmol, 3.0 eq.) were added to dichloromethane (20.0 mL). In an ice-water bath, bromoacetyl bromide (1.3 g, 6.46 mmol, 2.0 eq.) was added dropwise, and the resultant was reacted for 0.5 hours. TLC monitoring showed the completion of the reaction. Dichloromethane (50.0 mL) was added, and the resultant was washed with saturated aqueous ammonium chloride solution (50.0 mL). The organic phase was dried over anhydrous sodium sulfate and filtered. The filtrate was concentrated under reduced pressure. The crude product was purified by silica gel column chromatography (petroleum ether:ethyl acetate=6:1-3:1), so as to afford the product (1.0 g, yield: 71.9%).

### Step 2: Synthesis of 2-bromo-N-(5-cyclopropyl-6-(4-ethynyl-2-hydroxyphenyl)pyridazin-3-yl)acetamide

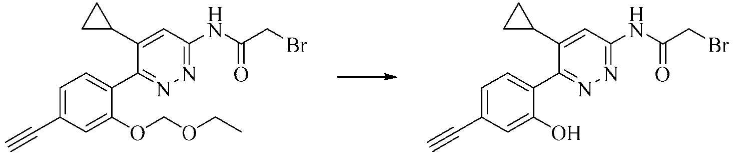

2-Bromo-*N*-(5-cyclopropyl-6-(2-(ethoxymethoxy)-4-ethynylphenyl)pyridazin-3-yl)aceta mide (1.0 g, 2.32 mmol, 1.0 eq.) was added to dichloromethane (10.0 mL), trifluoroacetic acid (2.0 mL) was added dropwise, and the resultant was reacted at room temperature for 1 hour. TLC monitoring showed the completion of the reaction. The resultant was adjusted to have a pH value of 7~8 with saturated aqueous sodium bicarbonate solution, and was extracted with dichloromethane (50.0 mL). The organic phase was dried over anhydrous sodium sulfate and filtered. The filtrate was concentrated under reduced pressure. The crude product was purified by silica gel column chromatography (dichloromethane:methanol=40:1-10:1), so as to afford the product (575.0 mg, yield: 66.4%).

### Step 3: Synthesis of N-(5-cyclopropyl-6-(4-ethynyl-2-hydroxyphenyl)pyridazin-3-yl)-2-(cyclopropylamino)acetami de

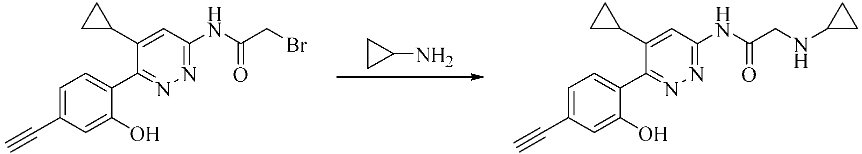

2-Bromo-*N*-(5-cyclopropyl-6-(4-ethynyl-2-hydroxyphenyl)pyridazin-3-yl)acetamide (130.0 mg, 0.35 mmol, 1.0 eq.), *N,N*-diisopropylethylamine (135.4 mg, 1.05 mmol, 3.0 eq.) and cyclopropylamine (39.8 mg, 0.70 mmol, 2.0 eq.) were added to DMF (3.0 mL), and the resultant was reacted for 1 hour. TLC monitoring showed the completion of the reaction. Ethyl acetate (50.0 mL) was added, and the resultant was washed with saturated aqueous ammonium chloride solution (50.0 mL). The organic phase was dried over anhydrous sodium sulfate and filtered. The filtrate was concentrated under reduced pressure. The crude product was purified by preparative thin-layer chromatography, so as to afford the product (80.0 mg, yield: 65.7%).

¹HNMR (400 MHz, DMSO-*d₆*) δ(ppm): 10.70 (s, 1H), 10.03 (s, 1H), 7.81 (s, 1H), 7.28-7.26 (d, *J*=8 Hz, 1H), 7.06-7.04 (m, 2H), 4.21 (s, 1H), 3.44 (s, 2H), 3.18-3.16 (d, *J*=8 Hz, 1H), 2.22-2.17 (m, 1H), 1.70-1.64 (m, 1H), 1.03-0.98 (m, 2H), 0.73-0.69 (m, 2H), 0.41-0.37 (m, 2H), 0.31-0.28 (m, 2H).

Molecular formula: C₂₀H₂₀N₄O₂ Precise molecular weight: 348.16 LC-MS (m/z)=349.20 [M+H]⁺.

### Example 8: Synthesis of N-(5-cyclopropyl-6-(4-ethynyl-2-hydroxyphenyl)pyridazin-3-yl)-2-(ethylamino)acetamide (Compound 8)

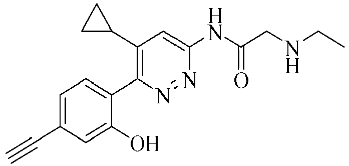

The product (yield: 55.0%) was prepared in accordance with the synthetic method in Step 3 of **Example 7.**

¹HNMR (400 MHz, DMSO-*d₆*) δ(ppm): 10.03 (s, 1H), 7.82 (s, 1H), 7.27-7.25 (d, *J*=8 Hz, 1H), 7.06-7.04 (m, 2H), 4.21 (s, 1H), 3.38 (s, 3H), 2.62-2.57 (m, 2H), 1.69-1.64 (m, 1H), 1.07-1.03 (m, 3H), 1.02-0.98 (m, 2H), 0.73-0.69 (m, 2H).

Molecular formula: C₁₉H₂₀N₄O₂ Precise molecular weight: 336.16 LC-MS (m/z)=337.19 [M+H]⁺.

### Example 9: Synthesis of N-(5-cyclopropyl-6-(4-ethynyl-2-hydroxyphenyl)pyridazin-3-yl)-2-((2,2,2-trifluoroethyl)a mino)acetamide (Compound 12)

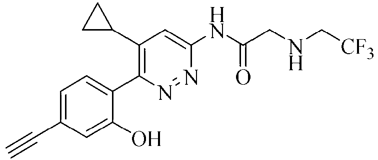

The compound was prepared in accordance with the synthetic method in Step 3 of **Example 7.**

¹HNMR (400 MHz, DMSO-*d₆*) δ(ppm): 10.79 (s, 1H), 10.02 (s, 1H), 7.80 (s, 1H), 7.28-7.26 (d, *J*=8 Hz, 1H), 7.07-7.06 (m, 2H), 4.22 (s, 1H), 3.55-3.53 (d, *J*=8 Hz, 2H), 3.42-3.38 (m, 2H), 3.07-3.01 (m, 1H), 1.71-1.64 (m, 1H), 1.03-0.99 (m, 2H), 0.73-0.69 (m, 2H).

Molecular formula: C₁₉H₁₇F₃N₄O₂ Precise molecular weight: 390.13 LC-MS (m/z)=391.15 [M+H]⁺.

### Example 10: Synthesis of N-(5-cyclopropyl-6-(4-ethynyl-2-hydroxyphenyl)pyridazin-3-yl)-2-(isopropylamino)aceta mide (Compound 9)

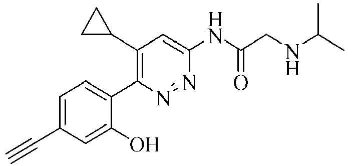

The compound was prepared in accordance with the synthetic method in Step 3 of **Example 7.**

¹HNMR (400 MHz, DMSO-*d₆*) δ(ppm): 10.03 (s, 1H), 7.82 (s, 1H), 7.27-7.25 (d, *J*=8 Hz, 1H), 7.06-7.04 (m, 2H), 4.21 (s, 1H), 3.38 (s, 4 H), 2.80-2.74 (m, 1H), 1.71-1.64 (m, 1H), 1.03-0.99 (m, 8H), 0.73-0.69 (m, 2H).

Molecular formula: C₂₀H₂₂N₄O₂ Precise molecular weight: 350.17 LC-MS (m/z)=351.20 [M+H]⁺.

### Example 11: Synthesis of N-(5-cyclopropyl-6-(4-ethynyl-2-hydroxyphenyl)pyridazin-3-yl)-2-((3-hydroxycyclobutyl) amino)acetamide (Compound 33)

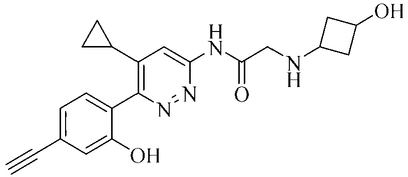

The compound was prepared in accordance with the synthetic method in Step 3 of **Example 7.**

¹HNMR (400 MHz, DMSO-*d₆*) δ(ppm): 10.04 (s, 1H), 7.79 (s, 1H), 7.28-7.26 (d, *J*=8 Hz, 1H), 7.06-7.04 (m, 2H), 4.99-4.94 (m, 1H), 4.21 (s, 1H), 3.77-3.72 (m, 1H), 3.37 (s, 4 H), 2.82-2.74 (m, 1H), 2.47-2.41 (m, 2H), 2.07-1.91 (m, 1H), 1.71-1.59 (m, 2H), 1.04-0.99 (m, 2H), 0.72-0.68 (m, 2H)

Molecular formula: C₂₁H₂₂N₄O₃ Precise molecular weight: 378.17 LC-MS (m/z)=379.21 [M+H]⁺.

### Example 12: Synthesis of 2-(tert-butylamino)-N-(5-cyclopropyl-6-(4-ethynyl-2-hydroxyphenyl)pyridazin-3-yl)aceta mide (Compound 39)

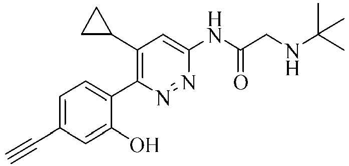

The compound was prepared in accordance with the synthetic method in Step 3 of **Example 7.**

¹HNMR (400 MHz, DMSO-*d₆*) δ(ppm): 10.06 (s, 1H), 7.81 (s, 1H), 7.27-7.25 (d, *J*=8 Hz, 1H), 7.07-7.04 (m, 2H), 4.22 (s, 1H), 1.70-1.64 (m, 1H), 1.08 (s, 9 H), 1.04-0.99 (m, 2H), 0.73-0.69 (m, 2H)

Molecular formula: C₂₁H₂₄N₄O₂ Precise molecular weight: 364.19 LC-MS (m/z)=365.25 [M+H]⁺.

### Example 13: Synthesis of N-(5-cyclopropyl-6-(4-ethynyl-2-hydroxyphenyl)pyridazin-3-yl)-2-((2-methoxyethyl)amin o)acetamide (Compound 40)

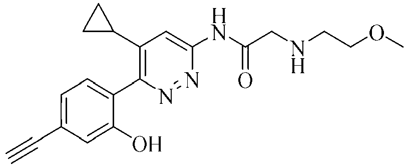

The compound was prepared in accordance with the synthetic method in Step 3 of Example 7.

¹HNMR (400 MHz, DMSO-*d₆*) δ(ppm): 10.05 (s, 1H), 7.82 (s, 1H), 7.27-7.25 (d, J=8 Hz, 1H), 7.06-7.04 (m, 2H), 4.22 (s, 1H), 3.42-3.40 (m, 5 H), 3.25 (s, 3H), 2.74-2.71 (m, 2H), 1.70-1.63 (m, 1H), 1.03-0.98 (m, 2H), 0.73-0.69 (m, 2H).

Molecular formula: C₂₀H₂₀N₄O₃ Precise molecular weight: 366.17 LC-MS (m/z)=367.16 [M+H]⁺.

### Example 14: Synthesis of N-(5-cyclopropyl-6-(4-ethynyl-2-hydroxyphenyl)pyridazin-3-yl)-3-(methylamino)propana mide (Compound 35)

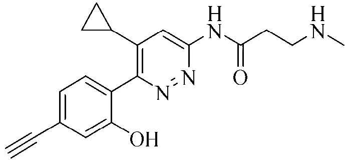

### Step 1 : Synthesis of tert-butyl (3-((5-cyclopropyl-6-(2-(ethoxymethoxy)-4-ethynylphenyl)pyridazin-3-yl)amino)-3-oxopropyl )(methyl)carbamate

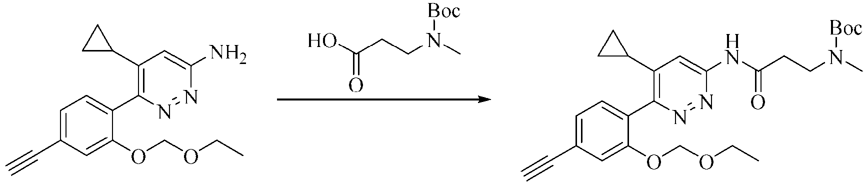

3-((*tert*-Butoxycarbonyl)(methyl)amino)propionic acid (743.4 mg, 3.66 mmol, 2.0 eq.), 1-methyl-1*H*-pyrazole (450.4 mg, 5.48 mmol, 3.0 eq.) and *N,N,N,N*-tetramethylchloroformamidinium hexafluorophosphate (1.02 g, 3.66 mmol, 2.0 eq.) were added to dichloromethane (15.0 mL), the resultant was stirred for 0.5 hours, 5-cyclopropyl-6-(2-(ethoxymethoxy)-4-ethynylphenyl)pyridazin-3-amine (566.0 mg, 1.83 mmol, 1.0 eq.) was then added, and the resultant was reacted at room temperature for 12 hours. TLC monitoring showed the completion of the reaction. Dichloromethane (50.0 mL) was added, and the resultant was washed with water (50.0 mL). The organic phase was dried over anhydrous sodium sulfate and filtered. The filtrate was concentrated under reduced pressure. The crude product was purified by silica gel column chromatography (petroleum ether:ethyl acetate=80:1-2:1), so as to afford the product (300.0 mg, yield: 33.1%).

### Step 2: Synthesis of N-(5-cyclopropyl-6-(4-ethynyl-2-hydroxyphenyl)pyridazin-3-yl)-3-(methylamino)propanamide

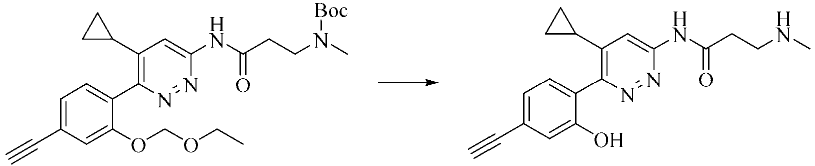

*tert-Butyl(3-((5-cyclopropyl-6-(2-(ethoxymethoxy)-4-ethynylphenyl)pyridazin-3-yl)amin* o)-3-oxopropyl)(methyl)carbamate (300.0 mg, 0.61 mmol, 1.0 eq.) was added to dichloromethane (10.0 mL), trifluoroacetic acid (2.0 mL) was added dropwise, and the resultant was reacted at room temperature for 1 hour. TLC monitoring showed the completion of the reaction. The resultant was adjusted to have a pH value of 7~8 with saturated aqueous sodium bicarbonate solution, and was extracted with dichloromethane (50.0 mL). The organic phase was dried over anhydrous sodium sulfate and filtered. The filtrate was concentrated under reduced pressure. The crude product was purified by preparative thin-layer chromatography, so as to afford the product (37.0 mg, yield: 18.0%).

¹HNMR (400 MHz, DMSO-*d₆*) δ(ppm): 11.30 (s, 1H), 10.21 (s, 1H), 7.91 (s, 1H), 7.26-7.24 (d, *J*=8 Hz, 1H), 7.13-7.12 (m, 1H), 7.05-7.03 (m, 1H), 4.22 (s, 1H), 3.18-3.15 (m, 2H), 2.96-2.93 (m, 2H), 2.55 (s, 3H), 1.69-1.64 (m, 1H), 1.03-0.98 (m, 2H), 0.70-0.66 (m, 2H).

Molecular formula: C₁₉H₂₀N₄O₂ Precise molecular weight: 336.16 LC-MS (m/z)=337.19 [M+H]⁺.

### Example 15: Synthesis of (R)-N-(5-cyclopropyl-6-(4-ethynyl-2-hydroxyphenyl)pyridazin-3-yl)-2-(methylamino)prop anamide (Compound 36)

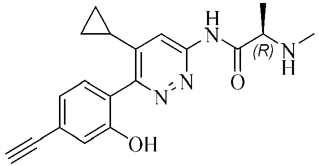

The compound was prepared in accordance with the synthetic method in **Example 14.**

¹HNMR (400 MHz, DMSO-*d₆*) δ(ppm): 10.03 (s, 1H), 7.84 (s, 1H), 7.27-7.25 (d, *J*=8 Hz, 1H), 7.06-7.04 (m, 2H), 4.22 (s, 1H), 3.29-3.26 (m, 3H), 2.28 (s, 3H), 1.70-1.63 (m, 1H), 1.25-1.22 (m, 3H), 1.03-0.98 (m, 2H), 0.74-0.70 (m, 2H).

Molecular formula: C₁₉H₂₀N₄O₂ Precise molecular weight: 336.16 LC-MS (m/z)=337.20 [M+H]⁺.

### Example 16: Synthesis of 1-amino-N-(5-cyclopropyl-6-(4-ethynyl-2-hydroxyphenyl)pyridazin-3-yl)cyclopropane-1-c arboxamide (Compound 54)

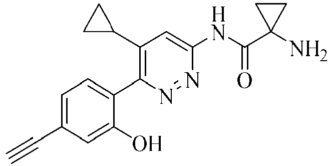

The compound was prepared in accordance with the synthetic method in **Example 14.**

¹HNMR (400 MHz, DMSO-*d₆*) δ(ppm): 10.06 (s, 1H), 7.80 (s, 1H), 7.28-7.26 (d, *J*=8 Hz, 1H), 7.06-7.04 (m, 2H), 4.24 (s, 1H), 3.17-3.16 (d, *J*=4 Hz, 1H), 1.67-1.64 (m, 1H), 1.27-1.23 (m, 2H), 1.00-0.99 (m, 2H), 0.72-0.70 (m, 2H).

Molecular formula: C₁₉H₁₈N₄O₂ Precise molecular weight: 334.14 LC-MS (m/z)=335.18 [M+H]⁺.

### Example 17: Synthesis of (S)-N-(5-cyclopropyl-6-(4-ethynyl-2-hydroxyphenyl)pyridazin-3-yl)-2-(methylamino)prop anamide (Compound 37)

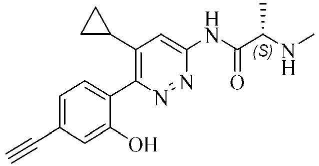

The compound was prepared in accordance with the synthetic method in **Example 14.**

¹HNMR (400 MHz, DMSO-*d₆*) δ(ppm): 10.03 (s, 1H), 7.84 (s, 1H), 7.27-7.25 (d, *J*=8 Hz, 1H), 7.06-7.04 (m, 2H), 4.22 (s, 1H), 3.29-3.26 (m, 3H), 2.28 (s, 3H), 1.70-1.63 (m, 1H), 1.25-1.22 (m, 3H), 1.03-0.98 (m, 2H), 0.74-0.70 (m, 2H)

Molecular formula: C₁₉H₂₀N₄O₂ Precise molecular weight: 336.16 LC-MS (m/z)=337.19 [M+H]⁺.

### Example 18: Synthesis of 2-amino-N-(5-cyclopropyl-6-(4-ethynyl-2-hydroxyphenyl)pyridazin-3-yl)-2-methylpropan amide (Compound 58)

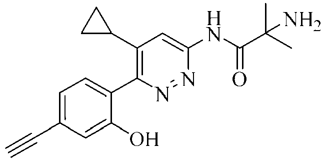

The compound was prepared in accordance with the synthetic method in **Example 14.**

¹HNMR (400 MHz, DMSO-*d₆*) δ(ppm): 10.04 (s, 1H), 7.83 (s, 1H), 7.27-7.25 (d, *J*=8 Hz, 1H), 7.06-7.03 (m, 2H), 5.24 (s, 2H), 4.23 (s, 1H), 1.69-1.65 (m, 1H), 1.31 (s, 6 H), 1.02-1.00 (m, 2H), 0.73-0.72 (m, 2H).

Molecular formula: C₁₉H₂₀N₄O₂ Precise molecular weight: 336.16 LC-MS (m/z)=337.15 [M+H]⁺.

### Example 19: Synthesis of N-(5-cyclopropyl-6-(2-hydroxy-4-(trifluoromethyl)phenyl)pyridazin-3-yl)-2-(methylamino )acetamide (Compound 20)

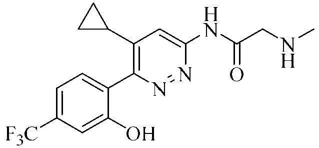

The compound was prepared in accordance with the synthetic methods in Step 2 and Step 4 of Example 3 by using intermediates *tert-butyl* (2-((6-chloro-5-cyclopropylpyridazin-3-yl)amino)-2-oxoethyl)(methyl)carbamate and (2-(ethoxymethoxy)-4-(trifluoromethyl)phenyl)boronic acid as raw materials.

¹HNMR (400 MHz, DMSO-*d₆*) δ(ppm): 7.78 (s, 1H), 7.50-7.48 (d, *J*=8 Hz, 1H), 7.30-7.27 (m, 2H), 2.50-2.47 (m, 3H), 1.66-1.62 (m, 1H), 1.03-1.01 (m, 2H), 0.73-0.71 (m, 2H)

Molecular formula: C₁₇H₁₇F₃N₄O₂ Precise molecular weight: 366.13 LC-MS (m/z)=367.13 [M+H]⁺.

### Example 20: Synthesis of compound N-(6-(2-hydroxy-4-(trifluoromethyl)phenyl)-5-methylpyridazin-3-yl)-2-(methylamino)acet amide (Compound 17)

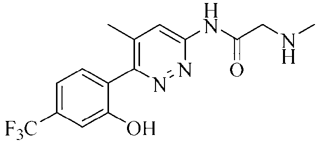

### Step 1 : Synthesis of intermediate 6-(2-methoxy-4-(trifluoromethyl)phenyl)-5-methylpyridazin-3-amine

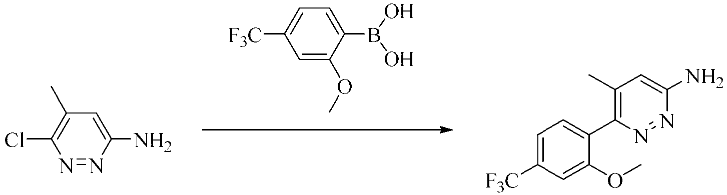

3-Amino-5-methyl-6-chloropyridazine (500 mg, 3.48 mmol, 1.0 eq), (2-methoxy-4-(trifluoromethyl)phenyl)boronic acid (919 mg, 4.18 mmol, 1.2 eq), an aqueous solution (1.5 mL) of potassium carbonate (962 mg, 6.96 mmol, 2.0 eq), and tetrakis(triphenylphosphine)palladium (402 mg, 0.348 mmol, 0.1 eq) were added to 1,4-dioxane (5 mL). Under nitrogen protection, the resultant was reacted at 90°C for 16 hours. TLC monitoring showed the completion of the reaction. The reaction solution was filtered through celite, and the filter cake was rinsed with dichloromethane (20 mL) and methanol (20 mL). The filtrate was dried over anhydrous magnesium sulfate and filtered. The filtrate was concentrated to give a crude product, which was purified by silica gel column chromatography (dichloromethane:methanol=100:1~50:1), so as to afford the product (745 mg, yield: 75.6%).

### Step 2: Synthesis of intermediate 2-bromo-N-(6-(2-methoxy-4-(trifluoromethyl)phenyl)-5-methylpyridazin-3-yl)acetamide

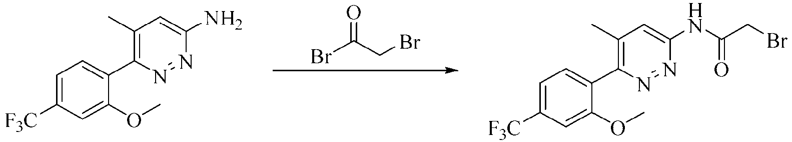

6-(2-Methoxy-4-(trifluoromethyl)phenyl)-5-methylpyridazin-3-amine (745 mg, 2.63 mmol, 1.0 eq) was dissolved in dichloromethane (20 mL), and *N,N*-diisopropylethylamine (680 mg, 5.26 mmol, 2.0 eq) was added thereto. At 0°C, bromoacetyl bromide (797 mg, 3.95 mmol, 1.5 eq) was added dropwise thereto, and the resultant was reacted at 0°C for 5 minutes. TLC monitoring showed the completion of the reaction. The reaction solution was poured into water (10 mL), and the resultant was extracted with dichloromethane (15 mL×3). The organic phases were combined, dried over anhydrous magnesium sulfate, and filtered. The filtrate was concentrated to give a crude product, which was used directly in the next step.

### Step 3: Synthesis of intermediate 2-bromo-N-(6-(2-hydroxy-4-(trifluoromethyl)phenyl)-5-methylpyridazin-3-yl)acetamide

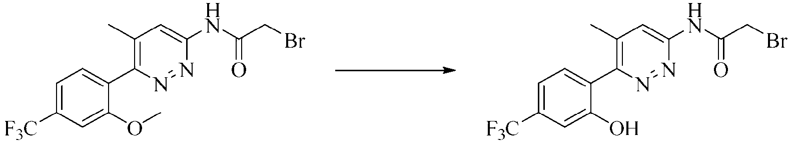

*2-Bromo-N-(6-(2-methoxy-4-(trifluoromethyl)phenyl)-5-methylpyridazin-3-yl)acetamide* (crude, 2.63 mmol, 1.0 eq) was dissolved in dichloromethane (10 mL), boron tribromide (3.3 g, 13.15 mmol, 5.0 eq) was added dropwise thereto, and the resultant was reacted at 25°C for 3 hours. TLC monitoring showed the completion of the reaction. The resultant was diluted by adding dichloromethane (40 mL). At 0°C, methanol (5 mL) was slowly added dropwise thereto to quench the excessive boron tribromide. After completion of the dropwise addition, the reaction solution was poured into saturated aqueous sodium bicarbonate solution (50 mL), and the resultant was extracted with dichloromethane (20 mL×5). The organic phases were combined, dried over anhydrous magnesium sulfate, and filtered. The filtrate was concentrated, so as to afford the product (741 mg, yield: 71.9%).

### Step 4: Synthesis of compound N-(6-(2-hydroxy-4-(trifluoromethyl)phenyl)-5-methylpyridazin-3-yl)-2-(methylamino)acetami de

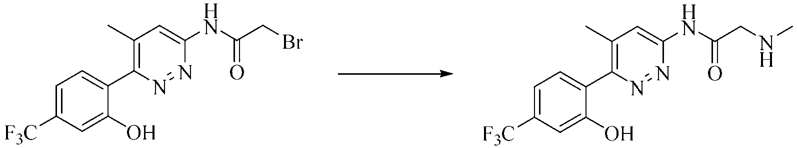

2-Bromo-*N*-(6-(2-hydroxy-4-(trifluoromethyl)phenyl)-5-methylpyridazin-3-yl)acetamide (200 mg, 0.513 mmol, 1.0 eq) was added to a mixed solvent of acetonitrile and methanol (2 mL/2 mL). *N,N*-diisopropylethylamine (199 mg, 1.539 mmol, 3.0 eq) and a 27% solution of methylamine in methanol (177 mg, 1.539 mmol, 3.0 eq) were added to the resulting suspension, and the resultant was reacted at 25°C for 0.5 hours. TLC monitoring showed the completion of the reaction. The reaction solution was poured into saturated aqueous ammonium chloride solution (10 mL), and the resultant was extracted with dichloromethane (10 mL×3). The organic phases were combined, dried over anhydrous magnesium sulfate, and filtered. The filtrate was concentrated to give a crude product, which was purified by preparative thin-layer chromatography (dichloromethane:methanol=10:1), so as to afford the product (30 mg, yield: 17.1%).

¹H-NMR (400 MHz, DMSO-*d₆*) δ(ppm): 8.27 (s, 1H), 7.48-7.46 (d, 1H), 7.30-7.27 (m, 2H), 3.46 (s, 2H), 2.39 (s, 3H), 2.20 (s, 3H).

Molecular formula: C₁₅H₁₅F₃N₄O₂ Precise molecular weight: 340.11 LC-MS (m/z): 341.14 [M+H]⁺ .

### Example 21: Synthesis of N-(6-(2-hydroxy-4-(trifluoromethyl)phenyl)-5-isopropylpyridazin-3-yl)-2-(methylamino)a cetamide (Compound 19)

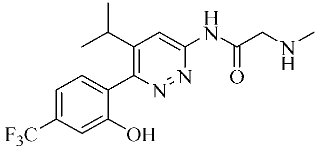

The compound was prepared in accordance with the synthetic method of **Example 19.**

¹HNMR (400 MHz, DMSO-*d₆*) δ(ppm): 8.37 (s, 1H), 7.48-7.46 (d, *J*=8 Hz, 1H), 7.29-7.23 (m, 2H), 3.37 (s, 3H), 2.76-2.69 (m, 1H), 1.11-1.09 (d, *J*=8 Hz, 6H).

Molecular formula: C₁₇H₁₉F₃N₄O₂ Precise molecular weight: 368.15 LC-MS (m/z)=369.17 [M+H]⁺.

### Example 22: Synthesis of 2-amino-N-(5-cyclopropyl-6-(4-ethynyl-2-hydroxyphenyl)pyridazin-3-yl)acetamide (Compound 13)

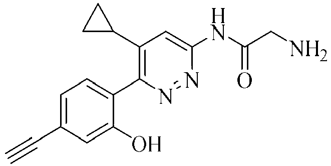

The compound was prepared in accordance with the synthetic method in **Example 14.**

¹HNMR (400 MHz, DMSO-*d₆*) δ(ppm): 7.78 (s, 1H), 7.28-7.26 (d, *J*=8 Hz, 1H), 7.07-7.04 (m, 2H), 4.23 (s, 1H), 3.58 (s, 2H), 1.70-1.66 (m, 1H), 1.05-1.01 (m, 2H), 0.72-0.69 (m, 2H)

Molecular formula: C₁₇H₁₆N₄O₂ Precise molecular weight: 308.13 LC-MS (m/z)=309.13 [M+H]⁺.

### Example 23: Synthesis of (R)-N-(5-cyclopropyl-6-(4-ethynyl-2-hydroxyphenyl)pyridazin-3-yl)azetidine-2-carboxami de (Compound 42)

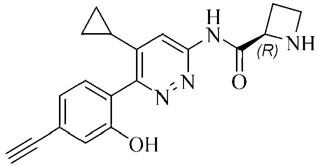

The compound was prepared in accordance with the synthetic method in **Example 14.**

¹HNMR (400 MHz, DMSO-*d₆*) δ(ppm): 10.49 (s, 1H), 7.83 (s, 1H), 7.28-7.26 (d, *J*=8 Hz, 1H), 7.07-7.04 (m, 2H), 4.37-4.33 (m, 1H), 4.22 (s, 1H), 3.64-3.58 (m, 1H), 3.30-3.25 (m, 1H), 2.62-2.53 (m, 1H), 2.36-2.28 (m, 1H), 1.71-1.65 (m, 1H), 1.04-0.99 (m, 1H), 0.75-0.71 (m, 2H).

Molecular formula: C₁₉H₁₈N₄O₂ Precise molecular weight: 334.14 LC-MS (m/z)=335.14 [M+H]⁺.

### Example 24: Synthesis of (R)-N-(5-cyclopropyl-6-(4-ethynyl-2-hydroxyphenyl)pyridazin-3-yl)pyrrolidine-2-carboxa mide (Compound 43)

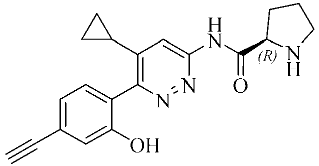

The compound was prepared in accordance with the synthetic method in **Example 14.**

¹HNMR (400 MHz, DMSO-*d₆*) δ(ppm): 10.71 (s, 1H), 10.21 (s, 1H), 7.82 (s, 1H), 7.27-7.25 (d, *J*=8 Hz, 1H), 7.06-7.04 (m, 2H), 4.22 (s, 1H), 3.88-3.85 (m, 1H), 2.99-2.88 (m, 2H), 2.13-2.08 (m, 1H), 1.87-1.80 (m, 1H), 1.72-1.64 (m, 3H), 1.03-0.98 (m, 2H), 0.73-0.69 (m, 2H).

Molecular formula: C₂₀H₂₀N₄O₂ Precise molecular weight: 348.16 LC-MS (m/z)=349.15 [M+H]⁺.

### Example 25: Synthesis of (R)-2-amino-N-(5-cyclopropyl-6-(4-ethynyl-2-hydroxyphenyl)pyridazin-3-yl)propanamide (Compound 44)

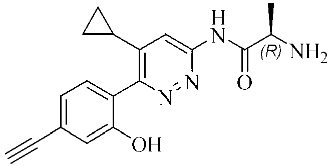

The compound was prepared in accordance with the synthetic method in **Example 14.**

¹HNMR (400 MHz, DMSO-*d₆*) δ(ppm): 7.83 (s, 1H), 7.27-7.25 (d, *J*=8 Hz, 1H), 7.06-7.04 (m, 2H), 4.22 (s, 1H), 3.58-3.53 (m, 1H), 1.69-1.64 (m, 1H), 1.25-1.23 (m, 3H), 1.03-0.98 (m, 2H), 0.73-0.69 (m, 2H).

Molecular formula: C₁₈H₁₈N₄O₂ Precise molecular weight: 322.14 LC-MS (m/z)=323.14 [M+H]⁺.

### Example 26: Synthesis of (R)-N-(5-cyclopropyl-6-(4-ethynyl-2-hydroxyphenyl)pyridazin-3-yl)-3-methyl-2-(methyla mino)butanamide (Compound 45)

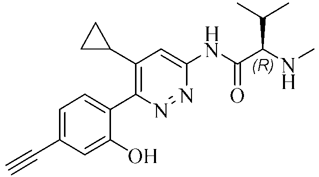

The compound was prepared in accordance with the synthetic method in **Example 14.**

¹HNMR (400 MHz, DMSO-*d₆*) δ(ppm): 10.03 (s, 1H), 7.87 (s, 1H), 7.28-7.26 (d, *J*=8 Hz, 1H), 7.07-7.04 (m, 2H), 4.22 (s, 1H), 2.97-2.96 (d, *J*=4 Hz, 1H), 2.28 (s, 3H), 1.92-1.87 (m, 1H), 1.69-1.65 (m, 1H), 1.03-0.98 (m, 2H), 0.94-0.92 (d, J=8 Hz, 6 H), 0.75-0.71 (m, 2H).

Molecular formula: C₂₁H₂₄N₄O₂ Precise molecular weight: 364.19 LC-MS (m/z)=365.20 [M+H]⁺.

### Example 27: Synthesis of (R)-2-amino-N-(5-cyclopropyl-6-(4-ethynyl-2-hydroxyphenyl)pyridazin-3-yl)-3-methoxyp ropanamide (Compound 51)

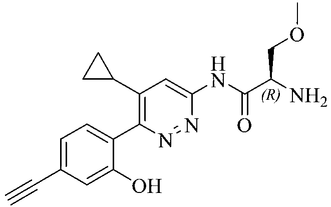

The compound was prepared in accordance with the synthetic method in **Example 14.**

¹HNMR (400 MHz, DMSO-*d₆*) δ(ppm): 10.04 (s, 1H), 7.83 (s, 1H), 7.28-7.26 (d, *J*=8 Hz, 1H), 7.07-7.04 (m, 2H), 4.22 (s, 1H), 3.69-3.66 (m, 1H), 3.58-3.49 (m, 2H), 3.27 (s, 3H), 1.70-1.64 (m, 1H), 1.04-0.99 (m, 2H), 0.73-0.69 (m, 2H).

Molecular formula: C₁₉H₂₀N₄O₃ Precise molecular weight: 352.12 LC-MS (m/z)=353.15 [M+H]⁺.

### Example 28: Synthesis of (R)-2-amino-N-(5-cyclopropyl-6-(4-ethynyl-2-hydroxyphenyl)pyridazin-3-yl)-3-phenylpro panamide (Compound 52)

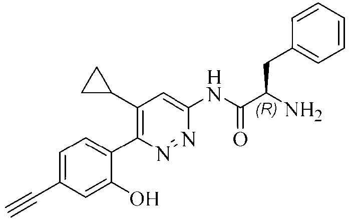

The compound was prepared in accordance with the synthetic method in **Example 14.**

¹HNMR (400 MHz, DMSO-*d₆*) δ(ppm): 10.07 (s, 1H), 7.81 (s, 1H), 7.31-7.20 (m, 6 H), 7.06-7.04 (m, 2H), 4.24 (s, 1H), 3.75-3.71 (m, 1H), 3.08-3.03 (m, 1H), 2.75-2.70 (m, 1H), 1.69-1.65 (m, 1H), 1.02-1.00 (m, 2H), 0.71-0.70 (m, 2H).

Molecular formula: C₂₄H₂₂N₄O₂ Precise molecular weight: 398.17 LC-MS (m/z)=399.14 [M+H]⁺.

### Example 29: Synthesis of (R)-2-amino-2-cyclopropyl-N-(5-cyclopropyl-6-(4-ethynyl-2-hydroxyphenyl)pyridazin-3-y l)acetamide (Compound 53)

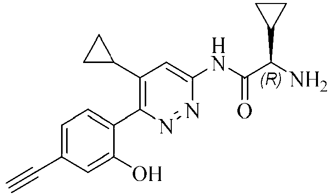

The compound was prepared in accordance with the synthetic method in **Example 14.**

¹HNMR (400 MHz, DMSO-*d₆*) δ(ppm): 10.08 (s, 1H), 7.85 (s, 1H), 7.28-7.26 (d, *J*=8 Hz, 1H), 7.06-7.04 (m, 2H), 4.23 (s, 1H), 3.00-2.98 (d, J=8 Hz, 1H), 1.68-1.65 (m, 1H), 1.05-1.00 (m, 3H), 0.73-0.71 (m, 2H), 0.53-0.50 (m, 1H), 0.42-0.40 (m, 2H), 0.30-0.27 (m, 1H).

Molecular formula: C₂₀H₂₀N₄O₂ Precise molecular weight: 348.16 LC-MS (m/z)=349.15 [M+H]⁺.

### Example 30: Synthesis of (R)-2-amino-N-(5-cyclopropyl-6-(4-ethynyl-2-hydroxyphenyl)pyridazin-3-yl)-3-methylbut anamide (Compound 56)

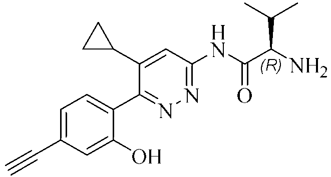

The compound was prepared in accordance with the synthetic method in **Example 14.**

¹HNMR (400 MHz, DMSO-*d₆*) δ(ppm): 10.04 (s, 1H), 7.85 (s, 1H), 7.27-7.25 (d, *J*=8 Hz, 1H), 7.06-7.04 (m, 2H), 4.23 (s, 1H), 3.29-3.28 (m, 1H), 2.03-1.98 (m, 1H), 1.68-1.65 (m, 1H), 1.01-0.99 (m, 2H), 0.95-0.94 (m, 3H), 0.86-0.85 (m, 3H), 0.72-0.71 (m, 2H).

Molecular formula: C₂₀H₂₂N₄O₂ Precise molecular weight: 350.17 LC-MS (m/z)=351.20 [M+H]⁺.

### Example 31: Synthesis of compound N-(6-(2-hydroxy-4-(propyn-1-yl)phenyl)-5-methylpyridazin-3-yl)-2-(methylamino)acetam ide (Compound 46)

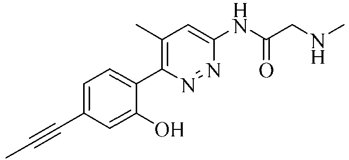

The compound was prepared in accordance with the synthetic method of **Example 20** by using intermediates (2-methoxy-4-(propyn-1-yl)phenyl)boronic acid and 3-amino-5-methyl-6-chloropyridazine as raw materials.

¹H-NMR (400 MHz, DMSO-*d₆*) δ(ppm): 8.21 (s, 1H), 7.20-7.18 (d, 1H), 6.97-6.95 (m, 2H), 3.61 (s, 2H), 2.46 (s, 3H), 2.19 (s, 3H), 2.07 (s, 3H).

Molecular formula: C₁₇H₁₈N₄O₂ Precise molecular weight: 310.14 LC-MS (m/z): 311.13 [M+H]⁺.

### Example 32: Synthesis of N-(5-cyclopropyl-6-(2-hydroxy-4-(prop-1-yn-1-yl)phenyl)pyridazin-3-yl)-2-(methylamino) acetamide (Compound 47)

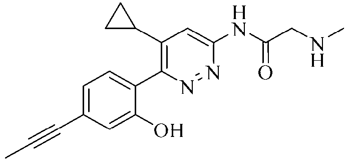

The compound was prepared in accordance with the synthetic method of **Example 20** by using intermediates (2-methoxy-4-(propyn-1-yl)phenyl)boronic acid and 6-chloro-5-cyclopropylpyridazin-3-amine as raw materials.

¹HNMR (400 MHz, DMSO-*d₆*) δ(ppm): 9.93 (s, 1H), 7.80 (s, 1H), 7.22-7.20 (d, *J*=8 Hz, 1H), 6.96-6.94 (m, 2H), 3.39(s, 2H), 2.35 (s, 3H), 2.06 (s, 3H), 1.71-1.66 (m, 1H), 1.03-0.99 (m, 2H), 0.73-0.69 (m, 2H).

Molecular formula: C₁₉H₂₀N₄O₂ Precise molecular weight: 336.16 LC-MS (m/z)=337.18 [M+H]⁺.

### Example 33: Synthesis of N-(5-cyclopropyl-6-(4-ethynyl-2-hydroxyphenyl)pyridazin-3-yl)-2-hydroxyacetamide (Compound 15)

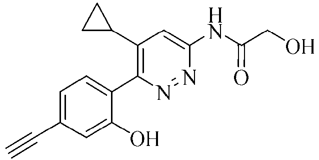

### Step 1: Synthesis of 2-((tert-butyldimethylsilyl)oxy)acetic acid

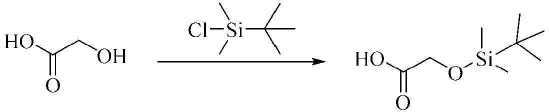

2-Hydroxyacetic acid (2.0 g, 26.29 mmol, 1.0 eq.), *tert*-butyldimethylchlorosilane (5.1 g, 34.18 mmol, 1.3 eq.) and imidazole (2.7 g, 39.45 mmol, 1.5 eq.) were added to DMF (20.0 mL), and the resultant was reacted at room temperature for 14 hours. TLC monitoring showed the completion of the reaction. Ethyl acetate (50.0 mL) was added, and the resultant was washed with water (50.0 mL). The organic phase was dried over anhydrous sodium sulfate and filtered. The filtrate was concentrated under reduced pressure, so as to afford the product (4.4 g, yield: 88.0%).

### Step 2: Synthesis of 2-((tert-butyldimethylsilyl)oxy)-N-(5-cyclopropyl-6-(2-(ethoxymethoxy)-4-ethynylphenyl)pyri dazin-3-yl)acetamide

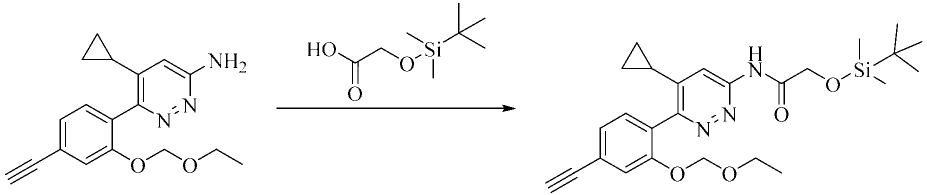

The compound was synthesized in accordance with the method in Step 3 of Example 5, and the crude product was purified by silica gel column chromatography (petroleum ether:ethyl acetate=10:1-3:1), so as to afford the product (600.0 mg, yield: 76.1%).

### Step 3: Synthesis of N-(5-cyclopropyl-6-(4-ethynyl-2-hydroxyphenyl)pyridazin-3-yl)-2-hydroxyacetamide

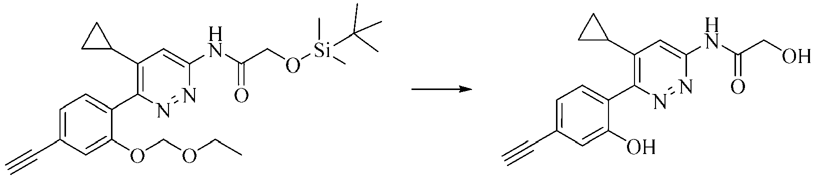

The compound was synthesized in accordance with the preparation method in Step 4 of Example 3, and the crude product was purified by silica gel column chromatography (dichloromethane:methanol=80:1-50:1), so as to afford the product (250.0 mg, yield: 71.0%).

¹HNMR (400 MHz, DMSO-*d₆*) δ(ppm): 10.32 (s, 1H), 10.05 (s, 1H), 7.79 (s, 1H), 7.28-7.26 (d, *J*=8 Hz, 1H), 7.07-7.04 (m, 2H), 5.70 (s, 1H), 4.21 (s, 1H), 4.11-4.10 (m, 2H), 1.70-1.64 (m, 1H), 1.04-0.99 (m, 2H), 0.74-0.70 (m, 2H).

Molecular formula: C₁₇H₁₅N₃O₃ Precise molecular weight: 309.11 LC-MS (m/z)=310.11 [M+H]⁺.

### Example 34: Synthesis of (R)-2-amino-N-(5-cyclopropyl-6-(4-ethynyl-2-hydroxyphenyl)pyridazin-3-yl)-3-hydroxypr opanamide (Compound 55)

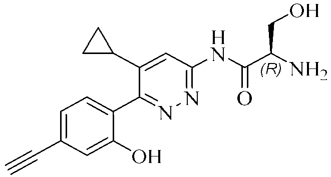

The compound was prepared in accordance with the synthetic method of **Example 33.**

¹HNMR (400 MHz, DMSO-*d₆*) δ(ppm): 7.84 (s, 1H), 7.27-7.25 (d, *J*=8 Hz, 1H), 7.05-7.03 (m, 2H), 4.91 (s, 1H), 4.22 (s, 1H), 3.62-3.59 (m, 2H), 3.52-3.49 (m, 1H), 1.69-1.67 (m, 1H), 1.02-1.00 (m, 2H), 0.71-0.70 (m, 2H).

Molecular formula: C₁₈H₁₈N₄O₃ Precise molecular weight: 338.14 LC-MS (m/z)=339.16 [M+H]⁺.

### Example 35: Synthesis of (R)-2-amino-N-(5-cyclopropyl-6-(2-hydroxy-4-(trifluoromethyl)phenyl)pyridazin-3-yl)pro panamide (Compound 50)

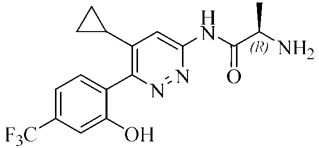

### Step 1: Synthesis of tert-butyl (R)-(1-((6-chloro-5-cyclopropylpyridazin-3-yl)amino)-1-oxopropan-2-yl)carbamate

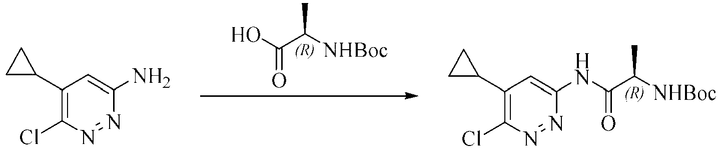

*N*-(*tert*-Butoxycarbonyl)-D-alanine (669.0 mg, 3.54 mmol, 2.0 eq.), *N,N*-diisopropylethylamine (457.0 mg, 3.54 mmol, 2.0 eq.) and HATU(1.34 g, 3.54 mmol, 2.0 eq.) were added to DMF (10.0 mL), the resultant was stirred for 1 hour, 6-chloro-5-cyclopropylpyridazin-3-amine (300.0 mg, 1.77 mmol, 1.0 eq.) was then added, and the resultant was reacted at room temperature for 12 hours. TLC monitoring showed the completion of the reaction. Ethyl acetate (50.0 mL) was added, and the resultant was washed with saturated aqueous ammonium chloride solution (50.0 mL). The organic phase was dried over anhydrous sodium sulfate and filtered. The filtrate was concentrated under reduced pressure. The crude product was purified by silica gel column chromatography (dichloromethane:methanol=100:1-80:1), so as to afford the product (501.0 mg, yield: 83.2%).

### Step 2: Synthesis of tert-butyl (R)-(1-((5-cyclopropyl-6-(2-(ethoxymethoxy)-4-(trifluoromethyl)phenyl)pyridazin-3-yl)amino) -1-oxopropan-2-yl)carbamate

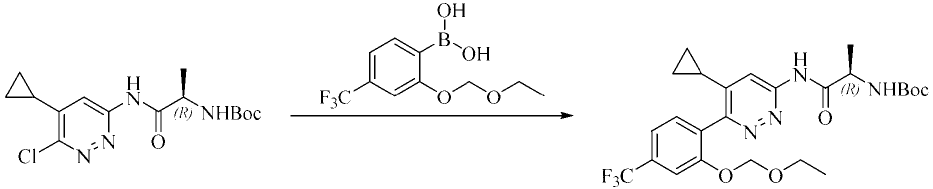

The compound was prepared in accordance with the preparation method in Step 2 of Example 3, and the crude product was purified by silica gel column chromatography (dichloromethane:methanol=100:1-80:1), so as to afford the product (250.0 mg, yield: 34.5%).

### Step 3: Synthesis of (R)-2-amino-N-(5-cyclopropyl-6-(2-hydroxy-4-(trifluoromethyl)phenyl)pyridazin-3-yl)propana mide

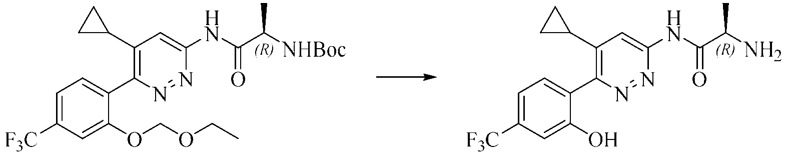

The compound was prepared in accordance with the preparation method in Step 4 of Example 3, and the crude product was purified by preparative thin-layer chromatography, so as to afford the product (30.0 mg, yield: 43.1%).

¹HNMR (400 MHz, DMSO-*d₆*) δ(ppm): 7.86 (s, 1H), 7.50-7.48 (d, *J*=8 Hz, 1H), 7.29-7.25 (m, 2H), 3.57-3.56 (m, 1H), 1.66-1.63 (m, 1H), 1.26-1.24 (m, 4 H), 1.04-0.99 (m, 2H), 0.75-0.71 (m, 2H).

Molecular formula: C₁₇H₁₇F₃N₄O₂ Precise molecular weight: 366.13 LC-MS (m/z)=367.14 [M+H]⁺.

### Example 36: Synthesis of (2R, 3S)-2-amino-N-(5-cyclopropyl-6-(4-ethynyl-2-hydroxyphenyl)pyridazin-3-yl)-3-hydroxyb utanamide (Compound 57)

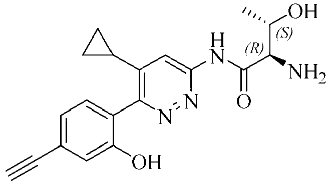

### Step 1: Synthesis of (9H-fluoren-9-yl)methyl [(2R,3S)-1-((5-cyclopropyl-6-(2-(ethoxymethoxy)-4-ethynylphenyl)pyridazin-3-yl)amino)-3-h ydroxy-1-oxobutan-2-yl]carbamate

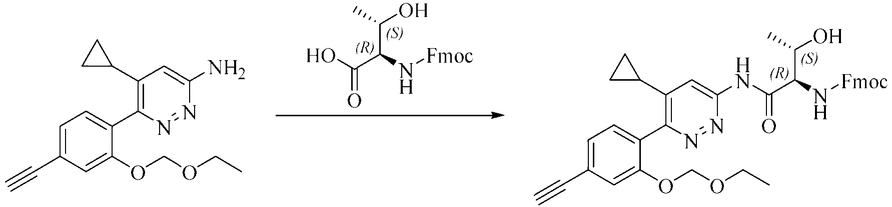

The compound was prepared in accordance with the preparation method in Step 3 of **Example 6,** and the crude product was purified by silica gel column chromatography (dichloromethane:methanol=100:1-60:1), so as to afford the product (300.0 mg, yield: 73.5%).

### Step 2: Synthesis of (9H-fluoren-9-yl)methyl [(2R,3S)-1-((5-cyclopropyl-6-(4-ethynyl-2-hydroxyphenyl)pyridazin-3-yl)amino)-3-hydroxy-1-oxobutan-2-yl]carbamate

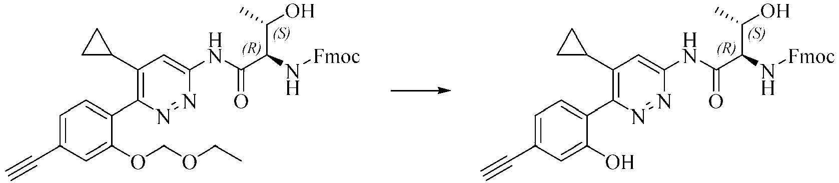

The compound was prepared in accordance with the method in Step 4 of **Example 3,** and the crude product was purified by silica gel column chromatography (dichloromethane:methanol=100:1-40:1), so as to afford the product (160.0 mg, yield: 58.8%).

### Step 3: Synthesis of (2R,3S)-2-amino-N-(5-cyclopropyl-6-(4-ethynyl-2-hydroxyphenyl)pyridazin-3-yl)-3-hydroxyb utanamide

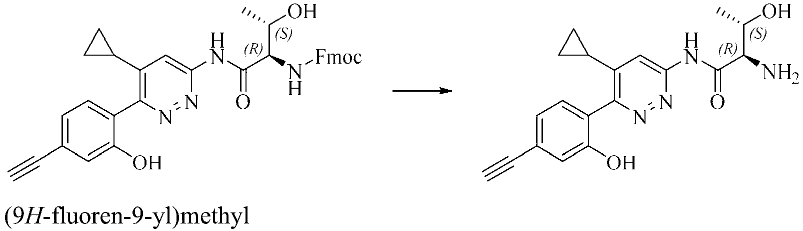

[(2*R*,3*S*)-1-((5-cyclopropyl-6-(4-ethynyl-2-hydroxyphenyl)pyridazin-3-yl)amino)-3-hydroxy-1-oxobutan-2-yl]carbamate (150.0 mg, 0.26 mmol, 1.0 eq.) was added to methanol (30.0 mL), piperidine (30.0 mL) was added dropwise, and the resultant was reacted at room temperature for 18 hours. TLC monitoring showed the completion of the reaction. The resultant was concentrated under reduced pressure. Dichloromethane (50.0 mL) was added, and the resultant was washed with saturated aqueous ammonium chloride solution (50.0 mL). The organic phase was dried over anhydrous sodium sulfate and filtered. The filtrate was concentrated under reduced pressure. The crude product was purified by preparative thin-layer chromatography, so as to afford the product (80.0 mg, yield: 86.9%).

¹HNMR (400 MHz, DMSO-*d₆*) δ(ppm): 10.04 (s, 1H), 7.85 (s, 1H), 7.27-7.25 (d, *J*=8 Hz, 1H), 7.06-7.04 (m, 2H), 4.79 (s, 1H), 4.23 (s, 1H), 4.07-4.05 (m, 1H), 3.28 (s, 1H), 1.69-1.65 (m, 1H), 1.15-1.13 (d, *J*=8 Hz, 3H), 1.02-1.00 (m, 2H), 0.71-0.70 (m, 2H).

Molecular formula: C₁₉H₂₀N₄O₃ Precise molecular weight: 352.15 LC-MS (m/z)=353.17 [M+H]⁺.

### Example 37: Synthesis of (2R,3R)-2-amino-N-(5-cyclopropyl-6-(4-ethynyl-2-hydroxyphenyl)pyridazin-3-yl)-3-hydro xybutanamide (Compound 59)

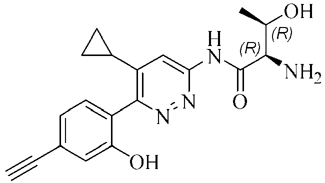

The compound was prepared in accordance with the synthetic method of **Example 36.**

¹HNMR (400 MHz, DMSO-*d₆*) δ(ppm): 10.16 (s, 1H), 7.75 (s, 1H), 7.28-7.26 (d, *J*=8 Hz, 1H), 7.08-7.05 (m, 2H), 4.24 (s, 1H), 4.14 (s, 1H), 4.00-3.99 (m, 1H), 3.38 (s, 1H), 1.72-1.66 (m, 1H), 1.19-1.17 (d, *J*=8 Hz, 3H), 1.04-1.02 (m, 2H), 0.71-0.70 (m, 2H).

Molecular formula: C₁₉H₂₀N₄O₃ Precise molecular weight: 352.15 LC-MS (m/z)=353.16 [M+H]⁺.

### Example 38: Synthesis of N-(5-cyclobutyl-6-(2-hydroxy-4-(trifluoromethyl)phenyl)pyridazin-3-yl)-2-(methylamino) acetamide (Compound 61)

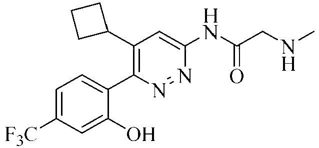

The compound was prepared in accordance with the synthetic methods in Step 1, Step 2 and Step 4 of **Example 3** by using *N*-(*tert*-Butoxycarbonyl)-*N*-methylglycine as well as intermediates 6-chloro-5-cyclobutylpyridazin-3-amine and (2-(ethoxymethoxy)-4-(trifluoromethyl)phenyl)boronic acid as raw materials.

¹HNMR (400 MHz, DMSO-*d₆*) δ(ppm): 8.34 (s, 1H), 7.42-7.40 (d, *J*=8 Hz, 1H), 7.27-7.24 (m, 2H), 3.55-3.45 (m, 4 H), 2.38 (s, 3H), 1.97-1.85 (m, 5 H), 1.72-1.68 (m, 1H).

Molecular formula: C₁₈H₁₉F₃N₄O₂ Precise molecular weight: 380.15 LC-MS (m/z)=381.12 [M+H]⁺.

### Example 39: Synthesis of N-(6-(4-ethynyl-2-hydroxyphenyl)-5-isopropylpyridazin-3-yl)-2-(methylamino)acetamide (Compound 16)

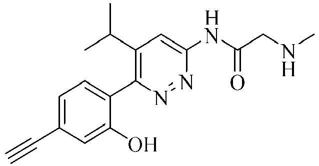

The compound was prepared in accordance with the synthetic method of **Example** 3 by using *N*-(*tert*-Butoxycarbonyl)-*N*-methylglycine as well as intermediates 6-chloro-5-isopropylpyridazin-3-amine and 3-(ethoxymethoxy)-4-(4,4,5,5-tetramethyl-1,3,2-dioxaborolan-2-yl)benzaldehyde as raw materials.

¹HNMR (400 MHz, DMSO-*d₆*) δ(ppm): 10.03 (s, 1H), 8.34 (s, 1H), 7.24-7.22 (d, *J*=8 Hz, 1H), 7.06-7.03 (m, 2H), 4.24 (s, 1H), 3.36 (s, 3H), 2.77-2.72 (m, 1H), 2.34 (s, 3H), 1.10-1.08 (d, *J*=8 Hz, 6H).

Molecular formula: C₁₈H₂₀N₄O₂ Precise molecular weight: 324.16 LC-MS (m/z)=325.16 [M+H]⁺.

### Example 40: Synthesis of N-(5-cyclobutyl-6-(4-ethynyl-2-hydroxyphenyl)pyridazin-3-yl)-2-(methylamino)acetamide (Compound 62)

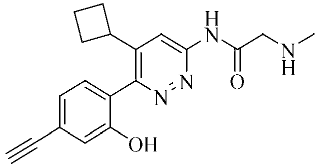

The compound was prepared in accordance with the synthetic method of **Example 3** by using *N*-(*tert*-Butoxycarbonyl)-*N*-methylglycine as well as intermediates 6-chloro-5-cyclobutylpyridazin-3-amine and 3-(ethoxymethoxy)-4-(4,4,5,5-tetramethyl-1,3,2-dioxaborolan-2-yl)benzaldehyde as raw materials.

¹HNMR (400 MHz, DMSO-*d₆*) δ(ppm): 8.32 (s, 1H), 7.18-7.16 (d, *J*=8 Hz, 1H), 7.02-7.01 (m, 2H), 4.23 (s, 1H), 3.54-3.50 (m, 1H), 3.37 (s, 3H), 2.34 (s, 3H), 1.96-1.82 (m, 5 H), 1.70-1.66 (m, 1H).

Molecular formula: C₁₉H₂₀N₄O₂ Precise molecular weight: 336.16 LC-MS (m/z)=337.17 [M+H]⁺.

### Example 41: Synthesis of (R)-2-amino-N-(5-cyclobutyl-6-(4-ethynyl-2-hydroxyphenyl)pyridazin-3-yl)propanamide (Compound 65)

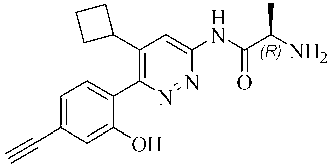

The compound was prepared in accordance with the synthetic method of **Example 3.**

¹HNMR (400 MHz, DMSO-*d₆*) δ(ppm): 8.34 (s, 1H), 7.18-7.16 (d, *J*=8 Hz, 1H), 7.02-7.00 (m, 2H), 4.22 (s, 1H), 3.61-3.50 (m, 2H), 1.96-1.66 (m, 6 H), 1.27-1.25 (d, *J*=8 Hz, 3H).

Molecular formula: C₁₉H₂₀N₄O₂ Precise molecular weight: 336.16 LC-MS (m/z)=337.10 [M+H]⁺.

### Example 42: Synthesis of (R)-2-amino-N-(6-(4-ethynyl-2-hydroxyphenyl)-5-isopropylpyridazin-3-yl)propanamide (Compound 66)

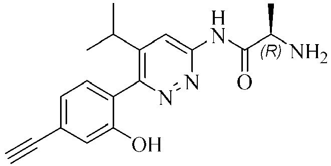

The compound was prepared in accordance with the synthetic method of **Example 3.**

¹HNMR (400 MHz, DMSO-*d₆*) δ(ppm): 8.35 (s, 1H), 7.23-7.21 (d, *J*=8 Hz, 1H), 7.05-7.02 (m, 2H), 4.22 (s, 1H), 3.60-3.55 (m, 1H), 2.78-2.74 (m, 1H), 1.27-1.25 (d, *J*=8 Hz, 3H), 1.10-1.08 (d, *J*=8 Hz, 6 H).

Molecular formula: C₁₈H₂₀N₄O₂ Precise molecular weight: 324.16 LC-MS (m/z)=325.14 [M+H]⁺.

### Example 43: Synthesis of (R)-2-amino-N-(5-cyclobutyl-6-(2-hydroxy-4-(trifluoromethyl)phenyl)pyridazin-3-yl)prop anamide (Compound 63)

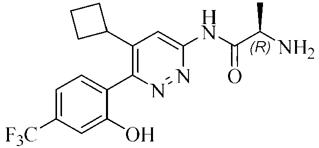

The compound was prepared in accordance with the synthetic methods in Step 1, Step 2 and Step 4 of **Example** 3 by using *N*-(*tert*-butoxycarbonyl)-D-alanine as well as intermediates 6-chloro-5-cyclobutylpyridazin-3-amine and (2-(ethoxymethoxy)-4-(trifluoromethyl)phenyl)boronic acid as raw materials.

¹HNMR (400 MHz, DMSO-*d₆*) δ(ppm): 8.37 (s, 1H), 7.41-7.39 (d, *J*=8 Hz, 1H), 7.25-7.21 (m, 2H), 3.62-3.47 (m, 2H), 1.97-1.82 (m, 5 H), 1.73-1.68 (m, 1H), 1.27-1.26 (d, *J*=4 Hz, 3H).

Molecular formula: C₁₈H₁₉F₃N₄O₂ Precise molecular weight: 380.15 LC-MS (m/z)=381.12 [M+H]⁺.

### Example 44: Synthesis of (R)-2-amino-N-(6-(2-hydroxy-4-(trifluoromethyl)phenyl)-5-isopropylpyridazin-3-yl)propa namide (Compound 64)

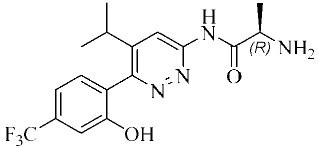

The compound was prepared in accordance with the synthetic methods in Step 1, Step 2 and Step 4 of **Example 3.**

¹HNMR (400 MHz, DMSO-*d₆*) δ(ppm): 8.38 (s, 1H), 7.47-7.45 (d, *J*=8 Hz, 1H), 7.28-7.22 (m, 2H), 3.62-3.56 (m, 1H), 2.75-2.72 (m, 1H), 1.27-1.25 (d, *J*=8 Hz, 3H), 1.11-1.09 (d, *J*=8 Hz, 6 H).

Molecular formula: C₁₇H₁₉F₃N₄O₂ Precise molecular weight: 368.15 LC-MS (m/z)=369.13 [M+H]⁺.

### Example 45: Synthesis of N-(6-(4-chloro-2-hydroxyphenyl)-5-cyclopropylpyridazin-3-yl)-2-(methylamino)acetamid e (Compound 67)

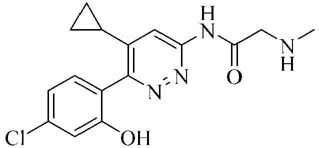

The compound was prepared in accordance with the synthetic methods in Step 1, Step 2 and Step 4 of **Example 3.**

¹HNMR (400 MHz, DMSO-*d₆*) δ(ppm): 7.82 (s, 1H), 7.29-7.27 (d, *J*=8 Hz, 1H), 6.99 (s, 2H), 3.34 (s, 3H), 2.33 (s, 3H), 1.67-1.66 (m, 1H), 1.02-1.00 (m, 2H), 0.72-0.71 (m, 2H).

Molecular formula: C₁₆H₁₇ClN₄O₂ Precise molecular weight: 332.10 LC-MS (m/z)=333.06 [M+H]⁺.

### Example 46: Synthesis of N-(5-cyclopropyl-6-(4-fluoro-2-hydroxyphenyl)pyridazin-3-yl)-2-(methylamino)acetamide (Compound 71)

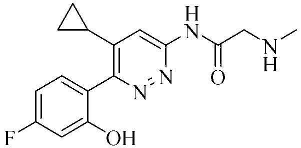

### Step 1 : Synthesis of 5-cyclopropyl-6-(2-(ethoxymethoxy)-4-fluorophenyl)pyridazin-3-amine

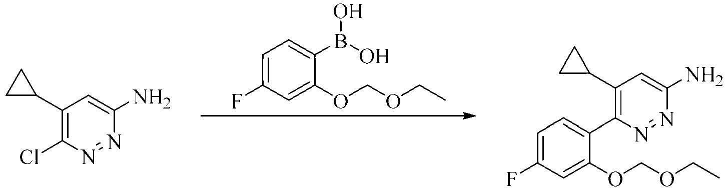

6-Chloro-5-cyclopropylpyridazin-3-amine (553.4 mg, 3.26 mmol, 1.0 eq.), (2-(ethoxymethoxy)-4-fluorophenyl)boronic acid (838.0 mg, 3.92 mmol, 1.2 eq.), sodium bicarbonate (411.1 mg, 4.89 mmol, 1.5 eq.) and Pd(dppf)Cl₂ (119.3 mg, 0.16 mmol, 0.05 eq.) were added to a mixed solution of 1,4-dioxane (8.0 mL) and water (4.0 mL). Under nitrogen protection, the resultant was reacted at 100°C for 12 hours. TLC monitoring showed the completion of the reaction. Ethyl acetate (50.0 mL) was added, and the resultant was washed with water (50.0 mL). The organic phase was dried over anhydrous sodium sulfate and filtered. The filtrate was concentrated under reduced pressure. The crude product was purified by silica gel column chromatography (dichloromethane:methanol=80:1-20:1), so as to afford the product (700.0 mg, yield: 70.7%).

### Step 2: Synthesis of tert-butyl (2-((5-cyclopropyl-6-(2-(ethoxymethoxy)-4-fluorophenyl)pyridazin-3-yl)amino)-2-oxoethyl)( methyl)carbamate

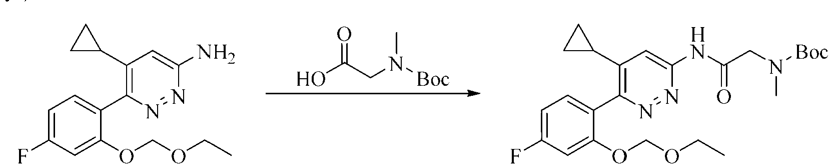

The compound was prepared in accordance with the synthetic method in Step 3 of **Example 6,** and the crude product was purified by silica gel column chromatography (dichloromethane:methanol=80:1-50:1), so as to afford the product (440.0 mg, yield: 80.7%).

### Step 3: Synthesis of N-(5-cyclopropyl-6-(4-fluoro-2-hydroxyphenyl)pyridazin-3-yl)-2-(methylamino)acetamide

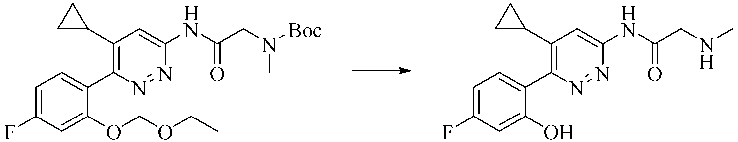

The compound was prepared in accordance with the method in Step 4 of **Example 3,** and the crude product was purified by preparative thin-layer chromatography, so as to afford the product (80.0 mg, yield: 60.1%).

¹HNMR (400 MHz, DMSO-*d₆*) δ(ppm): 7.80 (s, 1H), 7.31-7.27 (m, 1H), 6.80-6.73 (m, 2H), 3.38 (s, 2H), 3.33 (s, 1H), 2.35 (s, 3H), 1.71-1.65 (m, 1H), 1.02-1.00 (m, 2H), 0.72-0.70 (m, 2H).

Molecular formula: C₁₆H₁₇FN₄O₂ Precise molecular weight: 316.13 LC-MS (m/z)=317.07 [M+H]⁺.

### Example 47: Synthesis of (R)-2-amino-3-cyano-N-(5-cyclopropyl-6-(4-ethynyl-2-hydroxyphenyl)pyridazin-3-yl)prop anamide (Compound 73)

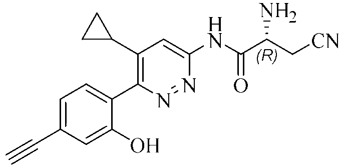

### Step 1 : Synthesis of (9H-fluoren-9-yl)methyl (R)-(3-cyano-1-((5-cyclopropyl-6-(2-(ethoxymethoxy)-4-ethynylphenyl)pyridazin-3-yl)amino)-1-oxopropan-2-yl)carbamate

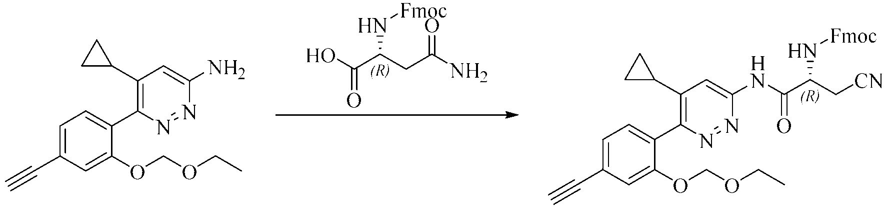

The compound was prepared in accordance with the synthetic method in Step 1 of **Example 14,** and the crude product was purified by silica gel column chromatography (dichloromethane:methanol=80:1-60:1), so as to afford the product (220.0 mg, yield: 36.2%).

### Step 2: Synthesis of (9H-fluoren-9-yl)methyl (R)-(3-cyano-1-((5-cyclopropyl-6-(4-ethynyl-2-hydroxyphenyl)pyridazin-3-yl)amino)-1-oxopr opan-2-yl)carbamate

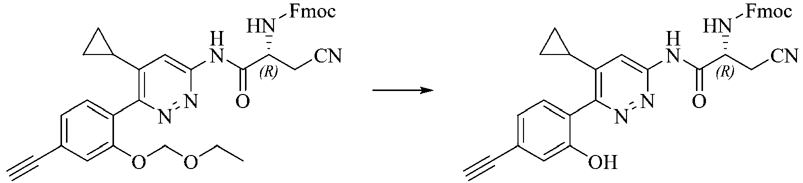

The compound was prepared in accordance with the method in Step 4 of **Example 3,** and the crude product was purified by silica gel column chromatography (dichloromethane:methanol=80:1-50:1) , so as to afford the product (167.0 mg, yield: 92.2%).

### Step 3: Synthesis of (R)-2-amino-3-cyano-N-(5-cyclopropyl-6-(4-ethynyl-2-hydroxyphenyl)pyridazin-3-yl)propana mide

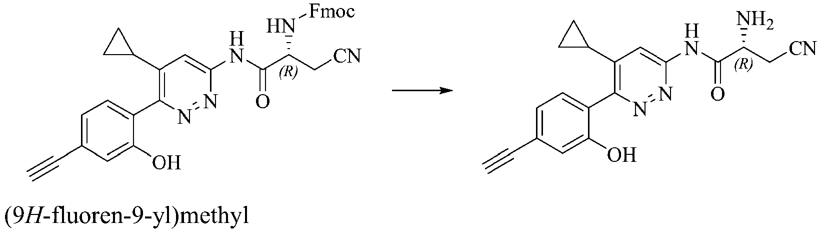

(*R*)-(3-cyano-1-((5-cyclopropyl-6-(4-ethynyl-2-hydroxyphenyl)pyridazin-3-yl)amino)-1-oxopr opan-2-yl)carbamate (160.0 mg, 0.28 mmol, 1.0 eq.) was added to acetonitrile (3.0 mL), diethylamine (3.0 mL) was added dropwise, and the resultant was reacted at room temperature for 14 hours. TLC monitoring showed the completion of the reaction. The resultant was concentrated under reduced pressure. Dichloromethane (50.0 mL) was added, and the resultant was washed with saturated aqueous ammonium chloride solution (50.0 mL). The organic phase was dried over anhydrous sodium sulfate and filtered. The filtrate was concentrated under reduced pressure. The crude product was purified by preparative thin-layer chromatography, so as to afford the product (7.0 mg, yield: 7.2%).

¹HNMR (400 MHz, DMSO-*d₆*) δ(ppm): 10.04 (s, 1H), 7.80 (s, 1H), 7.28-7.26 (d, *J*=8 Hz, 1H), 7.06-7.04 (m, 2H), 4.22 (s, 1H), 3.86-3.83 (m, 1H), 2.91-2.72 (m, 2H), 1.70-1.64 (m, 1H), 1.03-1.01 (m, 2H), 0.72-0.70 (m, 2H).

Molecular formula: C₁₉H₁₇N₅O₂ Precise molecular weight: 347.14 LC-MS (m/z)=348.08 [M+H]⁺.

### Example 48: Synthesis of (R)-N-(5-cyclopropyl-6-(4-ethynyl-2-hydroxyphenyl)pyridazin-3-yl)-2-hydroxypropanami de (Compound 74)

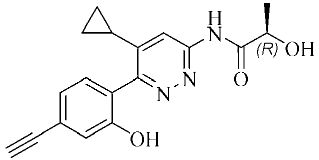

The compound was prepared in accordance with the synthetic method of **Example 33.**

¹HNMR (400 MHz, DMSO-*d₆*) δ(ppm): 10.23 (s, 1H), 10.04 (s, 1H), 7.80 (s, 1H), 7.28-7.26 (d, *J*=8 Hz, 1H), 7.06-7.04 (m, 2H), 5.87-5.86 (d, *J*=4 Hz, 1H), 4.32-4.25 (m, 1H), 4.22 (s, 1H), 1.69-1.64 (m, 1H), 1.34-1.32 (d, *J*=8 Hz, 3H), 1.04-0.99 (m, 2H), 0.75-0.71 (m, 2H).

Molecular formula: C₁₈H₁₇N₃O₃ Precise molecular weight: 323.13 LC-MS (m/z)=324.09 [M+H]⁺.

### Example 49: Synthesis of 2-cyano-N-(5-cyclopropyl-6-(4-ethynyl-2-hydroxyphenyl)pyridazin-3-yl)acetamide (Compound 75)

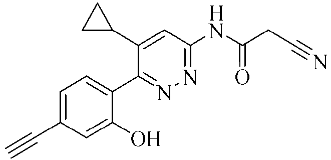

The compound was prepared in accordance with the synthetic method in **Example 14.**

¹HNMR (400 MHz, DMSO-*d₆*) δ(ppm): 11.42 (s, 1H), 10.08 (s, 1H), 7.70 (s, 1H), 7.28-7.26 (d, *J*=8 Hz, 1H), 7.06-7.04 (m, 2H), 4.24 (s, 1H), 4.06 (s, 2H), 1.69-1.64 (m, 1H), 1.03-1.01 (m, 2H), 0.73-0.72 (m, 2H).

Molecular formula: C₁₈H₁₄N₄O₂ Precise molecular weight: 318.11 LC-MS (m/z)=319.03 [M+H]⁺.

### Example 50: Synthesis of (R)-N-(5-cyclopropyl-6-(4-ethynyl-2-hydroxyphenyl)pyridazin-3-yl)-3-hydroxybutanamid e (Compound 76)

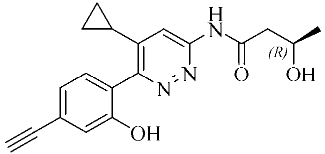

The compound was prepared in accordance with the synthetic method of **Example 33.**

¹HNMR (400 MHz, DMSO-*d₆*) δ(ppm): 10.96 (s, 1H), 10.05 (s, 1H), 7.81 (s, 1H), 7.27-7.25 (d, *J*=8 Hz, 1H), 7.06-7.03 (m, 2H), 4.80-4.79 (d, *J*=4 Hz, 1H), 4.23 (s, 1H), 4.13-4.07 (m, 1H), 2.59-2.53 (m, 1H), 2.48-2.44 (m, 1H), 1.69-1.62 (m, 1H), 1.14-1.12 (d, *J*=8 Hz, 3H), 1.01-0.98 (m, 2H), 0.71-0.68 (m, 2H).

Molecular formula: C₁₉H₁₉N₃O₃ Precise molecular weight: 337.14 LC-MS (m/z)=338.08 [M+H]⁺.

### Example 51: Synthesis of (S)-N-(5-cyclopropyl-6-(4-ethynyl-2-hydroxyphenyl)pyridazin-3-yl)-3-hydroxybutanamid e (Compound 77)

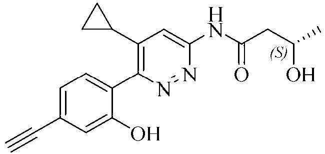

The compound was prepared in accordance with the synthetic method of **Example 33.**

¹HNMR (400 MHz, DMSO-*d₆*) δ(ppm): 10.96 (s, 1H), 10.04 (s, 1H), 7.81 (s, 1H), 7.27-7.25 (d, *J*=8 Hz, 1H), 7.06-7.03 (m, 2H), 4.80-4.79 (d, *J*=4 Hz, 1H), 4.23 (s, 1H), 4.13-4.09 (m, 1H), 2.59-2.53 (m, 1H), 2.48-2.44 (m, 1H), 1.69-1.62 (m, 1H), 1.14-1.13 (d, *J*=4 Hz, 3H), 1.01-0.98 (m, 2H), 0.70-0.69 (m, 2H).

Molecular formula: C₁₉H₁₉N₃O₃ Precise molecular weight: 337.14 LC-MS (m/z)=338.09 [M+H]⁺.

### Example 52: Synthesis of trifluoroacetate salt of (R)-3-amino-4-((5-cyclopropyl-6-(4-ethynyl-2-hydroxyphenyl)pyridazin-3-yl)amino)-4-ox obutyric acid (Compound 79-TF)

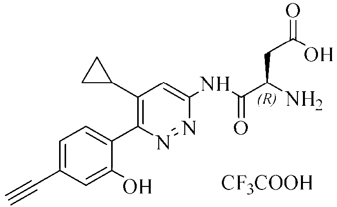

### Step 1 : Synthesis of tert-butyl (R)-3-((((9H-fluoren-9-yl)methoxy)carbonyl)amino)-4-((5-cyclopropyl-6-(2-(ethoxymethoxy)-4-ethynylphenyl)pyridazin-3-yl)amino)-4-oxobutyrate

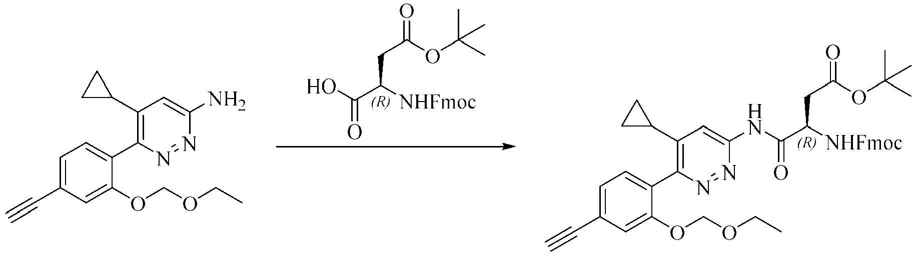

The compound was prepared in accordance with the method in Step 3 of **Example 6,** and the crude product was purified by silica gel column chromatography (dichloromethane:methanol=100:1-80:1), so as to afford the product (740.0 mg, yield: 84.6%).

### Step 2: Synthesis of tert-butyl (R)-3-amino-4-((5-cyclopropyl-6-(2-(ethoxymethoxy)-4-ethynylphenyl)pyridazin-3-yl)amino)-4-oxobutyrate

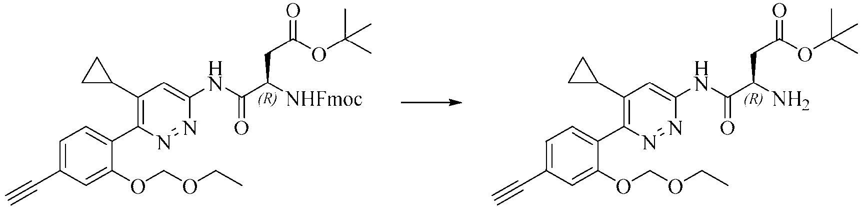

The compound was prepared in accordance with the synthetic method in Step 3 of **Example 47,** and the crude product was purified by silica gel column chromatography (dichloromethane:methanol=100:1-30:1), so as to afford the product (400.0 mg, yield: 79.0%).

### Step 3: Synthesis of trifluoroacetate salt of (R)-3-amino-4-((5-cyclopropyl-6-(4-ethynyl-2-hydroxyphenyl)pyridazin-3-yl)amino)-4-oxobut yric acid

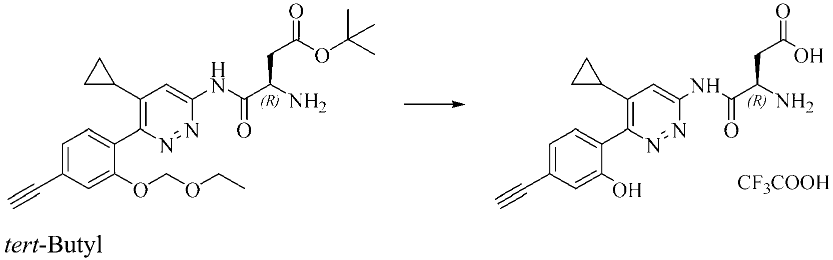

(*R*)-3-amino-4-((5-cyclopropyl-6-(2-(ethoxymethoxy)-4-ethynylphenyl)pyridazin-3-yl)amino)-4-oxobutyrate (380.0 mg, 0.79 mmol, 1.0 eq.) was added to dichloromethane (4.0 mL), trifluoroacetic acid (2.0 mL) was added dropwise, and the resultant was reacted at room temperature for 3 hours. TLC monitoring showed the completion of the reaction. Dichloromethane (50.0 mL) was added, and water (50.0 mL) was then added to conduct back-extraction. The aqueous phase was purified by reversed-phase preparative chromatography, so as to afford the product (110.0 mg, yield: 37.9%).

¹HNMR (400 MHz, DMSO-*d₆*) δ(ppm): 11.58 (s, 1H), 10.13 (s, 1H), 8.39 (s, 2H), 7.17 (s, 1H), 7.28-7.26 (d, *J*=8 Hz, 1H), 7.07-7.06 (m, 2H), 4.41 (s, 1H), 4.23 (s, 1H), 3.03-2.89 (m, 2H), 1.71-1.68 (m, 1H), 1.05-1.03 (m, 2H), 0.70-0.68 (m, 2H).

Molecular formula: C₁₉H₁₈N₄O₄ Precise molecular weight (free base): 366.13 LC-MS (m/z)=367.07 [M+H]⁺.

### Example 53: Synthesis of N-(4-(4-ethynyl-2-hydroxyphenyl)-5,6,7,8-tetrahydrophthalazin-1-yl)-2-(methylamino)ace tamide (Compound 24)

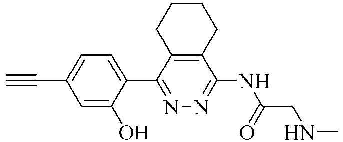

### Step 1: Synthesis of 4-chloro-5,6,7,8-tetrahydrophthalazin-1-amine

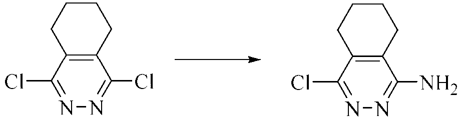

The compound was prepared in accordance with the method in Step 2 of **Intermediate preparation example 1,** and the crude product was purified by silica gel column chromatography (DCM:MeOH=100:1~20:1), so as to afford the product (4.10 g, yield: 90.7%).

### Step 2: Synthesis of 4-(4-amino-5,6,7,8-tetrahydrophthalazin-1-yl)-3-(ethoxymethoxy)benzaldehyde

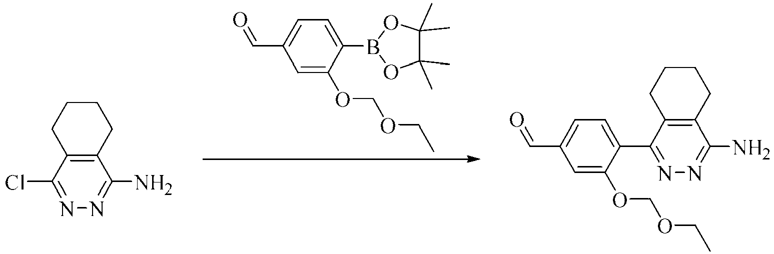

The compound was prepared in accordance with the method in Step 1 of **Example 6,** and the crude product was purified by silica gel column chromatography (DCM:MeOH=100:1~20:1), so as to afford the product (1.20 g, yield: 67.3%).

### Step 3: Synthesis of 4-(2-(ethoxymethoxy)-4-ethynylphenyl)-5,6,7,8-tetrahydrophthalazin-1-amine

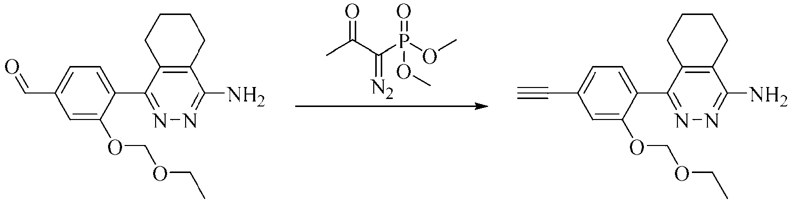

The compound was prepared in accordance with the method in Step 2 of **Example 6,** and the crude product was purified by silica gel column chromatography (DCM:MeOH=50:1~20:1), so as to afford the product (1.10 g, yield: 92.8%).

### Step 4: Synthesis of tert-butyl (2-(4-(2-(ethoxymethoxy)-4-ethynylphenyl)-5,6,7,8-tetrahydrophthalazin-1-yl)amino)-2-oxoet hyl)(methyl)carbamate

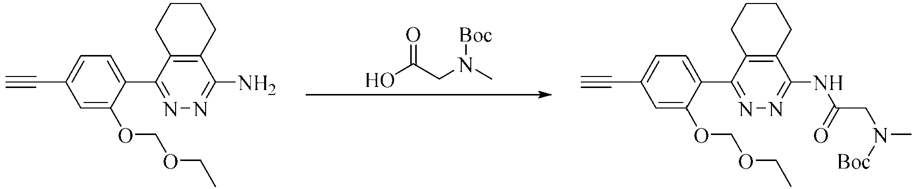

The compound was prepared in accordance with the synthetic method in Step 3 of **Example 6,** and the crude product was purified by silica gel column chromatography (DCM:MeOH= 100:1~50:1), so as to afford the product (456 mg, yield: 74.5%).

### Step 5: Synthesis of N-(4-(4-ethynyl-2-hydroxyphenyl)-5,6,7,8-tetrahydrophthalazin-1-yl)-2-(methylamino)acetami de

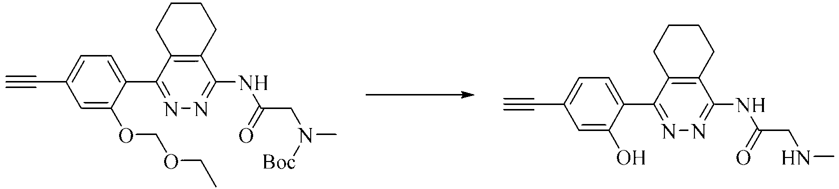

The compound was prepared in accordance with the synthetic method in Step 4 in **Example 6,** and the crude product was purified by silica gel column chromatography (DCM:MeOH= 50:1~10:1), so as to afford the product (110 mg, yield: 35.9%).

¹HNMR (400 MHz, DMSO-*d₆*) δ(ppm): 7.19 (d, *J=* 7.7 Hz, 1H), 7.06-7.03 (m, 2H), 4.23 (s, 1H), 3.48 (s, 3H), 2.61-2.58 (m, 2H), 2.49-2.46 (m, 2H), 2.41 (s, 3H), 1.72-1.37 (m, 4H).

Molecular formula: C₁₉H₂₀N₄O₂ Precise molecular weight: 336.16 LC-MS (m/z)=337.16[M+H]⁺.

### Example 54: Synthesis of N-(4-(2-hydroxy-4-(trifluoromethyl)phenyl)phthalazin-1-yl)-2-(methylamino)acetamide (Compound 25)

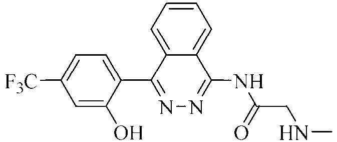

### Step 1: Synthesis of 4-chlorophthalazin-1-amine

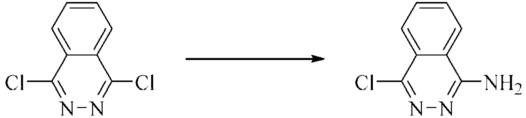

1,4-Dichlorophthalazine (8.00 g, 40.2 mmol, 1.0 eq) was added to an ammonia solution in isopropanol (50 mL), and the resultant was heated to 100°C and reacted for 20 hours. The resultant was cooled to room temperature and subjected to suction filtration. The filter cake was purified by silica gel column chromatography (DCM:MeOH=100:1~20:1), so as to afford the product (4.90 g, yield: 67.9%).

### Step 2: Synthesis of 4-(2-(ethoxymethoxy)-4-(trifluoromethyl)phenyl)phthalazin-1-amine

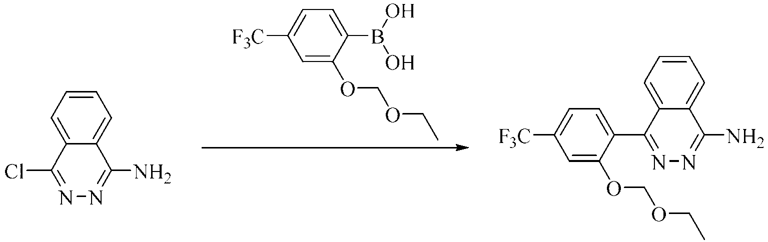

The compound was prepared in accordance with the method in Step 1 of **Example 6,** and the crude product was purified by silica gel column chromatography (DCM:MeOH=100:1~50:1), so as to afford the product (660 mg, yield: 65.3%).

### Step 3: Synthesis of tert-butyl (2-(4-(2-(ethoxymethoxy)-4-(trifluoromethyl)phenyl)phthalazin-1-yl)amino)-2-oxoethyl)(meth yl)carbamate

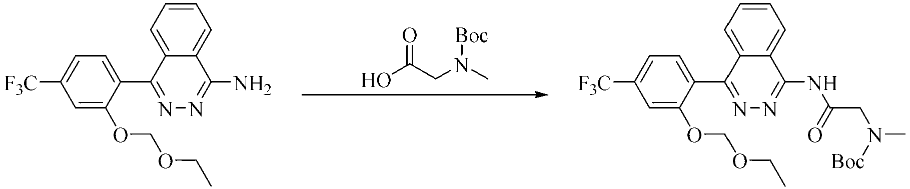

The compound was prepared in accordance with the method in Step 3 of **Example 6,** and the crude product was purified by silica gel column chromatography (DCM:MeOH=100:1~50:1), so as to afford the product (462 mg, yield: 78.5%).

### Step 4: Synthesis of N-(4-(2-hydroxy-4-(trifluoromethyl)phenyl)phthalazin-1-yl)-2-(methylamino)acetamide

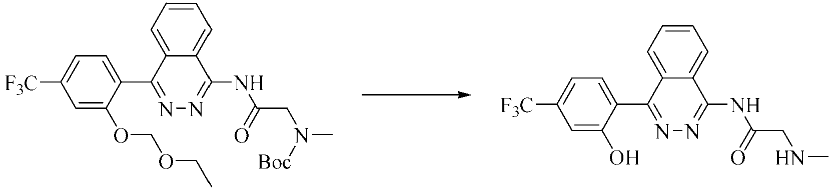

The compound was prepared in accordance with the method in Step 4 of **Example** 6, and the crude product was purified by silica gel column chromatography (DCM:MeOH= 50:1~10:1), so as to afford the product (85.0 mg, yield: 26.2%).

¹HNMR (400 MHz, DMSO-*d₆*) δ(ppm): 8.42 (s, 1H), 8.07-7.99 (m, 2H), 7.67-7.60 (m, 2H), 7.45 (s, 1H), 7.37 (d, *J=* 7.8 Hz, 1H), 4.23 (s, 2H), 2.67 (s, 3H).

Molecular formula: C₁₈H₁₅F₃N₄O₂ Precise molecular weight: 376.11 LC-MS (m/z)=377.12[M+H]⁺.

### Example 55: Synthesis of N-(4-(2-hydroxy-4-(trifluoromethyl)phenyl)-5,6,7,8-tetrahydrophthalazin-1-yl)-2-(methyl amino)acetamide (Compound 26)

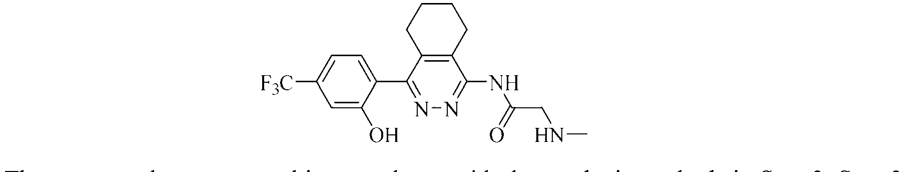

The compound was prepared in accordance with the synthetic methods in Step 2, Step 3 and Step 4 of **Example 54** by using intermediates 4-chloro-5,6,7,8-tetrahydrophthalazin-1-amine and (2-(ethoxymethoxy)-4-(trifluoromethyl)phenyl)boronic acid as well as N-(tert-butoxycarbonyl)sarcosine as raw materials.

¹HNMR (400 MHz, DMSO-*d₆*) δ(ppm): 7.42 (d, *J =* 8.1Hz, 1H), 7.27 (d, *J =* 6.7 Hz, 2H), 3.40 (s, 2H), 2.61-2.59 (m, 2H), 2.48 (s, 2H), 2.39 (s, 3H), 1.71-1.69 (m, 4H).

Molecular formula: C₁₈H₁₉F₃N₄O₂ Precise molecular weight: 380.15 LC-MS (m/z)=381.17[M+H]⁺.

### Example 56: Synthesis of N-(4-(4-ethynyl-2-hydroxyphenyl)phthalazin-1-yl)-2-(methylamino)acetamide (Compound 23)

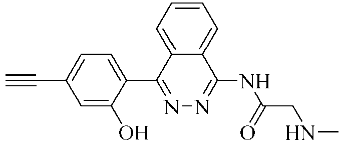

The compound was prepared in accordance with the synthetic method of **Example 53** by using 4-chlorophthalazin-1-amine, 3-(ethoxymethoxy)-4-(4,4,5,5-tetramethyl-1,3,2-dioxaborolan-2-yl)benzaldehyde and *N*-(*tert*-butoxycarbonyl)sarcosine as raw materials.

¹HNMR (400 MHz, DMSO-*d₆*) δ(ppm): 8.38 (s, 1H), 8.06-7.98 (m, 2H), 7.68-7.66 (m, 1H), 7.38 (d, *J =* 7.8 Hz, 1H), 7.18 (d, *J =* 1.3Hz, 1H), 7.14-7.12 (m, 1H), 4.31 (s, 1H), 4.16 (s, 2H), 2.66 (s, 3H).

Molecular formula: C₁₉H₁₆N₄O₂ Precise molecular weight: 332.13 LC-MS (m/z)=333.13[M+H]⁺.

### Example 57: Synthesis of N-(5-(tert-butyl)-6-(4-ethynyl-2-hydroxyphenyl)pyridazin-3-yl)-2-(methylamino)acetamid e (Compound 68)

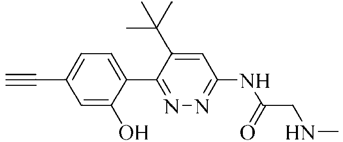

The compound was prepared in accordance with the synthetic method of **Example 53** by using 5-(*tert*-butyl)-6-chloropyridazin-3-amine, 3-(ethoxymethoxy)-4-(4,4,5,5-tetramethyl-1,3,2-dioxaborolan-2-yl)benzaldehyde and *N*-(*tert*-butoxycarbonyl)sarcosine as raw materials.

¹HNMR (400 MHz, DMSO-*d₆*) δ(ppm): 8.52 (s, 1H), 7.16 (d, *J=* 7.6 Hz, 1H), 7.02-7.00 (m, 2H), 4.21 (s, 1H), 3.36 (s, 2H), 3.17 (s, 1H), 2.34 (s, 3H), 1.17 (s, 9H).

Molecular formula: C₁₉H₂₂N₄O₂ Precise molecular weight: 338.17 LC-MS (m/z)=339.13[M+H]⁺.

### Example 58: Synthesis of N-(5-(tert-butyl)-6-(2-hydroxy-4-(trifluoromethyl)phenyl)pyridazin-3-yl)-2-(methylamino) acetamide (Compound 69)

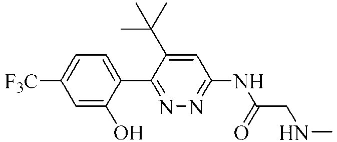

### Step 1: Synthesis of 2-(6-amino-4-(tert-butyl)pyridazin-3-yl)-5-(trifluoromethyl)phenol

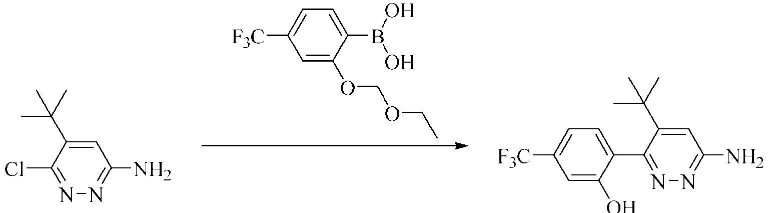

The compound was prepared in accordance with the method in Step 1 of **Example 6,** and the crude product was purified by silica gel column chromatography (DCM:MeOH=50:1~15:1), so as to afford the product (504 mg, yield: 60.1%).

### Step 2: Synthesis of 5-(tert-butyl)-6-(2-((tert-butyldimethylsilyl)oxy)-4-(trifluoromethyl)phenyl)pyridazin-3-amine

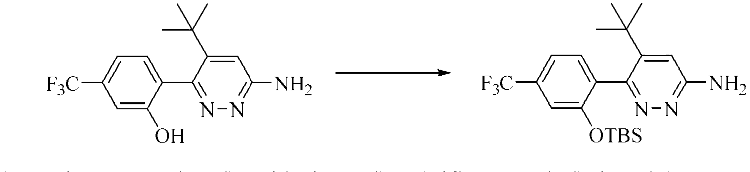

2-(6-Amino-4-(*tert*-butyl)pyridazin-3-yl)-5-(trifluoromethyl)phenol (450 mg, 1.45 mmol, 1.1 eq) was dissolved in DMF (10 mL), imidazole (148 mg, 2.17 mmol, 1.5 eq) and TBSCl (262 mg, 1.74 mmol, 1.2 eq) were then added, and the resultant was reacted at room temperature for 1 hour. Water (60 mL) was added, the resultant was extracted with EA (30 mL), and the organic phase was washed with water (20 mL×2). The organic phase was dried over anhydrous sodium sulfate and filtered. The filtrate was concentrated under reduced pressure and purified by silica gel column chromatography (DCM:MeOH=50:1), so as to afford the product (480 mg, yield: 78.0%).

### Step 3: Synthesis of tert-butyl (2-((5-(tert-butyl)-6-(2-hydroxy-4-(trifluoromethyl)phenyl)pyridazin-3-yl)amino)-2-oxoethyl)( methyl)carbamate

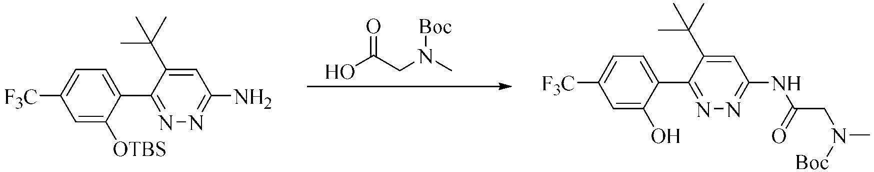

The compound was prepared in accordance with the method in Step 3 of **Example 6,** and the crude product was purified by silica gel column chromatography (DCM:MeOH=50:1~20:1), so as to afford the product (342 mg, yield: 62.8%).

### Step 4: Synthesis of N-(5-(tert-butyl)-6-(2-hydroxy-4-(trifluoromethyl)phenyl)pyridazin-3-yl)-2-(methylamino)acet amide

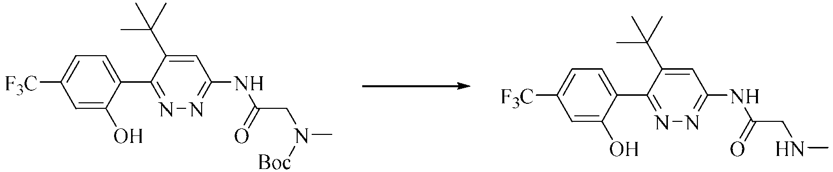

The compound was prepared in accordance with the method in Step 4 in **Example 6,** and the crude product was purified by silica gel column chromatography (DCM:MeOH=20:1~8:1), so as to afford the product (103 mg, yield: 39.1%).

¹HNMR (400 MHz, DMSO-*d*₆) δ(ppm): 11.75 (s, 1H), 10.50 (s, 1H), 9.18 (s, 1H), 8.46 (s, 1H), 7.42 (d, *J =* 7.8 Hz, 1H), 7.29-7.24 (m, 2H), 4.07 (s, 2H), 2.65 (s, 3H), 1.18 (s, 9H).

Molecular formula: C₁₈H₂₁F₃N₄O₂ Precise molecular weight: 382.16 LC-MS (m/z)=383.12[M+H]⁺.

### Example 59: Synthesis of (R)-2-amino-N-(5-(tert-butyl)-6-(2-hydroxy-4-(trifluoromethyl)phenyl)pyridazin-3-yl)pro panamide (Compound 70)

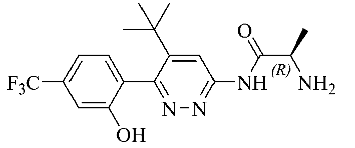

### Step 1: Synthesis of 2-(6-amino-4-(tert-butyl)pyridazin-3-yl)-6-(trifluoromethyl)phenol

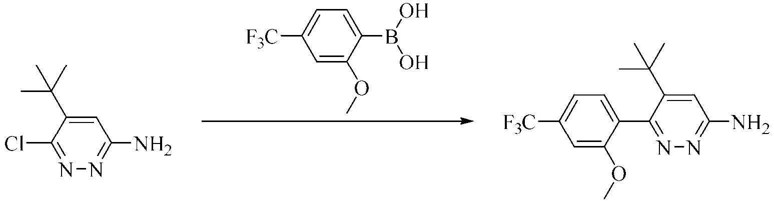

The compound was prepared in accordance with the method in Step 1 of **Example 6,** and the crude product was purified by silica gel column chromatography (DCM:MeOH=100:1~20:1), so as to afford the product (135 mg, yield: 19.3%).

### Step 2: Synthesis of tert-butyl (R)-(1-((5-(tert-butyl)-6-(2-methoxy-4-(trifluoromethyl)phenyl)pyridazin-3-yl)amino)-1-oxopr opan-2-yl)carbamate

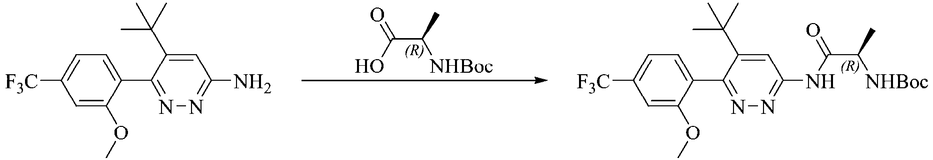

The compound was prepared in accordance with the method in Step 3 of **Example 6,** and the crude product was purified by silica gel column chromatography (DCM:MeOH=50:1), so as to afford the product (36.0 mg, yield: 67.4%).

### Step 3: Synthesis of (R)-2-amino-N-(5-(tert-butyl)-6-(2-hydroxy-4-(trifluoromethyl)phenyl)pyridazin-3-yl)propana mide

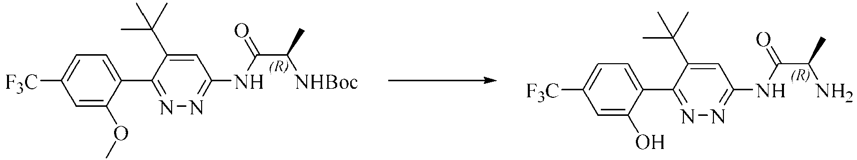

The compound was prepared in accordance with the method in Step 3 of **Example 20,** and the crude product was purified by preparative thin-layer chromatography (DCM:MeOH =10:1), so as to afford the product (13.0 mg, yield: 46.9%).

¹HNMR (400 MHz, DMSO-*d₆*) δ(ppm): 10.56 (s, 1H), 8.49 (s, 1H), 7.41 (d, *J =* 7.8 Hz, 1H), 7.32 (s, 1H), 7.25 (d, *J=* 8.0 Hz, 1H), 4.21-4.17 (m, 1H), 1.52 (d, *J=* 7.0 Hz, 3H), 1.18 (s, 9H).

Molecular formula: C₁₈H₂₁F₃N₄O₂ Precise molecular weight: 382.16 LC-MS (m/z)=383.09[M+H]⁺.

### Example 60: Synthesis of compound N-(6-(4-ethynyl-2-hydroxyphenyl)-5-methylpyridazin-3-yl)-2-(methylamino)acetamide (Compound 1)

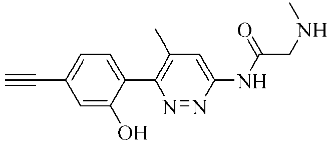

The compound was prepared in accordance with the synthetic method of **Example 7** by using intermediate 6-(2-(ethoxymethoxy)-4-ethynylphenyl)-5-methylpyridazin-3-amine as raw material.

¹H-NMR (400 MHz, DMSO-*d₆*) δ(ppm): 10.19-10.18 (d, 1H), 8.20 (s, 1H), 7.25-7.23 (d, 1H), 7.09-7.04 (m, 2H), 4.24 (s, 1H), 3.78 (s, 2H), 3.57-3.54 (m, 1H), 3.08-3.06 (d, 1H), 2.53(s, 3H), 2.20(s, 3H).

Molecular formula: C₁₆H₁₆N₄O₂ Precise molecular weight: 296.13 LC-MS (m/z): 297.17 [M+H]⁺.

### Example 61: Synthesis of compound N-(6-(4-ethynyl-2-hydroxyphenyl)-5-methylpyridazin-3-yl)-2-(isopropylamino)acetamide (Compound 3)

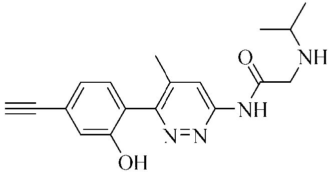

The compound was prepared in accordance with the synthetic method in Step 3 of **Example 7.**

¹H-NMR (400 MHz, DMSO-*d₆*) δ(ppm): 11.51 (s, 1H), 10.24 (s, 1H), 8.90 (s, 1H), 8.17 (s, 1H), 7.26-7.24 (d, 1H), 7.11-7.04 (m, 2H), 4.24(s, 1H), 4.03 (s, 2H), 3.38-3.36(m, 1H), 2.21 (s, 3H), 1.27-1.25(d, 6H).

Molecular formula: C₁₈H₂₀N₄O₂ Precise molecular weight: 324.16 LC-MS (m/z): 325.19 [M+H]⁺.

### Example 62: Synthesis of compound N-(6-(4-ethynyl-2-hydroxyphenyl)-5-methylpyridazin-3-yl)-2-(cyclopropylamino)acetami de (Compound 4)

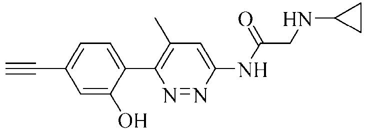

The compound was prepared in accordance with the synthetic method in Step 3 of **Example 7.**

¹H-NMR (400 MHz, DMSO-*d₆*) δ(ppm): 10.72 (s, 1H), 10.12 (s, 1H), 8.24 (s, 1H), 7.24-7.23 (d, 1H), 7.06-7.04 (d, 2H), 4.23 (s, 1H), 3.47 (s, 2H), 3.18-3.17 (d, 1H), 2.23-2.21 (m, 1H), 2.19 (s, 3H), 0.42-0.38 (m, 2H), 0.33-0.29 (m, 2H).

Molecular formula: C₁₈H₁₈N₄O₂ Precise molecular weight: 324.16 LC-MS (m/z): 325.19 [M+H]⁺.

### Example 63: Synthesis of compound N-(6-(4-ethynyl-2-hydroxyphenyl)-5-methylpyridazin-3-yl)-2-(ethylamino)acetamide (Compound 2)

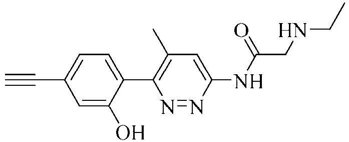

The compound was prepared in accordance with the synthetic method in Step 3 of **Example 7.**

¹H-NMR (400 MHz, DMSO-*d₆*) δ(ppm): 11.56 (s, 1H), 10.19 (s, 1H), 9.02-8.98 (s, 1H), 8.16 (s, 1H), 7.26-7.24 (d, 1H), 7.08-7.05 (d, 2H), 4.24 (s, 1H), 4.07 (s, 2H), 3.08-3.02 (m, 2H), 2.21 (s, 3H), 1.25-1.22 (t, 3H).

Molecular formula: C₁₇H₁₈N₄O₂ Precise molecular weight: 310.14 LC-MS (m/z): 311.19 [M+H]⁺.

### Example 64: Synthesis of compound N-(6-(4-ethynyl-2-hydroxyphenyl)-5-methylpyridazin-3-yl)-2-(cyclobutylamino)acetamide (Compound 31)

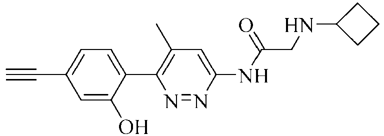

The compound was prepared in accordance with the synthetic method in Step 3 of **Example 7.**

¹H-NMR (400 MHz, DMSO-*d₆*) δ(ppm): 10.19 (s, 1H), 8.23 (s, 1H), 7.24-7.22 (d, 1H), 7.08-7.03 (m, 2H), 4.23 (s, 1H), 3.36-3.33 (m, 4H), 3.28-3.22 (m, 1H), 2.19 (s, 3H), 2.14-2.07 (m, 2H), 1.81-1.72 (m, 2H), 1.68-1.52 (m, 2H).

Molecular formula: C₁₉H₂₀N₄O₂ Precise molecular weight: 336.16 LC-MS (m/z): 337.19 [M+H]⁺.

### Example 65: Synthesis of compound N-(6-(4-ethynyl-2-hydroxyphenyl)-5-methylpyridazin-3-yl)-2-(isobutylamino)acetamide (Compound 32)

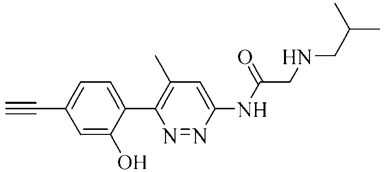

The compound was prepared in accordance with the synthetic method in Step 3 of **Example 7.**

¹H-NMR (400 MHz, DMSO-*d₆*) δ(ppm): 10.11 (s, 1H), 8.26 (s, 1H), 7.25-7.23 (d, 1H), 7.06-7.04 (m, 2H), 4.23 (s, 1H), 4.12-4.08 (m, 1H), 3.39 (s, 2H), 3.18-3.17 (d, 1H), 2.39-2.38 (d, 2H), 2.19 (s, 3H), 1.74-1.67 (m, 1H), 0.93-0.91 (d, 6H).

Molecular formula: C₁₉H₂₂N₄O₂ Precise molecular weight: 338.17 LC-MS (m/z): 339.20 [M+H]⁺.

### Example 66: Synthesis of compound N-(6-(4-ethynyl-2-hydroxyphenyl)-5-methylpyridazin-3-yl)-2-((cyclopropylmethyl)amino) acetamide (Compound 34)

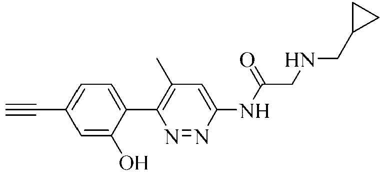

The compound was prepared in accordance with the synthetic method in Step 3 of **Example 7.**

¹H-NMR (400 MHz, DMSO-*d₆*) δ(ppm): 10.10 (s, 1H), 8.25 (s, 1H), 7.25-7.23 (d, 1H), 7.06-7.04 (m, 2H), 4.23 (s, 1H), 3.43 (s, 2H), 2.47-2.45 (d, 2H), 2.19 (s, 3H), 0.96-0.86 (m, 1H), 0.46-0.41 (m, 2H), 0.16-0.12 (m, 2H).

Molecular formula: C₁₉H₂₀N₄O₂ Precise molecular weight: 336.16 LC-MS (m/z): 337.19 [M+H]⁺.

### Example 67: Synthesis of compound N-(6-(4-ethynyl-2-hydroxyphenyl)-5-methylpyridazin-3-yl)-2-((2-hydroxy-2-methylpropyl )amino)acetamide (Compound 35)

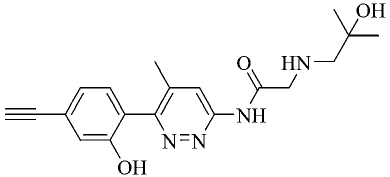

The compound was prepared in accordance with the synthetic method in Step 3 of **Example 7.**

¹H-NMR (400 MHz, DMSO-*d₆*) δ(ppm): 10.11 (s, 1H), 8.26 (s, 1H), 7.25-7.23 (d, 1H), 7.06-7.04 (m, 2H), 4.39 (s, 1H), 4.23 (s, 1H), 3.44 (s, 2H), 2.47 (s, 2H), 2.19 (s, 3H), 1.14 (s, 6H).

Molecular formula: C₁₉H₂₂N₄O₃ Precise molecular weight: 354.17 LC-MS (m/z): 355.24[M+H]⁺.

### Example 68: Synthesis of compound N-(6-(4-ethynyl-2-hydroxyphenyl)-5-(trifluoromethyl)pyridazin-3-yl)-2-(methylamino)ace tamide (Compound 41)

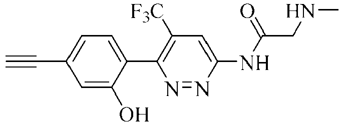

### Step 1: Synthesis of intermediate 6-chloro-5-(trifluoromethyl)pyridazin-3-amine

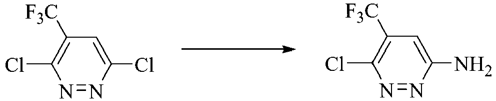

3,6-Dichloro-4-trifluoromethylpyridazine (3.0 g, 13.83 mmol, 1.0 eq) was dissolved in 3.5 mol/L solution of ammonia in isopropanol (30 mL), and the resultant was reacted in a sealed tube at 100°C for 1 h. TLC monitoring showed the completion of the reaction. The reaction solution was concentrated. The crude product was purified by silica gel column chromatography (dichloromethane:methanol=100:1), so as to afford the product (570 mg, yield: 21.1%).

### Step 2: Synthesis of intermediate 4-(6-amino-4-(trifluoromethyl)pyridazin-3-yl)-3-(ethoxymethoxy)benzaldehyde

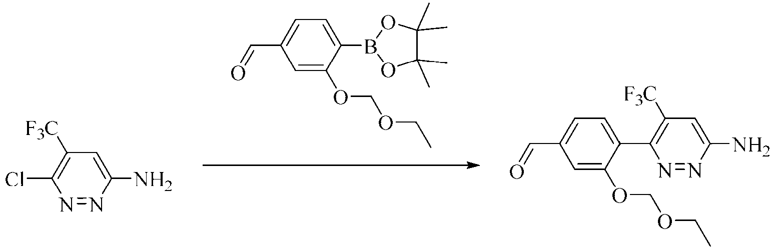

The compound was prepared in accordance with the method in Step 1 of **Example 6,** and the crude product was purified by silica gel column chromatography (dichloromethane:methanol=100:1~50:1), so as to afford the product (790 mg, yield: 80.4%).

### Step 3: Synthesis of intermediate 6-(2-(ethoxymethoxy)-4-ethynylphenyl)-5-(trifluoromethyl)pyridazin-3-amine

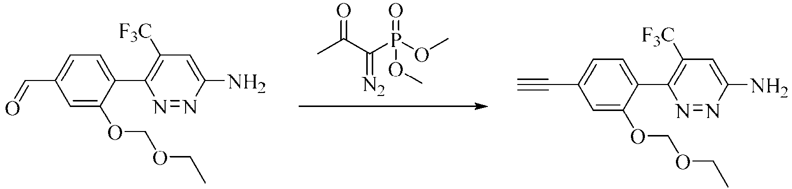

The compound was prepared in accordance with the method in Step 2 of **Example 6,** and the crude product was purified by silica gel column chromatography (dichloromethane:methanol=100:1~20:1), so as to afford the product (430 mg, yield: 55.1%).

### Step 4: Synthesis of intermediate 2-bromo-N-(6-(2-(ethoxymethoxy)-4-ethynylphenyl)-5-(trifluoromethyl)pyridazin-3-yl)acetam ide

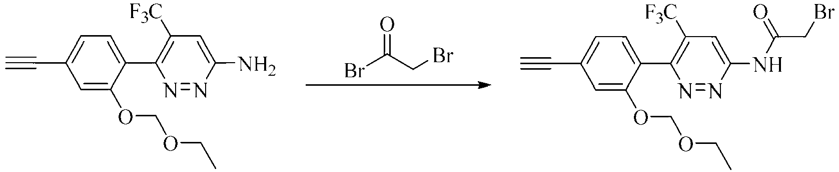

The compound was prepared in accordance with the method in Step 1 of **Example 7,** and the crude product was purified by preparative thin-layer chromatography (petroleum ether:ethyl acetate=2:1), so as to afford the product (100 mg, yield: 36.8%).

### Step 5: Synthesis of intermediate 2-bromo-N-(6-(4-ethynyl-2-hydroxyphenyl)-5-(trifluoromethyl)pyridazin-3-yl)acetamide

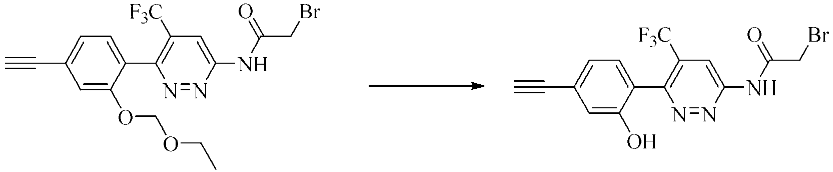

The compound was prepared in accordance with the method in Step 2 of **Example 7,** and the crude product was purified by preparative thin-layer chromatography (dichloromethane:methanol=10:1), so as to afford the product (30 mg, yield: 34.5%).

### Step 6: Synthesis of compound N-(6-(4-ethynyl-2-hydroxyphenyl)-5-(trifluoromethyl)pyridazin-3-yl)-2-(methylamino)acetami de

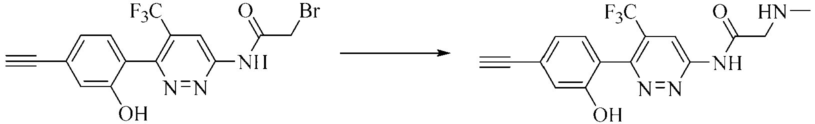

The compound was prepared in accordance with the method in Step 3 of **Example 7,** and the crude product was purified by preparative thin-layer chromatography (dichloromethane:methanol=7:1), so as to afford the product (6 mg, yield: 23.1%).

¹H-NMR (400 MHz, DMSO-*d₆*) δ(ppm): 8.70 (s, 1H), 7.26-7.24 (d, 1H), 7.05-7.02 (m, 2H), 4.24 (s, 1H), 3.76(s, 2H), 2.37 (s, 3H).

Molecular formula: C₁₆H₁₃F₃N₄O₂ Precise molecular weight: 350.10 LC-MS (m/z): 351.11 [M+H]⁺.

### Example 69: Synthesis of N-(6-(5-cyano-3-hydroxypyridin-2-yl)-5-cyclopropylpyridazin-3-yl)-2-(methylamino)acetamid e (Compound 78)

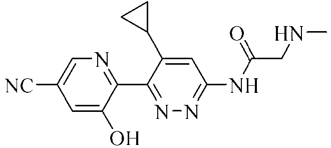

### Step 1: Synthesis of 5-hydroxy-6-iodonicotinonitrile

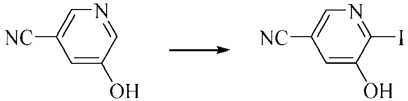

5-Hydroxynicotinonitrile (10.0 g, 83.26 mmol, 1.0 eq.) was added to water (100.0 mL), potassium carbonate (23.0 g, 166.52 mmol, 2.0 eq.) was added, the resultant was stirred for 2 min, and then iodine (19.0 g, 74.93 mmol, 0.9 eq.) was added. After further stirring for 18h, sodium sulfite was added to quench the reaction. The pH value was adjusted to approximately 3 with citric acid. Ethyl acetate (200 mL) was added, and the resultant was subjected to liquid-liquid phase separation. The organic phase was dried over anhydrous sodium sulfate and filtered. The filtrate was concentrated under reduced pressure. Methyl *tert-butyl* ether (50 mL) was added, and the resultant was slurried and subjected to suction filtration. The filter cake was dried at 50°C, so as to afford the product (8.65 g, yield: 42.2%).

### Step 2: Synthesis of 5-(ethoxymethoxy)-6-iodonicotinonitrile

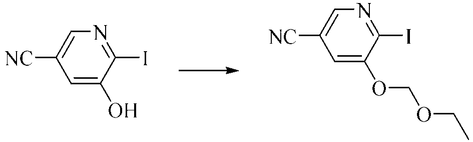

5-Hydroxy-6-iodonicotinonitrile (8.6 g, 34.96 mmol, 1.0 eq.) was added to tetrahydrofuran (100.0 mL). The temperature was held at approximately 10°C. Sodium hydride (2.1 g, 52.44 mmol, 1.5 eq.) was added, the resultant was stirred for 0.5h, and chloromethyl ethyl ether (5.0g, 52.44 mmol, 1.5 eq.) was added. After further stirring for 1h, TLC monitoring showed the completion of the reaction. Water (100.0 mL) and ethyl acetate (100.0 mL) were added, and the resultant was allowed to stand and separated into two phases. The organic phase was dried over anhydrous sodium sulfate and filtered. The filtrate was concentrated under reduced pressure. The crude product was purified by silica gel column chromatography (petroleum ether:ethyl acetate=10:1), so as to afford the product (5.2 g, yield: 49.0%).

### Step 3: Synthesis of 5-(ethoxymethoxy)-6-(tributylstannyl)nicotinonitrile

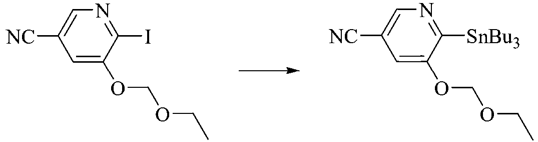

5-(Ethoxymethoxy)-6-iodonicotinonitrile (2.0 g, 6.58 mmol, 1.0 eq.) was added to tetrahydrofuran (20.0 mL). Under nitrogen protection, the temperature was held at -60°C, a solution of isopropylmagnesium chloride in tetrahydrofuran (3.9 mL, 7.90 mmol, 1.2 eq.) was added dropwise, and the resultant was stirred for 0.5h. Tributyltin chloride (2.6 g, 7.90 mmol, 1.2 eq.) was added, and the resultant was stirred for 3h. TLC monitoring showed the completion of the reaction. An aqueous solution of potassium fluoride (50.0 mL) was added, and the resultant was stirred for 0.5h. Ethyl acetate (50.0 mL) was added for extraction. The organic phase was dried over anhydrous sodium sulfate and filtered. The filtrate was concentrated under reduced pressure. The crude product was purified by silica gel column chromatography (petroleum ether:ethyl acetate=20:1), so as to afford the product (0.6 g, yield: 19.4%).

### Step 4: Synthesis of tert-butyl (2-((6-(5-cyano-3-hydroxypyridin-2-yl)-5-cyclopropylpyridazin-3-yl)amino)-2-oxoethyl)(meth yl)carbamate

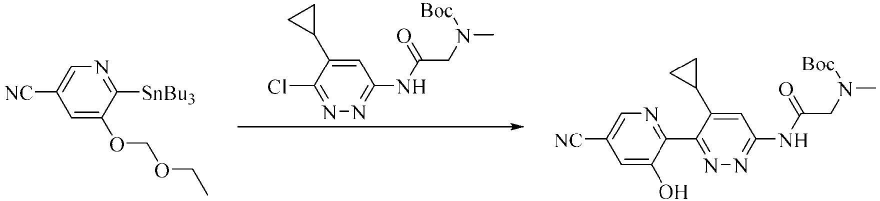

5-(Ethoxymethoxy)-6-(tributylstannyl)nicotinonitrile (0.6 g, 1.28 mmol, 1.0 eq.) was added to *N*,*N*-dimethylformamide (5.0 mL), and *tert-*butyl (2-((6-chloro-5-cyclopropylpyridazin-3-yl)amino)-2-oxoethyl)(methyl)carbamate (0.3 g, 0.77 mmol, 0.6 eq.), tetrakis(triphenylphosphine)palladium (0.2 g, 0.13 mmol, 0.1 eq.), cesium fluoride (0.4 g, 2.56 mmol, 2.0 eq.) and cuprous iodide (24.7 mg, 0.13 mmol, 0.1 eq.) were added. Under nitrogen protection, the resultant was reacted at 150°C for 2h under microwave irradiation. TLC monitoring showed the completion of the reaction. Water (20.0 mL) was added, and the resultant was extracted with ethyl acetate (20.0 mL). The organic phase was dried over anhydrous sodium sulfate and filtered. The filtrate was concentrated under reduced pressure. The crude product was purified by silica gel column chromatography (petroleum ether:ethyl acetate=2:1), so as to afford the product (90.0 mg, yield: 16.6%).

### Step 5: Synthesis of N-(6-(5-cyano-3-hydroxypyridin-2-yl)-5-cyclopropylpyridazin-3-yl)-2-(methylamino)acetamid e

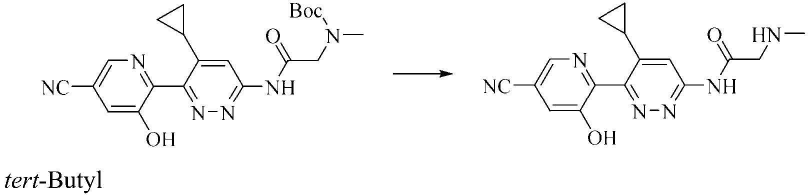

(2-((6-(5-cyano-3-hydroxypyridin-2-yl)-5-cyclopropylpyridazin-3-yl)amino)-2-oxoethyl)(meth yl)carbamate (90.0 mg, 0.21 mmol, 1.0 eq.) was added to a mixed solvent of dichloromethane and methanol (1.0 mL/1.0 mL), a solution of hydrogen chloride in ethyl acetate (2.0 mL) was added, and the resultant was stirred for 1h. TLC monitoring showed the completion of the reaction. The pH value was adjusted to approximately 8 with saturated aqueous sodium carbonate solution, and the resultant was concentrated under reduced pressure. The residue was added with a mixed solvent of dichloromethane and methanol (20.0 mL/2 mL), followed by filtration. The filtrate was concentrated under reduced pressure. The crude product was purified by preparative thin-layer chromatography (dichloromethane:methanol=7:1), so as to afford the product (10.0 mg, yield: 14.7%).

¹HNMR (400 MHz, DMSO-*d₆*) δ(ppm): 8.60-8.59 (d, 1H), 7.91 (s, 1H), 7.75-7.74 (d, 1H), 3.43 (s, 2H), 2.37 (s, 3H), 1.73-1.66 (m, 1H), 1.03-1.00 (m, 2H), 0.75 (s, 2H).

Molecular formula: C₁₆H₁₆N₆O₂ Precise molecular weight: 324.13 LC-MS (m/z)=325.09[M+H]⁺.

### Example 70: Synthesis of compound N-(6-(4-cyano-2-hydroxyphenyl)-5-cyclopropylpyridazin-3-yl)-2-(methylamino)acetamide (Compound 72)

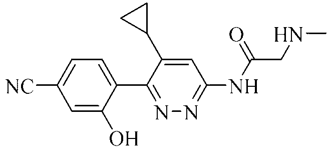

### Step 1: Synthesis of intermediate 4-bromo-3-(ethoxymethoxy)benzonitrile

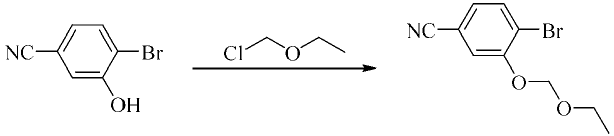

The product (3.5 g, yield: 93.7%) was prepared in accordance with the method in Step 1 of **Intermediate preparation example 6.**

### Step 2: Synthesis of 3-(ethoxymethoxy)-4-(4,4,5,5-tetramethyl-1,3,2-dioxaborolan-2-yl)benzonitrile

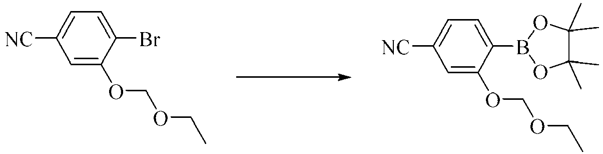

The product (1.1 g, yield: 46.4%) was prepared in accordance with the method in Step 3 of **Intermediate preparation example 5.**

### Step 3: Synthesis of tert-butyl (2-((6-(4-cyano-2-(ethoxymethoxy)phenyl)-5-cyclopropylpyridazin-3-yl)amino)-2-oxoethyl)(m ethyl)carbamate

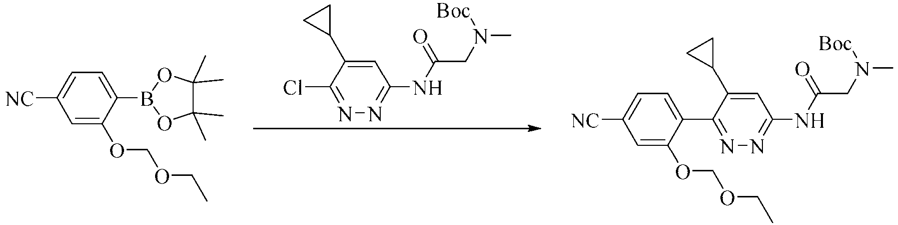

The compound was prepared in accordance with the method in Step 1 of **Example 46,** and the crude product was purified by silica gel column chromatography (dichloromethane:methanol=50:1), so as to afford the product (600 mg crude product).

### Step 4: Synthesis of N-(6-(4-cyano-2-hydroxyphenyl)-5-cyclopropylpyridazin-3-yl)-2-(methylamino)acetamide

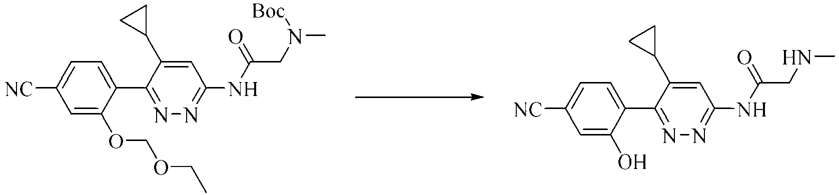

The compound was prepared in accordance with the method in Step 5 of **Example 69,** and the crude product was purified by preparative thin-layer chromatography (dichloromethane:methanol=10:1), so as to afford the product (80 mg, yield: 24.7%).

¹H-NMR (400 MHz, DMSO-*d₆*) δ(ppm): 7.82 (s, 1H), 7.49-7.47 (d, 1H), 7.42-7.40 (d, 1H), 7.34 (s, 1H), 3.57 (s, 2H), 2.43 (s, 2H), 1.66-1.60 (m, 1H), 1.04-0.99 (m, 2H), 0.74 (m, 2H).

Molecular formula: C₁₇H₁₇N₅O₂ Precise molecular weight: 323.14 LC-MS (m/z): 324.09 [M+H]⁺.

### Example 71: Synthesis of compound N-(6-(2-hydroxy-4-ethynylphenyl)-5-methyl-1,2,4-triazin-3-yl)-2-(methylamino)acetamide (Compound 21)

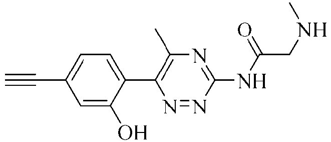

### Step 1: Synthesis of 5-methyl-3-(methylthio)-1,2,4-triazine

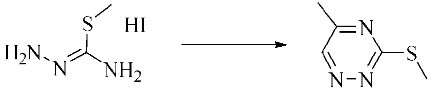

S-methylisothiosemicarbazide hydroiodide (30.92 g, 0.429 mol, 1.0 eq) was dissolved in water (300 mL). In an ice-water bath, sodium bicarbonate (36.04 g, 0.429 mol, 1.0 eq) was added thereto, an aqueous solution (360 mL) of 2-oxopropionaldehyde (100 g, 0.429 mol, 1.0 eq) was slowly added dropwise, and the resultant was reacted at 25°C for 24 h. TLC monitoring showed the completion of the reaction. The reaction solution was extracted with dichloromethane (100 mL×3). The organic phases were combined, dried over anhydrous sodium sulfate and concentrated. The crude product was purified by silica gel column chromatography (petroleum ether:ethyl acetate=10:1~4:1), so as to afford the product (24 g, yield: 39%).

### Step 2: Synthesis of 5-methyl-3-(methylsulfinyl)-1,2,4-triazine

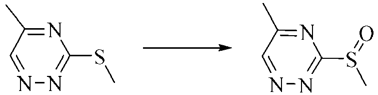

5-Methyl-3-(methylthio)-1,2,4-triazine (20 g, 0.1416 mol, 1.0 eq) was dissolved in dichloromethane (200 mL). In an ice-water bath, 85% m-chloroperoxybenzoic acid (31.63 g, 0.1833 mol, 1.1 eq) was slowly added thereto, and the resultant was reacted for 40 minutes. TLC monitoring showed the completion of the reaction. The resultant was filtered, and the filtrate was concentrated. The crude product was purified by silica gel column chromatography (dichloromethane), so as to afford the product (17.5 g, yield: 78.6%).

### Step 3: Synthesis of 5-methyl-1,2,4-triazin-3-amine

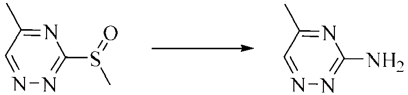

5-Methyl-3-(methylsulfinyl)-1,2,4-triazine (17.5 g, 0.1113 mol, 1.0 eq) was dissolved in tetrahydrofuran (25 mL). In an ice-water bath, the resultant was slowly added dropwise to a solution of ammonia in isopropanol (175 mL), and the reaction was allowed to proceed for 2.5 hours. TLC monitoring showed the completion of the reaction. The reaction solution was concentrated. The crude product was purified by silica gel column chromatography (methanol:ethyl acetate=0~1:10), so as to afford the product (4.16 g, yield: 34%).

### Step 4: Synthesis of intermediate 6-bromo-5-methyl-1,2,4-triazin-3-amine

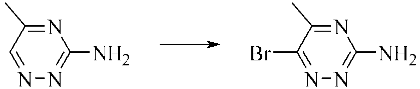

5-Methyl-1,2,4-triazin-3-amine (1 g, 9.1 mmol, 1.0 eq) was dissolved in dichloromethane (10 mL). At -10°C, dibromohydantoin (1.56 g, 5.4 mmol, 0.6 eq) was added, and the resultant was reacted at 25°C for 1 hour. TLC monitoring showed the completion of the reaction. The reaction solution was poured into saturated aqueous sodium bicarbonate solution (10 mL), and the resultant was extracted with dichloromethane (10 mL×3). The organic phases were combined, dried over anhydrous magnesium sulfate, filtered and concentrated. The crude product was purified by silica gel column chromatography (petroleum ether:ethyl acetate=1:1), so as to afford the product (1.1 g, yield: 64.3%).

### Step 5: Synthesis of 4-(3-amino-5-methyl-1,2,4-triazin-6-yl)-3-(ethoxymethoxy)benzaldehyde

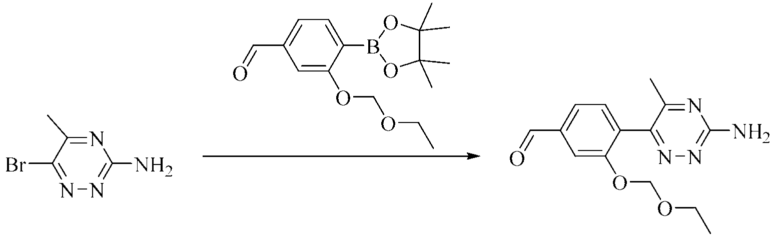

6-Bromo-5-methyl-1,2,4-triazin-3-amine (1.1 g, 5.8 mmol, 1.0 eq), 3-(ethoxymethoxy)-4-(4,4,5,5-tetramethyl-1,3,2-dioxaborolan-2-yl)benzaldehyde (3.86 g, 8.7 mmol, 1.5 eq), an aqueous solution (2 mL) of sodium bicarbonate (0.49 g, 5.8 mmol, 1.0 eq), and [1,1'-bis(diphenylphosphino)ferrocene]palladium(II) dichloride (0.426 g, 0.6 mmol, 0.1 eq) were added to 1,4-dioxane (8 mL). Under nitrogen protection, the resultant was reacted at 110°C for 3.5 hours. TLC monitoring showed the completion of the reaction. The reaction solution was filtered through celite. The filtrate was added with water (10 mL) and was extracted with dichloromethane (10 mL×3). The organic phases were combined, dried over anhydrous magnesium sulfate, and filtered. The filtrate was concentrated, and the crude product was purified by silica gel column chromatography (petroleum ether:ethyl acetate=3:1), so as to afford the product (0.8 g, yield: 50%).

### Step 6: Synthesis of 6-(2-(ethoxymethoxy)-4-ethynylphenyl)-5-methyl-1,2,4-triazin-3-amine

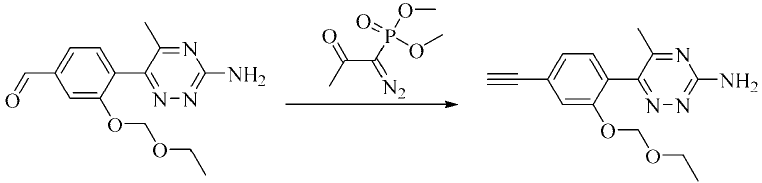

The compound was prepared in accordance with the method in Step 2 of Example 6, and the crude product was purified by silica gel column chromatography (petroleum ether:ethyl acetate=1:1), so as to afford the product (610 mg, yield: 77%).

### Step 7: Synthesis of intermediate tert-butyl (2-((6-(2-(ethoxymethoxy)-4-ethynylphenyl)-5-methyl-1,2,4-triazin-3-yl)amino)-2-oxoethyl)( methyl)carbamate

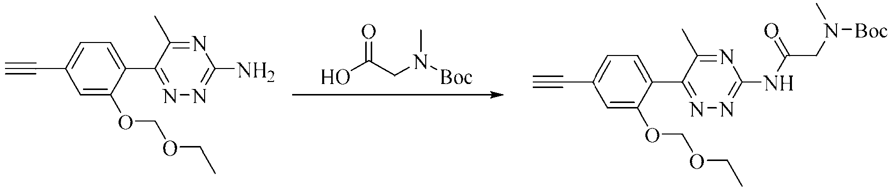

The compound was prepared in accordance with the method in Step 3 of **Example 6,** and the crude product was purified by silica gel column chromatography (petroleum ether:ethyl acetate=4:1~ethyl acetate only), so as to afford the product (210 mg, yield: 87.5%).

### Step 8: compound Synthesis of N-(6-(2-hydroxy-4-ethynylphenyl)-5-methyl-1,2,4-triazin-3-yl)-2-(methylamino)acetamide

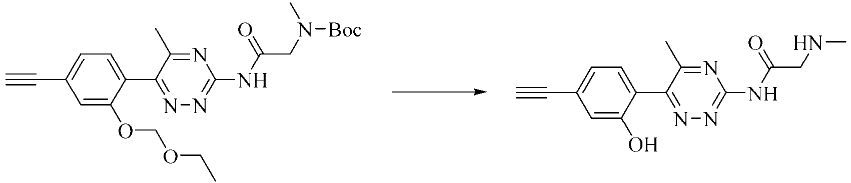

The compound was prepared in accordance with the method in Step 4 in **Example 3,** and the crude product was purified by silica gel column chromatography, so as to afford the product (13 mg, yield: 19.9%).

¹H-NMR (400 MHz, DMSO-*d₆*) δ(ppm): 7.49 (s, 1H), 7.23-7.22 (d, 1H), 7.05-7.03 (d, 2H), 6.97-6.95 (d, 2H), 4.26 (s, 2H), 4.17 (s, 1H), 3.20 (s, 3H), 2.23 (s, 3H).

Molecular formula: C₁₅H₁₅N₅O₂ Precise molecular weight: 297.12 LC-MS (m/z): 298.17 [M+H]⁺ .

### Example 72: Synthesis of compound N-(6-(2-hydroxy-4-ethynylphenyl)-5-cyclopropyl-1,2,4-triazin-3-yl)-2-(methylamino)aceta mide (Compound 48)

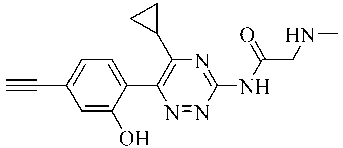

### Step 1: Synthesis of 2-cyclopropyl-2-oxoacetaldehyde

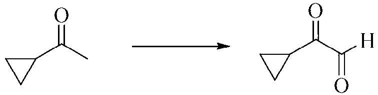

Selenium dioxide (18.47 g, 0.1664 mol, 1.4 eq), acetic acid (4.76 mL, 0.0832 mol, 0.7 eq) and water (3.32 g, 0.1842 mol, 1.55 eq) were added to 1,4-dioxane (75 mL), and the resultant was refluxed for two hours. 1-Cyclopropylethan-1-one (10 g, 0.1189 mmol, 1.0 eq) was added, and the resultant was reacted under reflux for 22 hours. TLC monitoring showed the completion of the reaction. The reaction solution was filtered, and the filter cake was rinsed with 1,4-dioxane. The filtrate was concentrated, and the crude product was used directly in the next step.

### Step 2: Synthesis of intermediate 5-cyclopropyl-3-(methylthio)-1,2,4-triazine

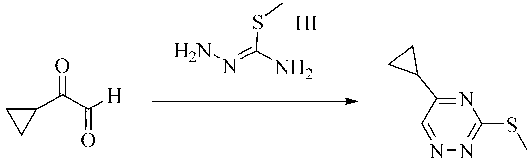

S-methylisothiosemicarbazide hydroiodide (35.58 g, 0.153 mol, 1.1 eq) was dissolved in water (100 mL). In an ice-water bath, sodium bicarbonate (28.31 g, 0.337 mol, 2.43 eq) was added, a mixture of the above solution of 2-cyclopropyl-2-oxoacetaldehyde and water (50 mL) was slowly added dropwise, and the resultant was reacted at 25°C for 24 h. TLC monitoring showed the completion of the reaction. Dichloromethane (300 mL×3) was added for extraction. The organic phases were combined, dried over anhydrous sodium sulfate and concentrated. The crude product was purified by silica gel column chromatography (petroleum ether:ethyl acetate=10:1~4:1), so as to afford the product (11.02 g, two-step yield: 55.4%).

### Step 3: Synthesis of intermediate 5-cyclopropyl-3-(methylsulfinyl)-1,2,4-triazine

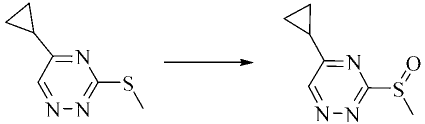

The compound was prepared in accordance with the method in Step 2 of **Example 71,** and the crude product was purified by silica gel column chromatography (petroleum ether:ethyl acetate=10:1), so as to afford the product (8.72 g, yield: 72.2%).

### Step 4: Synthesis of intermediate 5-cyclopropyl-1,2,4-triazin-3-amine

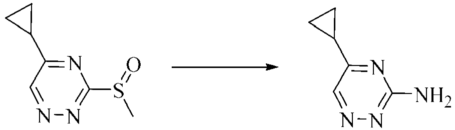

The compound was prepared in accordance with the method in Step 3 of **Example 71,** and the crude product was purified by silica gel column chromatography (ethyl acetate), so as to afford the product (5.86 g, yield: 90.4%).

### Step 5: Synthesis of intermediate 6-bromo-5-cyclopropyl-1,2,4-triazin-3-amine

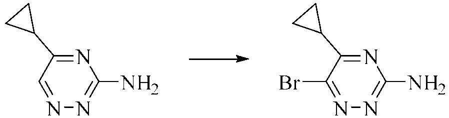

The compound was prepared in accordance with the method in Step 4 of **Example 71,** and the crude product was purified by silica gel column chromatography (petroleum ether:ethyl acetate=2:1), so as to afford the product (4.8 g, yield: 58.6%).

### Step 6: Synthesis of intermediate 4-(3-amino-5-cyclopropyl-1,2,4-triazin-6-yl)-3-(ethoxymethoxy)benzaldehyde

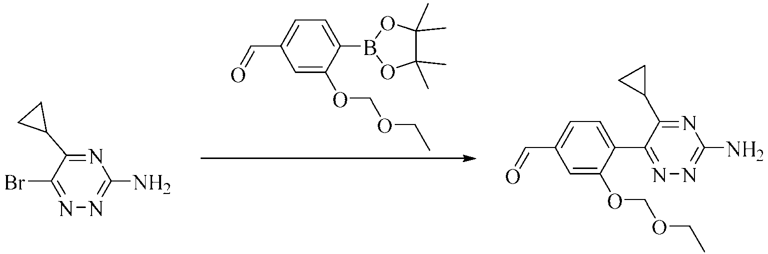

The compound was prepared in accordance with the method in Step 5 of **Example 71,** and the crude product was purified by silica gel column chromatography (petroleum ether:ethyl acetate=2:1), so as to afford the product (980 mg, yield: 67.1%).

### Step 7: Synthesis of compound 6-(2-(ethoxymethoxy)-4-ethynylphenyl)-5-cyclopropyl-1,2,4-triazin-3-amine

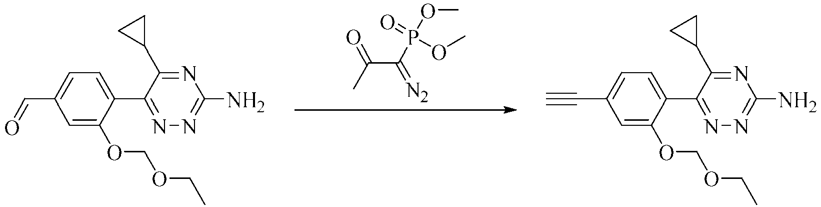

The compound was prepared in accordance with the method in Step 2 of **Example 6,** and the crude product was purified by silica gel column chromatography (petroleum ether:ethyl acetate=2: 1), so as to afford the product (860 mg, yield: 89.6%).

### Step 8: Synthesis of intermediate tert-butyl (2-((6-(2-(ethoxymethoxy)-4-ethynylphenyl)-5-cyclopropyl-1,2,4-triazin-3-yl)amino)-2-oxoeth yl)(methyl)carbamate

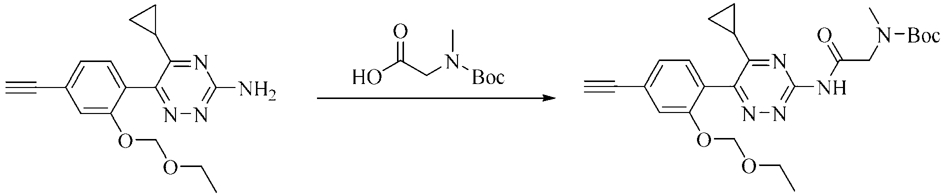

The compound was prepared in accordance with the method in Step 3 of **Example 6,** and the crude product was purified by silica gel column chromatography (petroleum ether:ethyl acetate=1:1), so as to afford the product (790 mg, yield: 59.4%).

### Step 9: Synthesis of compound N-(6-(2-hydroxy-4-ethynylphenyl)-5-cyclopropyl-1,2,4-triazin-3-yl)-2-(methylamino)acetamid e

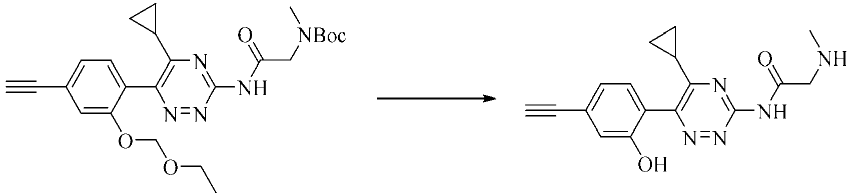

The compound was prepared in accordance with the method in Step 4 of **Example 3,** and the crude product was purified by preparative thin-layer chromatography, so as to afford the product (8 mg, yield: 2.3%).

¹H-NMR (400 MHz, DMSO-*d₆*) δ(ppm): 10.11 (s, 1H), 7.43 (s, 1H), 7.29-7.27 (d, 1H), 7.05-7.01 (m, 3H), 4.21 (m, 3H), 3.19 (s, 3H), 1.69 (s, 1H), 1.04-0.97 (m, 4H).

Molecular formula: C₁₇H₁₇N₅O₂ Precise molecular weight: 323.14 LC-MS (m/z): 324.12 [M+H]⁺ .

### Example 73: Synthesis of (R)-2-amino-N-(6-(2-hydroxy-4-ethynylphenyl)-5-cyclopropyl-1,2,4-triazin-3-yl)propana mide (Compound 49)

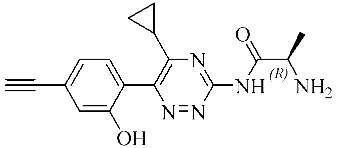

The compound was prepared in accordance with the synthetic methods in Steps 8 and 9 of **Example 72.**

¹H-NMR (400 MHz, DMSO-*d₆*) δ(ppm): 10.05 (s, 1H), 7.55 (s, 1H), 7.29-7.27 (d, 2H), 7.04-7.02 (m, 2H), 6.94 (s, 1H), 4.20 (s, 2H), 1.67(s, 1H), 1.36-1.34(d, 3H), 1.05-0.95 (m, 4H).

Molecular formula: C₁₇H₁₇N₅O₂ Precise molecular weight: 323.14 LC-MS (m/z): 324.14 [M+H]⁺ .

### Example 74: Synthesis of N-(5-cyclopropyl-6-(2-hydroxy-4-(trifluoromethyl)phenyl)-1,2,4-triazin-3-yl)-2-(methyla mino)acetamide (Compound 60)

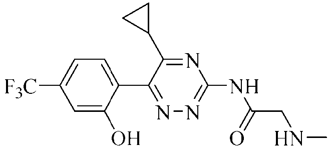

### Step 1: Synthesis of 5-cyclopropyl-6-(2-(ethoxymethoxy)-4-(trifluoromethyl)phenyl)-1,2,4-triazin-3-amine

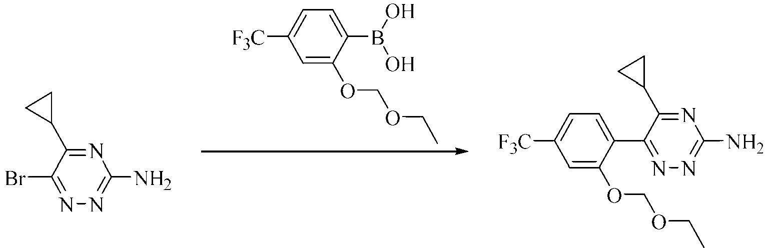

The compound was prepared in accordance with the method in Step 1 of **Example 6,** and the crude product was purified by silica gel column chromatography (PE:EA=1:1), so as to afford the product (1.47g, yield: 91.8%).

### Step 2: Synthesis of tert-butyl (2-((5-cyclopropyl-6-(2-(ethoxymethoxy)-4-(trifluoromethyl)phenyl)-1,2,4-triazin-3-yl)amino) -2-oxoethyl)(methyl)carbamate

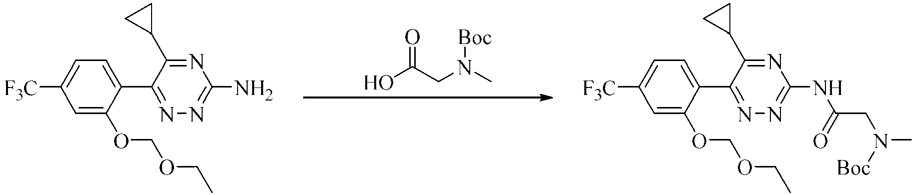

The compound was prepared in accordance with the method in Step 3 of **Example 6,** so as to afford the product (0.51 g, yield: 80.0%).

### Step 3: Synthesis of N-(5-cyclopropyl-6-(2-hydroxy-4-(trifluoromethyl)phenyl)-1,2,4-triazin-3-yl)-2-(methylamino) acetamide

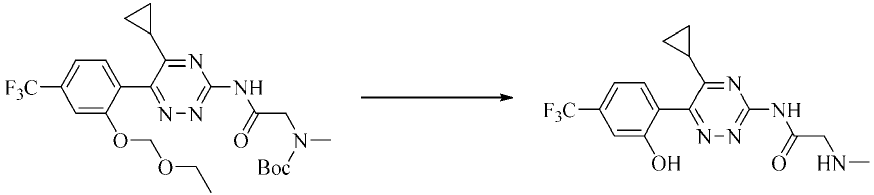

The compound was prepared in accordance with the method in Step 4 of **Example 3,** and the crude product was purified by reversed-phase column chromatography, so as to afford the product (21 mg).

¹HNMR (400 MHz, DMSO-*d*₆) δ(ppm): 11.64 (s, 1H), 10.88 (s, 1H), 8.88 (s, 2H), 7.63-7.61 (d, 1H), 7.36-7.34 (d, 1H), 7.30 (s, 1H), 4.29 (s, 2H), 3.65 (s, 3H), 1.86-1.80 (m, 1H), 1.18 (s, 3H).

Molecular formula: C₁₆H₁₆F₃N₅O₂ Precise molecular weight: 368.26 LC-MS (m/z): 367.13 [M+H]⁺.

### Example 75: Synthesis of N-(6-(2-hydroxy-4-(trifluoromethyl)phenyl)-5-methyl-1,2,4-triazin-3-yl)-2-(methylamino) acetamide (Compound 22)

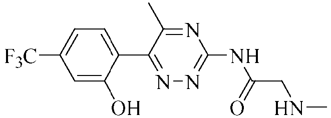

### Step 1: Synthesis of 6-(2-(ethoxymethoxy)-4-(trifluoromethyl)phenyl)-5-methyl-1,2,4-triazin-3-amine

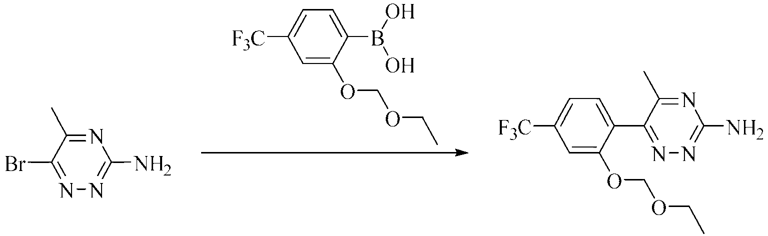

The compound was prepared in accordance with the method in Step 1 of **Example 6,** so as to afford the product (0.64 g, yield: 52.9%).

### Step 2: Synthesis of tert-butyl (2-((6-(2-(ethoxymethoxy)-4-(trifluoromethyl)phenyl)-5-methyl-1,2,4-triazin-3-yl)amino)-2-ox oethyl)(methyl)carbamate

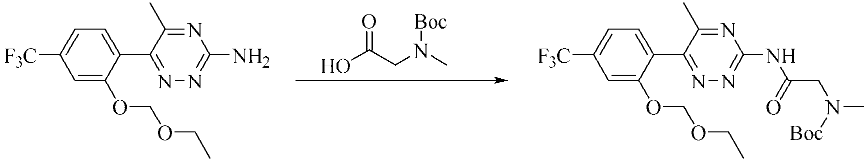

The product (0.36 g, yield: 69.6%) was prepared in accordance with the method in Step 3 of **Example 6.**

### Step 3: Synthesis of N-(6-(2-hydroxy-4-(trifluoromethyl)phenyl)-5-methyl-1,2,4-triazin-3-yl)-2-(methylamino)aceta mide

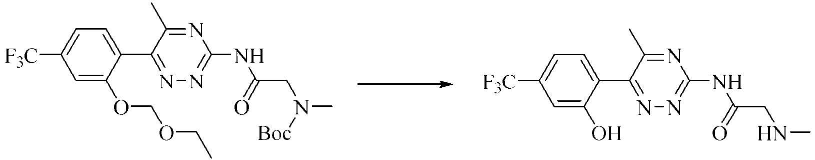

The compound was prepared in accordance with the method in Step 4 of **Example 3,** and the crude product was purified by reversed-phase column chromatography, so as to afford the product (4.0 mg).

¹HNMR (400 MHz, DMSO-*d₆*) δ(ppm): 7.49 (s, 1H), 7.46-7.44 (d, 1H), 7.18 (s, 2H), 7.07 (s, 1H), 4.28 (s, 2H), 3.22 (s, 3H), 2.25 (s, 3H).

Molecular formula: C₁₄H₁₄F₃N₅O₂ Precise molecular weight: 341.11 LC-MS (m/z): 342.21 [M+H]⁺.

### Experimental Example 1: Cell assay of inhibitory activity of the compounds of the present disclosure against NLRP3 inflammasome

Test compounds: the compounds of the present disclosure prepared in accordance with the methods described in the Examples

THP-1 was an immortalized human macrophage cell line.

Test instrument: Microplate reader (manufactured by PE)

### Experimental method:

1. THP-1 cells were cultured in 1640 complete medium (500 ml 1640+56 ml FBS+560 µl 1000× P/S+2 µl mercaptoethanol), and used within 3 to 20 passages.
2. Coating of culture plates: 100 µl of polylysine solution was added into a 96-well cell culture plate and incubated at 37°C for 30min. The solution was discarded, and the plate was washed twice with PBS for later use.
3. Induction of differentiation of THP-1: THP-1 cells were resuspended in an appropriate amount of complete medium containing 10 ng/ml PMA to achieve a cell density of 5×10⁵ cells /ml in the suspension. The cell suspension was added into a 96-well plate with 100 µl of cell suspension per well, and the 96-well plate was cultured overnight for 16 hours in a 37°C CO₂ cell incubator.
4. Stimulation of THP-1:
   a. Serum-free THP-1 culture medium containing LPS was added to achieve a final concentration of 500 ng/ml, and the resultant was cultured in a 37°C CO₂ cell incubator for 3h;
   b. The compounds to be tested were prepared into stock solutions at gradient concentrations with DMSO. The resulting solutions were added to the cells, evenly mixed, and finally diluted at 1:1000. The 96-well plate was cultured in a 37°C CO₂ cell incubator for 1h.
   c. Nigericin was added to each well to achieve a final concentration of 10 µg/ml, and the resultant was cultured in a 37°C CO₂ cell incubator for 30 min.
   d. The resulting culture medium in each well was transferred to a new culture plate, which was then centrifuged at 3000 rpm for 5min. Afterwards, the supernatant was transferred to a new 96-well plate.
   e. The collected supernatant samples of the cell culture medium were determined by using a commercial caspase-1 glo kit, and the content of human active caspase-1 was determined according to the manufacturer's instructions.

The test results were as shown in Table 2 below.

**Table 2 Inhibitory Activity of the compounds of the present disclosure against NLRP3 in THP-1 cells**

| Test compound | IC₅₀ (nM) |
|---|---|
| Compound 1 | 16.5 |
| Compound 2 | 18.1 |
| Compound 3 | 32.4 |
| Compound 7 | 1.3 |
| Compound 8 | 1.4 |
| Compound 9 | 9.7 |
| Compound 10 | 19.2 |
| Compound 13 | 23.2 |
| Compound 16 | 8.5 |
| Compound 17 | 17.8 |
| Compound 19 | 3.4 |
| Compound 20 | 0.3 |
| Compound 31 | 13.1 |
| Compound 33 | 14.5 |
| Compound 34 | 18.8 |
| Compound 36 | 2.9 |
| Compound 37 | 29.4 |
| Compound 43 | 21.4 |
| Compound 44 | 6.4 |
| Compound 50 | 6.8 |
| Compound 51 | 14.6 |
| Compound 52 | 12.9 |
| Compound 53 | 5.9 |
| Compound 55 | 23.6 |
| Compound 56 | 7.1 |
| Compound 57 | 17.5 |
| Compound 59 | 32.3 |
| Compound 60 | 32.1 |
| Compound 61 | 6.2 |
| Compound 62 | 20.9 |
| Compound 63 | 7.2 |
| Compound 64 | 14.1 |
| Compound 65 | 31.5 |
| Compound 66 | 13.9 |
| Compound 67 | 5.6 |

As could be seen from the experimental results in Table 2, the compounds of the present disclosure exhibited good inhibitory activity against the NLRP3 inflammasome. Therefore, the compounds of the present disclosure could be used for preventing and/or treating NLRP3 inflammasome-related diseases.

### Experimental Example 2: Experimental protocol for pharmacokinetics of pompounds in mice

1. Mouse Strain: C57BL/6J.
2. A mixed vehicle was formulated using dimethyl sulfoxide (5%), PEG400 (20%) and 20% polyoxyethylene hydrogenated castor oil (75%). An appropriate amount of the compound of the present disclosure was weighed and dissolved in said mixed vehicle for oral gavage and intravenous administration.
3. Time points for blood sampling: For oral gavage: 15 min, 30 min, 1 h, 2 h, 4 h, 6 h, 8 h and 24 h after administration; for intravenous administration: 5 min, 15 min, 30 min, 1 h, 2 h, 4 h, 6 h, 8 h and 24 h after administration.
4. Sample analysis method: The samples to be tested were vortexed for 5 min. 10 µL of plasma samples from different individuals were precisely pipetted into 1.5 mL centrifuge tubes, and then the working solution of the internal standard (for example, a solution of tolbutamide in acetonitrile) was added thereto. The resultant was evenly mixed, vortexed for 5 min, and then centrifuged at 12000 rpm for 5 min. 50 µL of the supernatant was precisely pipetted into a 96-well plate previously added with 150 µL/well of water. The resulting mixture was vortexed and evenly mixed, and was then subjected to LC-MS/MS analysis.
5. Data Processing Method: The concentrations of the test compounds were output using Analyst 1.6.3 (AB Sciex). Parameters such as mean, standard deviation and coefficient of variation were calculated by Microsoft Excel (parameters directly output by Analyst 1.6.3 did not require calculation). PK parameters were calculated using the NCA in WinNonlin Phenoix 8.2 software (Tₘₐₓ represented the median value).

### Experimental Example 3: Experimental protocol for inhibition of hERG potassium ion channel by compounds

1. Cell Culture
   1.1 HEK-293 cell line stably expressing the hERG potassium ion channel was cultured in DMEM medium containing 10% fetal bovine serum and 0.8 mg/mL G418.
   1.2 Prior to the patch-clamp assay, cells were detached using TrypLE^{™} Express. 4×10³ cells were plated onto a coverslip, and cultured in a 24-well plate.
2. Formulation method of the stock solutions of the compounds of the present disclosure
   An appropriate mass of the compound was weighed. The volume of DMSO required was calculated according to the equation: the volume of DMSO = actual mass × purity (content) / (molecular weight × theoretical concentration). The corresponding volume of DMSO was pipetted, and then the weighed compound was dissolved with the pipetted DMSO.
3. Formulation method of the working solutions of the compounds of the present disclosure
   The highest test concentration of the compound was achieved by directly diluting the stock solution with extracellular fluid or by further diluting the stock solution with DMSO as needed. Other concentrations were achieved by formulating serial dilutions from high concentration to low concentration with DMSO and then diluting the dilutions with extracellular fluid to the concentrations of working solutions.
4. Patch-clamp Testing
   After forming a whole-cell configuration, the cell membrane voltage was clamped at -80 mV. The clamping voltage was depolarized from -80 mV to -50 mV for 0.5 s (for leakage current detection), then stepped to 30 mV for 2.5 s, and quickly returned to -50 mV for 4 s, so as to elicit the tail current of the hERG channel. The data were collected repeatedly every 10 s, so as to observe the effect of the compounds on the hERG tail current. The stimulation at -50 mV for 0.5 s was used for detecting the leakage current. Experimental data were collected by an EPC 10 amplifier (HEKA) and stored in PatchMaster (HEKA) software.
5. Data Analysis
   The calculation of IC₅₀ and curve fitting were completed using GraphPad Prism software. The above descriptions are merely preferred examples of the present disclosure, and are not intended to limit the present disclosure. Any modifications, equivalent substitutions, improvements and the like made within the spirits and principles of the present disclosure shall be included within the protection scope of the present disclosure.

## Claims

1. A compound represented by general formula (I), or a deuterated compound thereof, a stereoisomer thereof or a pharmaceutically acceptable salt thereof:
Y-W-R₃ (I)
wherein W is selected from the group consisting of 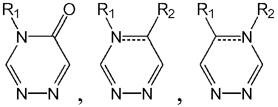 and 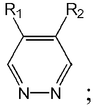
-̅ -̅ -̅ -̅ -̅ is a single bond or a double bond;
R₁ is independently selected from the group consisting of hydrogen, oxo, thio, hydroxyl, amino, carboxyl, cyano, nitro, halogen, carbonyl, C₁₋₆ alkyl, halogenated C₁₋₆ alkyl, C₁₋₆ alkoxy, -S-C₁₋₆ alkyl, halogenated C₁₋₆ alkoxy, C₂₋₆ alkenyl, C₂₋₆ alkynyl, 3-7 membered heterocyclyl, 3-7 membered cycloalkyl, 5-7 membered cycloalkenyl, aryl, 5-7 membered heteroaryl, sulfonyl, -N(C₁₋₆ alkyl)₂, and null;
R₂ is independently selected from the group consisting of hydrogen, oxo, thio, hydroxyl, amino, carboxyl, cyano, nitro, halogen, carbonyl, C₁₋₆ alkyl, halogenated C₁₋₆ alkyl, C₁₋₆ alkoxy, -S-C₁₋₆ alkyl, halogenated C₁₋₆ alkoxy, C₂₋₆ alkenyl, C₂₋₆ alkynyl, 3-7 membered heterocyclyl, 3-7 membered cycloalkyl, 5-7 membered cycloalkenyl, aryl, 5-7 membered heteroaryl, sulfonyl, -N(C₁₋₆ alkyl)₂, and null;
said R₁ and R₂ are independently and optionally substituted with 1-3 substituents selected from the group consisting of hydroxyl, C₀₋₆ alkylamino, carboxyl, cyano, nitro, halogen, C₀₋₆ alkylcarbonyl, C₁₋₆ alkyl, halogenated C₁₋₆ alkyl, C₁₋₆ alkoxy, halogenated C₁₋₆ alkoxy, C₂₋₆ alkenyl, C₂₋₆ alkynyl, 3-7 membered heterocyclyl, 3-7 membered cycloalkyl, 5-7 membered cycloalkenyl, aryl, 5-7 membered heteroaryl, and C₀₋₆ alkylsulfonyl;
or, R₁ and R₂, together with the C atom or N atom to which they are attached, form a 5-12 membered ring A;
said 5-12 membered ring A is optionally substituted with 1-4 substituents selected from the group consisting of hydroxyl, amino, carboxyl, cyano, nitro, halogen, carbonyl, oxo, C₁₋₆ alkyl, -NH-C₁₋₆ alkyl, halogenated C₁₋₆ alkyl, C₁₋₆ alkoxy, halogenated C₁₋₆ alkoxy, 3-7 membered heterocyclyl, 3-7 membered cycloalkyl, aryl, 5-7 membered heteroaryl, C₁₋₆ alkylsulfonyl, and -N(C₁₋₆ alkyl)₂;
R₃ is -(CH₂)ₙ-NR₄-Z-(CH₂)ₘ-R₅ or -(CH₂)ₙ-NR₄-Z-CR^{c}R^{d}-R₅;
n is an integer from 0 to 6;
m is an integer from 0 to 3;
R₄ is hydrogen or C₁₋₆ alkyl;
Z is selected from the group consisting of C=O, C=S, S(O) and S(O)₂;
R₅ is selected from the group consisting of 3-7 membered heterocyclyl, 3-7 membered cycloalkyl, 5-7 membered cycloalkenyl, aryl, 5-7 membered heteroaryl, NR^{a}R^{b}, OR^{b}, and C₁₋₆ alkyl;
R^{a} is hydrogen or C₁₋₆ alkyl;
R^{c} is hydrogen or C₁₋₆ alkyl;
R^{b} is selected from the group consisting of hydrogen, C₁₋₆ alkyl, halogenated C₁₋₆ alkyl, C₁₋₆ alkoxy, -S-C₁₋₆ alkyl, halogenated C₁₋₆ alkoxy, C₂₋₆ alkenyl, C₂₋₆ alkynyl, 3-7 membered heterocyclyl, 3-7 membered cycloalkyl, 5-7 membered cycloalkenyl, aryl, 5-7 membered heteroaryl, sulfonyl, and -N(C₁₋₆ alkyl)₂;
said R^{b} is optionally substituted with 1-2 substituents selected from the group consisting of hydroxyl, halogen, 3-7 membered cycloalkyl, halogenated C₁₋₆ alkyl, and C₁₋₆ alkoxy;
R^{d} is selected from the group consisting of hydrogen, C₁₋₆ alkyl, halogenated C₁₋₆ alkyl, C₁₋₆ alkoxy, -S-C₁₋₆ alkyl, halogenated C₁₋₆ alkoxy, C₂₋₆ alkenyl, C₂₋₆ alkynyl, 3-7 membered heterocyclyl, 3-7 membered cycloalkyl, 5-7 membered cycloalkenyl, aryl, 5-7 membered heteroaryl, sulfonyl, and -N(C₁₋₆ alkyl)₂;
said R^{d} is optionally substituted with 1-2 substituents selected from the group consisting of hydroxyl, halogen, 3-7 membered cycloalkyl, halogenated C₁₋₆ alkyl, C₁₋₆ alkoxy, aryl, cyano, and carboxyl;
or, R^{c} and R^{d}, together with the C atom to which they are attached, form a 3-7 membered cycloalkyl;
or, when R₅ is NR^{a}R^{b}, R^{a} is attached to R₄ such that R₃ forms a 4-7 membered heterocyclyl;
said R₅ is optionally substituted with 1-4 substituents selected from the group consisting of oxo, thio, hydroxyl, amino, carboxyl, cyano, nitro, halogen, carbonyl, C₁₋₆ alkyl, halogenated C₁₋₆ alkyl, C₁₋₆ alkoxy, -S-C₁₋₆ alkyl, halogenated C₁₋₆ alkoxy, C₂₋₆ alkenyl, C₂₋₆ alkynyl, 3-7 membered heterocyclyl, 3-7 membered cycloalkyl, 5-7 membered cycloalkenyl, aryl, 5-7 membered heteroaryl, sulfonyl, ureido, and hydrazino;
a substituent on R₅ may be further optionally substituted with 1-3 substituents selected from the group consisting of hydroxyl, C₀₋₆ alkylamino, carboxyl, cyano, nitro, halogen, C₀₋₆ alkylcarbonyl, C₁₋₆ alkyl, halogenated C₁₋₆ alkyl, C₁₋₆ alkoxy, -S-C₁₋₆ alkyl, halogenated C₁₋₆ alkoxy, C₂₋₆ alkenyl, C₂₋₆ alkynyl, 3-6 membered heterocyclyl, 3-6 membered cycloalkyl, 5-6 membered cycloalkenyl, aryl, 5-7 membered heteroaryl, C₀₋₆ alkylsulfonyl, ureido, and hydrazino;
Y is selected from the group consisting of aryl, 5-14 membered heteroaryl, 3-14 membered heterocyclyl, 3-12 membered cycloalkyl, and 3-12 membered cycloalkenyl;
said Y is optionally substituted with 1-4 substituents selected from the group consisting of oxo, thio, hydroxyl, amino, carboxyl, cyano, nitro, halogen, carbonyl, C₁₋₆ alkyl, halogenated C₁₋₆ alkyl, C₁₋₆ alkoxy, -S-C₁₋₆ alkyl, halogenated C₁₋₆ alkoxy, C₂₋₆ alkenyl, C₂₋₆ alkynyl, 3-7 membered heterocyclyl, 3-7 membered cycloalkyl, 5-7 membered cycloalkenyl, aryl, 5-7 membered heteroaryl, and sulfonyl;
a substituent on Y may be further optionally substituted with 1-3 substituents selected from the group consisting of hydroxyl, C₀₋₆ alkylamino, carboxyl, cyano, nitro, halogen, C₀₋₆ alkylcarbonyl, C₁₋₆ alkyl, halogenated C₁₋₆ alkyl, C₁₋₆ alkoxy, -S-C₁₋₆ alkyl, halogenated C₁₋₆ alkoxy, C₂₋₆ alkenyl, C₂₋₆ alkynyl, 3-6 membered heterocyclyl, 3-7 membered cycloalkyl, 5-6 membered cycloalkenyl, aryl, 5-7 membered heteroaryl, and C₀₋₆ alkylsulfonyl.

2. The compound, or the deuterated compound thereof, the stereoisomer thereof or the pharmaceutically acceptable salt thereof according to claim 1, wherein W is

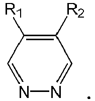

3. The compound, or the deuterated compound thereof, the stereoisomer thereof or the pharmaceutically acceptable salt thereof according to claim 1 or 2,
wherein R₁ and R₂ are independently selected from the group consisting of hydrogen, cyano, halogen, C₁₋₆ alkyl, halogenated C₁₋₆ alkyl, C₁₋₆ alkoxy, halogenated C₁₋₆ alkoxy, 3-7 membered cycloalkyl, -N(C₁₋₆ alkyl)₂, and null;
preferably, R₁ and R₂ are independently selected from the group consisting of hydrogen, cyano, halogen, C₁₋₆ alkyl, halogenated C₁₋₆ alkyl, cyclopropyl, cyclobutyl, and null.

4. The compound, or the deuterated compound thereof, the stereoisomer thereof or the pharmaceutically acceptable salt thereof according to claim 1,
wherein R₃ is -(CH₂)ₙ-NR₄-Z-(CH₂)ₘ-R₅ or -(CH₂)ₙ-NR₄-Z-CR^{c}R^{d}-R₅;
n is an integer from 0 to 2;
m is an integer from 0 to 2;
R₄ is hydrogen or C₁₋₆ alkyl;
Z is C=O;
R₅ is selected from the group consisting of 3-7 membered heterocyclyl, NR^{a}R^{b}, OR^{b}, and C₁₋₆ alkyl;
R^{a} is hydrogen or C₁₋₆ alkyl;
R^{c} is hydrogen or C₁₋₆ alkyl;
R^{b} is selected from the group consisting of hydrogen, C₁₋₆ alkyl, halogenated C₁₋₆ alkyl, C₁₋₆ alkoxy, -S-C₁₋₆ alkyl, halogenated C₁₋₆ alkoxy, 3-7 membered heterocyclyl, 3-7 membered cycloalkyl, aryl, and 5-7 membered heteroaryl;
said R^{b} is optionally substituted with 1-2 substituents selected from the group consisting of hydroxyl, halogen, 3-7 membered cycloalkyl, halogenated C₁₋₆ alkyl, and C₁₋₆ alkoxy;
R^{d} is selected from the group consisting of hydrogen, C₁₋₆ alkyl, halogenated C₁₋₆ alkyl, C₁₋₆ alkoxy, -S-C₁₋₆ alkyl, halogenated C₁₋₆ alkoxy, 3-7 membered heterocyclyl, 3-7 membered cycloalkyl, aryl, and 5-7 membered heteroaryl;
said R^{d} is optionally substituted with 1-2 substituents selected from the group consisting of hydroxyl, halogen, 3-7 membered cycloalkyl, halogenated C₁₋₆ alkyl, C₁₋₆ alkoxy, aryl, cyano, and carboxyl.

5. The compound, or the deuterated compound thereof, the stereoisomer thereof or the pharmaceutically acceptable salt thereof according to claim 4, wherein
n is 0;
m is 0;
R₅ is 3-7 membered heterocyclyl;
said R₅ is optionally substituted with 1-4 substituents selected from the group consisting of hydroxyl, amino, carboxyl, cyano, halogen, C₁₋₆ alkyl, halogenated C₁₋₆ alkyl, C₁₋₆ alkoxy, 3-7 membered heterocyclyl, 3-7 membered cycloalkyl, and aryl;
a substituent on R₅ may be further optionally substituted with 1-3 substituents selected from the group consisting of hydroxyl, halogen, and C₁₋₆ alkyl;
preferably, R₅ is a 4-6 membered heterocyclyl containing one N heteroatom.

6. The compound, or the deuterated compound thereof, the stereoisomer thereof or the pharmaceutically acceptable salt thereof according to claim 4, wherein
n is 0;
m is 0;
R₅ is NR^{a}R^{b}; R^{a} is hydrogen or C₁₋₆ alkyl;
R^{c} is hydrogen or C₁₋₆ alkyl;
R^{b} is selected from the group consisting of hydrogen, C₁₋₆ alkyl, halogenated C₁₋₆ alkyl, C₁₋₆ alkoxy, cyclopropyl, and cyclobutyl;
said R^{b} is optionally substituted with 1-2 substituents selected from the group consisting of hydroxyl, halogen, cyclopropyl, cyclobutyl, and C₁₋₆ alkoxy;
R^{d} is selected from the group consisting of hydrogen, C₁₋₆ alkyl, halogenated C₁₋₆ alkyl, C₁₋₆ alkoxy, cyclopropyl, and cyclobutyl;
said R^{d} is optionally substituted with 1-2 substituents selected from the group consisting of hydroxyl, halogen, cyclopropyl, cyclobutyl, halogenated C₁₋₆ alkyl, C₁₋₆ alkoxy, and phenyl.

7. The compound, or the deuterated compound thereof, the stereoisomer thereof or the pharmaceutically acceptable salt thereof according to claim 1 or 2,
wherein Y is phenyl or 5-14 membered heteroaryl;
said Y is optionally substituted with 1-4 substituents selected from the group consisting of hydroxyl, amino, cyano, halogen, C₁₋₆ alkyl, halogenated C₁₋₆ alkyl, C₁₋₆ alkoxy, -S-C₁₋₆ alkyl, 3-7 membered cycloalkyl, C₂₋₆ alkenyl, and C₂₋₆ alkynyl;
when a substituent on Y is selected from the group consisting of C₁₋₆ alkyl, 3-7 membered cycloalkyl, C₂₋₆ alkenyl, and C₂₋₆ alkynyl, said substituent is further optionally substituted with 1-3 substituents selected from the group consisting of hydroxyl, halogen, C₁₋₆ alkyl, halogenated C₁₋₆ alkyl, and 3-7 membered cycloalkyl;
preferably, said Y is substituted with hydroxyl, Y may also be further optionally substituted with 1-3 substituents selected from the group consisting of amino, cyano, halogen, C₁₋₆ alkyl, halogenated C₁₋₆ alkyl, C₁₋₆ alkoxy, C₂₋₆ alkenyl, C₂₋₆ alkynyl, and cyclopropyl, the substituent on Y may be further optionally substituted with 1-3 substituents selected from the group consisting of halogen, C₁₋₆ alkyl, cyclopropyl, trifluoromethyl, and difluoromethyl.

8. Compounds as follows, or deuterated compounds thereof, stereoisomers thereof or pharmaceutically acceptable salts thereof:

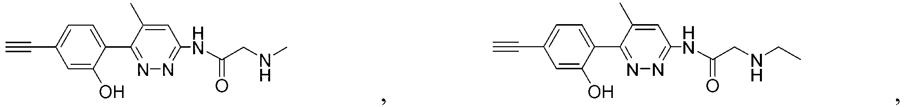

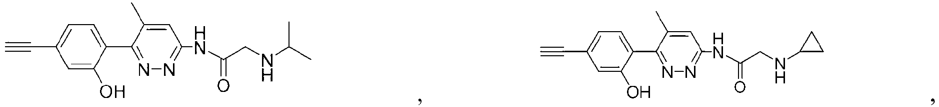

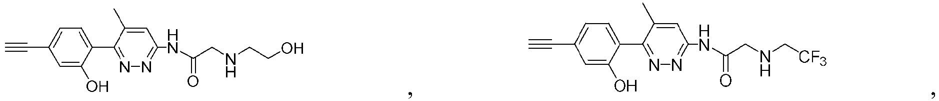

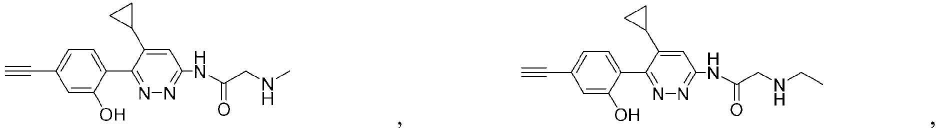

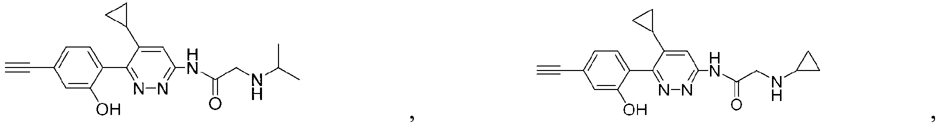

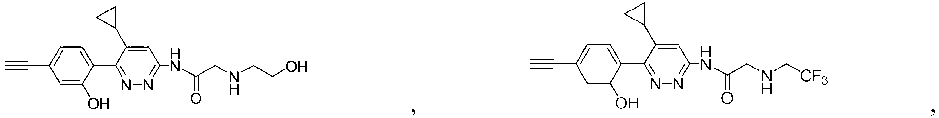

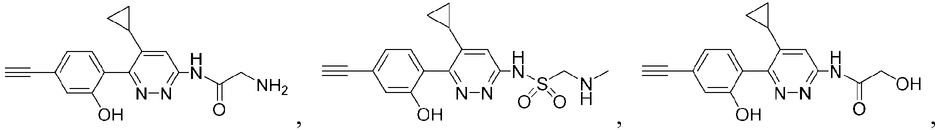

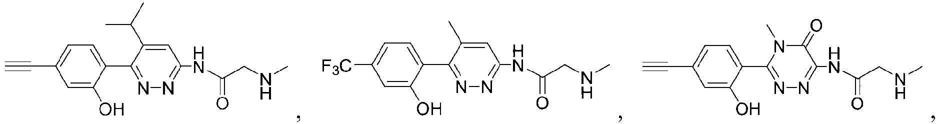

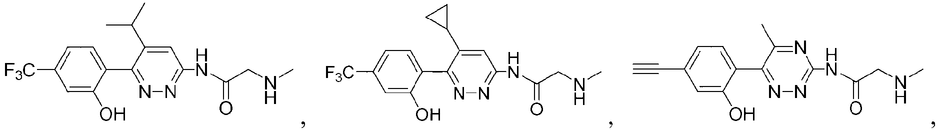

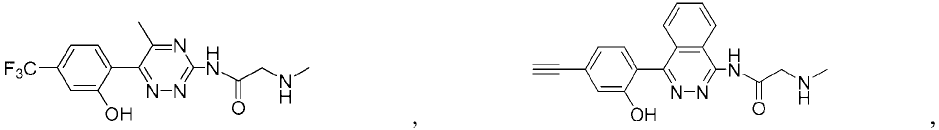

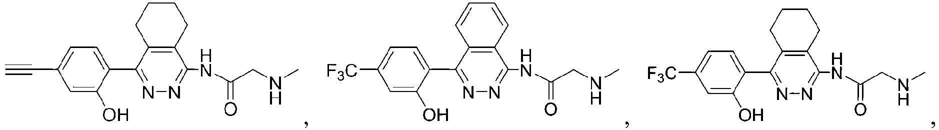

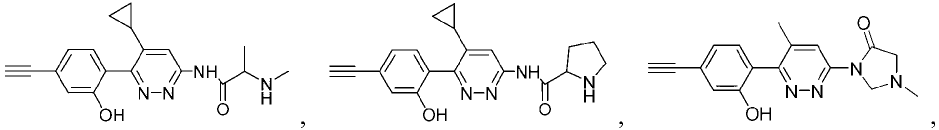

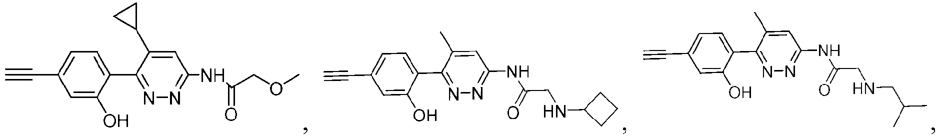

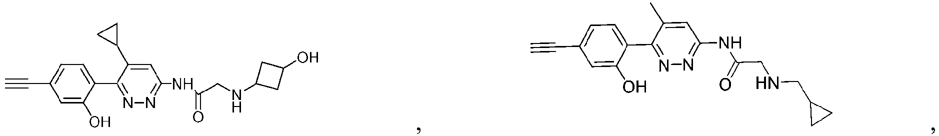

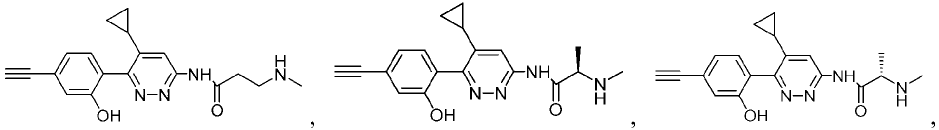

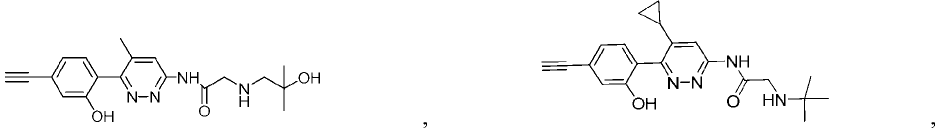

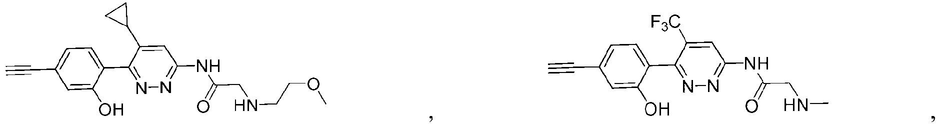

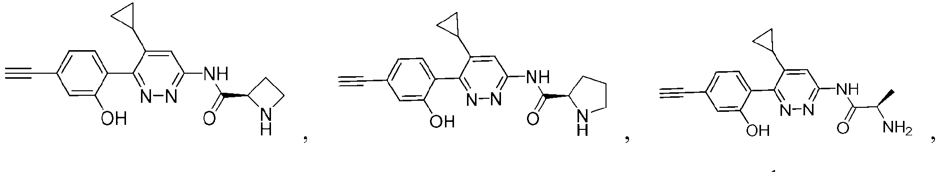

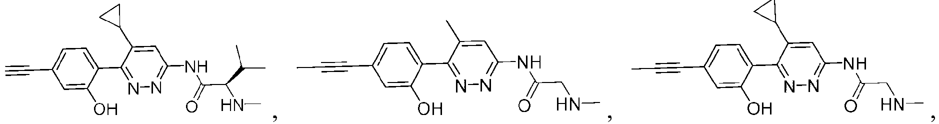

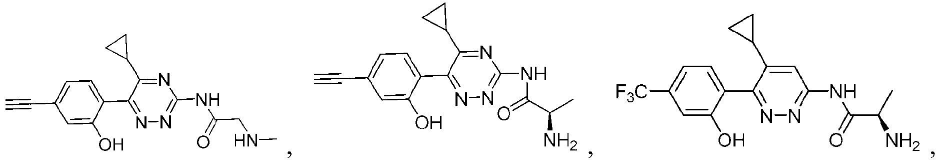

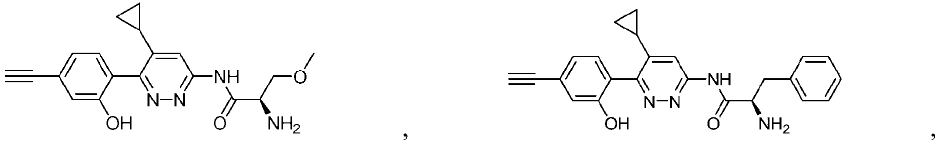

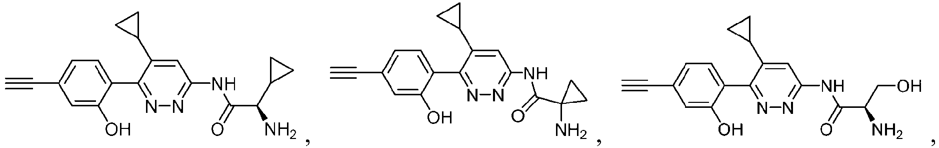

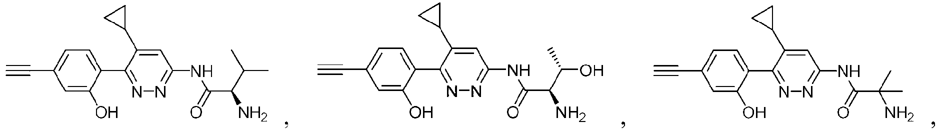

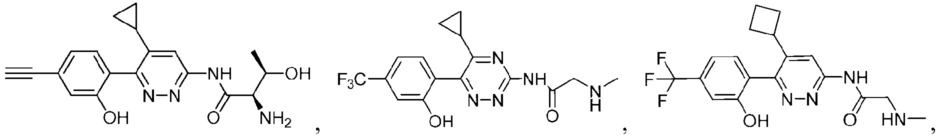

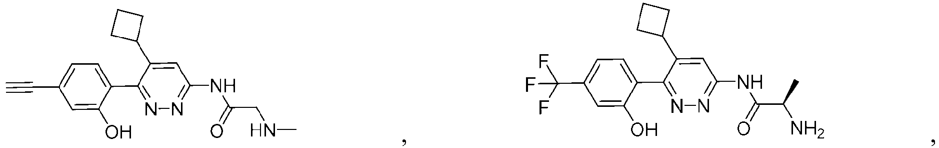

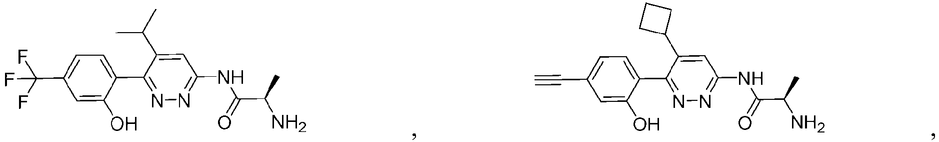

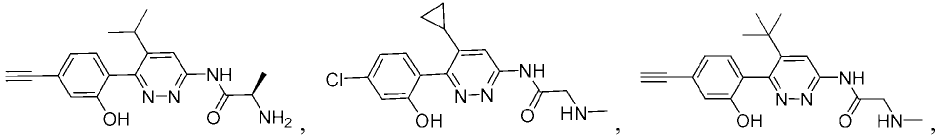

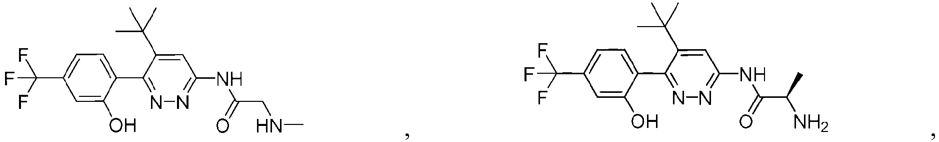

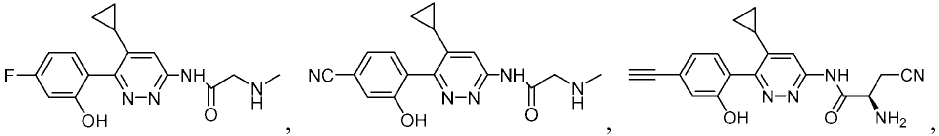

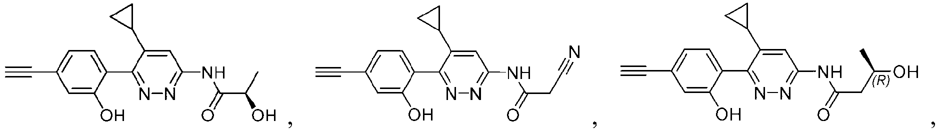

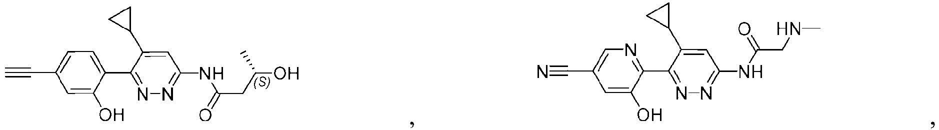

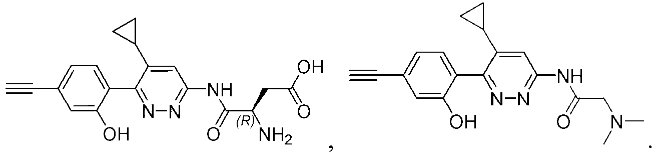

9. A pharmaceutical composition, comprising a combination of the compound, or the deuterated compound thereof, the stereoisomer thereof or the pharmaceutically acceptable salt thereof according to any one of claims 1 to 8 and one or more pharmaceutical carriers.

10. Use of the compound, or the deuterated compound thereof, the stereoisomer thereof or the pharmaceutically acceptable salt thereof according to any one of claims 1 to 8, or the pharmaceutical composition according to claim 9 in the manufacture of a medicament for preventing and/or treating an NLRP3 inflammasome-related disease.

11. Use of the compound, or the deuterated compound thereof, the stereoisomer thereof or the pharmaceutically acceptable salt thereof according to any one of claims 1 to 8, or the pharmaceutical composition according to claim 9 in the manufacture of a medicament for preventing and/or treating an inflammasome-related disease, an immune disease, an inflammatory disease, an autoimmune disease or an autoinflammatory disease.
